(19) Europäisches Patentamt
European Patent Office
Office européen des brevets

(11) Publication number: 0 023 785
B1

(12) **EUROPEAN PATENT SPECIFICATION**

(45) Date of publication of patent specification: 03.04.85

(51) Int. Cl.$^4$: C 07 D 241/44, C 07 D 241/52, A 01 N 43/60

(21) Application number: 80302411.6

(22) Date of filing: 17.07.80

(54) **Quinoxalinyloxyphenoxyalkane carboxylic acid derivatives, their preparation and their use as herbicides.**

(30) Priority: 17.07.79 AU 9617/79
11.04.80 AU 3093/80

(43) Date of publication of application:
11.02.81 Bulletin 81/06

(45) Publication of the grant of the patent:
03.04.85 Bulletin 85/14

(84) Designated Contracting States:
AT BE CH DE FR GB IT LI NL SE

(56) References cited:
EP-A-0 002 800
DE-A-2 640 730
FR-A-2 109 005
GB-A-2 042 539

R. WEGLER, Chemie der Pflanzenschutz- und Schädlingsbekämpfungsmittel, Band 5, Seite 665, Springer-Verlag, Berlin, Heidelberg, New York, 1977

The file contains technical information submitted after the application was filed and not included in this specification

(73) Proprietor: ICI AUSTRALIA LIMITED
ICI House 1 Nicholson Street P.O.Box 4311
Melbourne Victoria 3001 (AU)

(72) Inventor: Serban, Alexander
3 Maple Court
Doncaster Victoria, 3108 (AU)
Inventor: Watson, Keith Geoffrey
36 Medway Street
Box Hill North Victoria, 3129 (AU)
Inventor: Farquharson
10 Steane Street
Reservoir Victoria, 3073 (AU)

(74) Representative: Overin, Alison Diana et al
Imperial Chemical Industries PLC Legal Department: Patents PO Box 6 Bessemer Road
Welwyn Garden City Hertfordshire, A17 1HD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 023 785 B1

**Description**

This invention relates to quinoxaline derivatives having biological activity, in particular having herbicidal properties, to processes for the preparation of such compounds, to intermediates useful in the preparation of such compounds and to herbicidal compositions and processes utilizing such compounds.

Herbicidal heterocyclic ether-type phenoxy acid derivatives are known from DE—A—2640 730 and EP—A—2800. The present invention provides a compound of formula I:

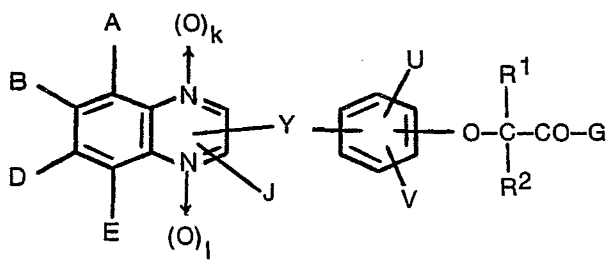

I

or a salt thereof wherein:

A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group —O—N=$R^6$ wherein $R^6$ is a $C_1$ to $C_{10}$ alkylidene, and the group —OM wherein M is an alkali metal ion or an alkaline earth metal ion;

Y is chosen from oxygen and sulfur; and

k and l are independently chosen from 0 and 1 and k + l is 0 or 1.

Preferred compounds of formula I are those 2-quinoxalinyl compounds in which the phenyl ring is 1,4-substituted, that is compounds of formula II

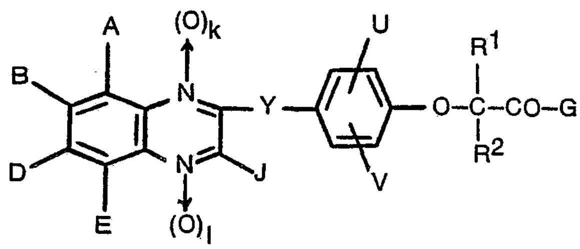

II

Some of these compounds are, however, excluded from the scope of certain aspects of the present invention as follows: when the compound has the formula II identified above in which A, B, D and E are hydrogen or one or two of A, B, D and E are halogen and the others hydrogen, J, U, V and $R^2$ are hydrogen, $R^1$ is hydrogen or $C_1$ to $C_6$ alkyl, Y is oxygen and k and l are 0, then G cannot represent hydroxy, $C_1$ to $C_{10}$ alkoxy or the group OM where M is as defined above, subject to the proviso that the methyl ester of 2-[4-(6-chloro-2-quinoxalinyloxy)phenoxy] propionic acid is specifically included within the scope of all aspects of this invention.

Preferred compounds of the present invention are those wherein;

A is chosen from hydrogen and halogen;

B is chosen from hydrogen, halogen, $C_1$ to $C_6$ alkyl and $C_1$ to $C_6$ haloalkyl;

D is chosen from hydrogen, halogen, $C_1$ to $C_6$ alkyl, $C_1$ to $C_6$ haloalkyl, amino, N,N-di($C_1$ to $C_6$ alkyl)amino and nitro;

E and V are hydrogen;

J and U are independently chosen from hydrogen and halogen;

$R^1$ is chosen from $C_1$ to $C_6$ alkyl;

$R^2$ is chosen from hydrogen and $C_1$ to $C_6$ alkyl;

G is chosen from hydroxy, $C_1$ to $C_{10}$ alkoxy, $C_2$ to $C_{10}$ alkenyloxy, $C_3$ to $C_{10}$ alkynyloxy, $C_1$ to $C_{10}$ alkylthio, cyclohexyloxy, the group $C_2$ to $C_{10}$ alkoxy substituted other than at its α-carbon atom with a substituent chosen from amino, N,N-di($C_1$ to $C_6$ alkyl)amino and N,N,N-tri($C_1$ to $C_6$ alkyl)ammonio, the group —O—N=$R^6$ wherein $R^6$ is $C_1$ to $C_{10}$ akylidene, and the group OM wherein M is an alkali metal ion;

Y is oxygen; and

k and l are independently chosen from 0 and 1 and k + l is 0 or 1.

More preferred compounds of the present invention are those wherein;

A, E, J, U, V and $R^2$ are hydrogen;

B and D are independently chosen from hydrogen, halogen and methyl;

$R^1$ is methyl;

G is chosen from hydroxy, $C_1$ to $C_6$ alkoxy, $C_2$ to $C_6$ alkenyloxy, $C_3$ to $C_6$ alkynyloxy, cyclohexyloxy, the group $C_2$ to $C_6$ alkoxy substituted other than at its α-carbon atom with a substituent chosen from N,N-di($C_1$ to $C_6$ alkyl)amino and N,N,N-tri($C_1$ to $C_6$ alkyl)ammonio, and the group OM wherein M is an alkali metal ion;

Y is oxygen;

k is 0 or 1; and

l is 0.

Particularly preferred compounds of the present invention are those wherein;

A, B, E, J, U, V and $R^2$ are hydrogen;

D is chosen from bromine and chlorine;

$R^2$ is methyl;

G is chosen from hydroxy, $C_1$ to $C_6$ alkoxy, allyloxy, cyclohexyloxy, 2-[N,N-di($C_1$ to $C_6$ alkyl)amino]-ethoxy, 2-[N,N,N-tri($C_1$ to $C_6$ alkyl)ammonio]ethoxy and the group OM wherein M is sodium or potassium;

Y is oxygen;

k is 0 or 1; and

l is 0.

The compounds of formula I wherein $R^1$ and $R^2$ are not the same are optically active and the present invention also includes the individual stereoisomers (i.e. enantiomers) of such compounds, and mixtures of those stereoisomers in addition to the racemic mixture of stereoisomers. It is known in the herbicidal art that certain stereoisomers are more herbicidally active than others, cf. for example European Patent published application 0002800.

Examples of compounds embraced by the invention (either *per se* or otherwise) include:

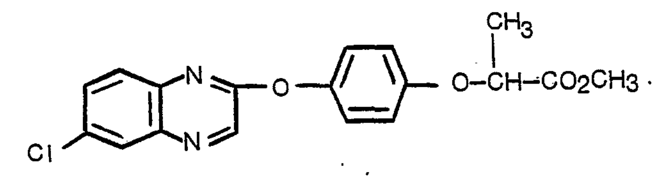

1

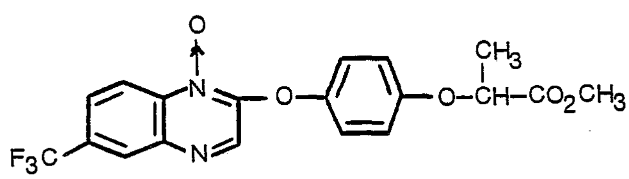

2

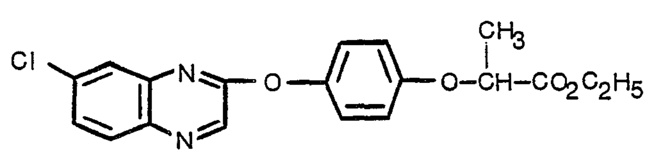

3

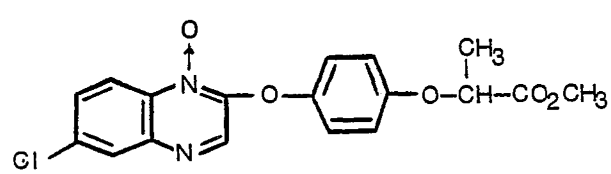

4

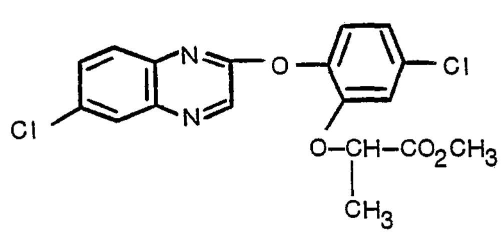

5

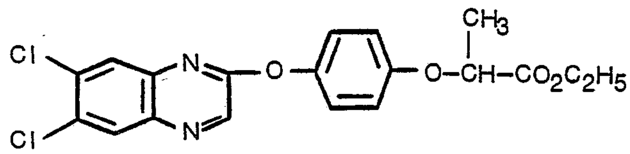

6

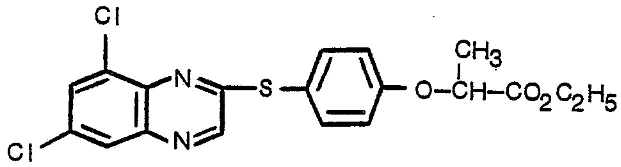

7

Specific examples of compounds of the invention include those detailed in Tables 1a and 1b below.

TABLE 1a

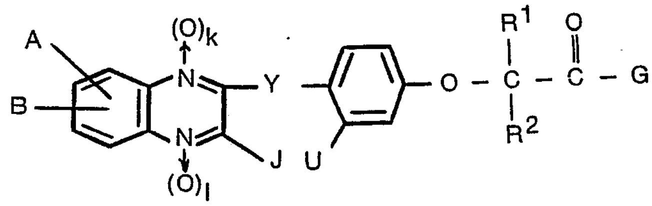

| Compound No. | Substituents | | | | | | | |
|---|---|---|---|---|---|---|---|---|
| | A,B,J | k | l | Y | U | $R^1$ | $R^2$ | G |
| 1 | 6–Cl | 0 | 0 | O | H | $CH_3$ | H | $CH_3O$ |
| 4 | 6–Cl | 1 | 0 | O | H | $CH_3$ | H | $CH_3O$ |
| 8 | 6,7–$Cl_2$ | 0 | 0 | O | H | $CH_3$ | H | $CH_3O$ |
| 9 | 3–Cl | 0 | 0 | O | H | $CH_3$ | H | $CH_3O$ |
| 10 | 6–Cl | 0 | 0 | O | H | $CH_3$ | H | $C_2H_5O$ |
| 11 | 7–Cl | 0 | 0 | O | H | $CH_3$ | H | $CH_3O$ |
| 12 | 6–Cl | 0 | 0 | O | H | $CH_3$ | H | n-$C_3H_7O$ |
| 13 | 6–Cl | 0 | 0 | O | H | $CH_3$ | H | n-$C_4H_9O$ |
| 14 | 6–Cl | 0 | 0 | O | H | $CH_3$ | H | a |
| 15 | 3,6,7–$Cl_3$ | 0 | 0 | O | H | $CH_3$ | H | $CH_3O$ |
| 16 | all H | 0 | 0 | O | H | $CH_3$ | H | $C_2H_5O$ |
| 17 | 6–$NO_2$ | 0 | 0 | O | H | $CH_3$ | H | $C_2H_5O$ |
| 18 | 6–$CH_3$ | 0 | 0 | O | H | $CH_3$ | H | $C_2H_5O$ |
| 19 | 6,8–$Cl_2$ | 0 | 0 | O | H | $CH_3$ | H | $C_2H_5O$ |
| 20 | 6,7–$(CH_3)_2$ | 0 | 0 | O | H | $CH_3$ | H | $C_2H_5O$ |
| 21 | 6–Br | 0 | 0 | O | H | $CH_3$ | H | $C_2H_5O$ |
| 22 | 6–Cl | 0 | 0 | O | H | $CH_3$ | H | $(CH_3)_2CHCH_2O$ |
| 23 | 6–Cl | 0 | 0 | O | H | $CH_3$ | H | $CH_2$=$CHCH_2O$ |
| 24 | 6–Cl | 0 | 0 | O | H | $CH_3$ | H | CH≡$CCH_2O$ |
| 25 | 6–Cl | 0 | 0 | O | H | $CH_3$ | H | HO |

TABLE 1a (Continued)

| Compound No. | Substituents | | | | | | | |
|---|---|---|---|---|---|---|---|---|
| | A,B,J | k | l | Y | U | $R^1$ | $R^2$ | G |
| 26 | 6–Cl | 0 | 0 | O | H | $CH_3$ | H | $Na^{\oplus}\,O^{\ominus}$– |
| 27 | 6–Cl | 0 | 0 | S | H | $CH_3$ | H | $C_2H_5O$ |
| 28 | 6–Cl | 0 | 0 | O | H | $CH_3$ | H | $(CH_3)_2C{=}N{-}O$ |
| 29 | 6–Cl | 0 | 0 | O | H | $CH_3$ | H | b |
| 30 | 6–Cl | 0 | 0 | O | H | $CH_3$ | H | c |
| 31 | 6–Cl | 1 | 0 | O | H | $CH_3$ | H | $C_2H_5O$ |
| 32 | 7–$CF_3$ | 0 | 0 | O | H | $CH_3$ | H | $C_2H_5O$ |
| 33 | 6–Cl | 0 | 0 | O | F | $CH_3$ | H | $C_2H_5O$ |
| 34 | 6–F | 0 | 0 | O | H | $CH_3$ | H | $C_2H_5O$ |
| 35 | 6–Cl | 0 | 1 | O | H | $CH_3$ | H | $C_2H_5O$ |
| 36 | 6–Cl | 0 | 0 | O | H | H | H | $C_2H_5O$ |
| 37 | 6–Cl | 0 | 0 | O | H | $CH_3$ | $CH_3$ | $C_2H_5O$ |
| 38 | 6–Cl | 0 | 0 | O | H | $C_2H_5$ | H | $C_2H_5O$ |
| 39 | 6–$NH_2$ | 0 | 0 | O | H | $CH_3$ | H | $C_2H_5O$ |
| 40 | 6–$N(CH_3)_2$ | 0 | 0 | O | H | $CH_3$ | H | $C_2H_5O$ |
| 41 | 6–Cl | 0 | 0 | O | H | $CH_3$ | H | n-$C_4H_9S$ |
| 42 | 6–Cl | 0 | 0 | O | H | $CO_2C_2H_5$ | $CH_3$ | $C_2H_5O$ |

Footnotes: a. cyclohexyloxy

b. $(CH_3)_2NCH_2CH_2O$

c. $I^{\ominus}(CH_3)_3\overset{\oplus}{N}CH_2CH_2O$

TABLE 1b

| Compound No. | Structure |
|---|---|
| 43 | 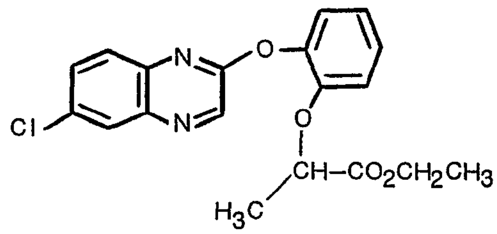 |
| 44 | 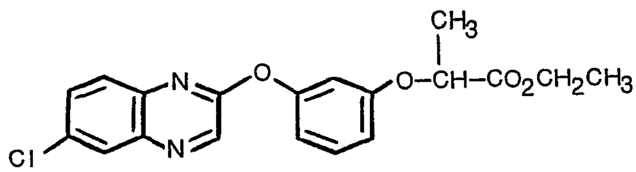 |

The compounds of the invention may be prepared by a variety of methods and in a further aspect the invention provides methods for the preparation of the compounds of formula I.

Compounds of formula I wherein G is not hydroxy may be prepared from the corresponding acid (i.e. the compound of formula I wherein G is hydroxy) by, for example, neutralisation of the acid with a base to give an acid salt or esterification of the acid with an alcohol or phenol to give an acid ester (SCHEME A). Processes known in the art for the preparation of acid salts, acid esters and acid halides may be adapted, without undue experimentation.

SCHEME A

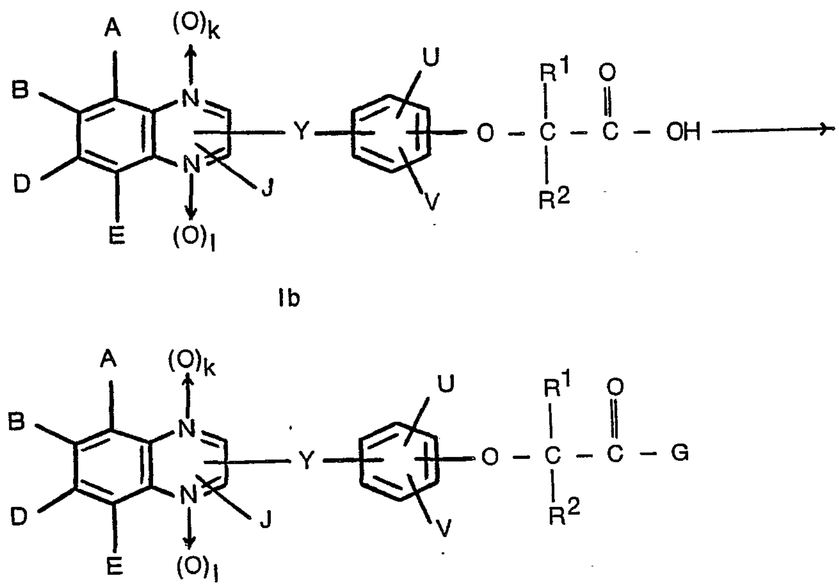

Ib

Ia

N-oxides of the invention of formula I wherein k and/or l is 1 may be prepared from compounds of the invention of formula I wherein k and/or l is 0 by oxidation. Processes known in the art for the conversion of quinoxalines to quinoxaline N-oxides, for example oxidations using persulfates, peroxides, peracids or peresters, may be adapted without undue experimentation to prepare N-oxides of the invention.

Compounds of formula I wherein A, B, D, E, J, U, V, Y, $R^1$, $R^2$, G, k and l are as hereinbefore defined may be prepared by the condensation of a phenol of formula IX with a compound of formula X wherein hal is chlorine, bromine or iodine, preferably in the presence of an alkaline material according to SCHEME B.

SCHEME B

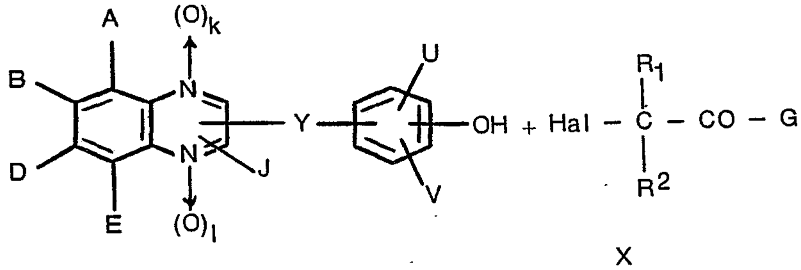

X

IX

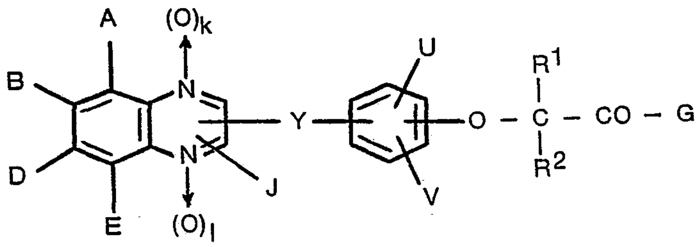

I

Compounds of formula I may also be prepared by:

a) the condensation of the appropriate quinoxaline derivative of formula V, wherein L is a leaving group (for example, alkylsulfonyl, chlorine, bromine or iodine) with the appropriate phenol or thiophenol of formula VI according to SCHEME C.

SCHEME C

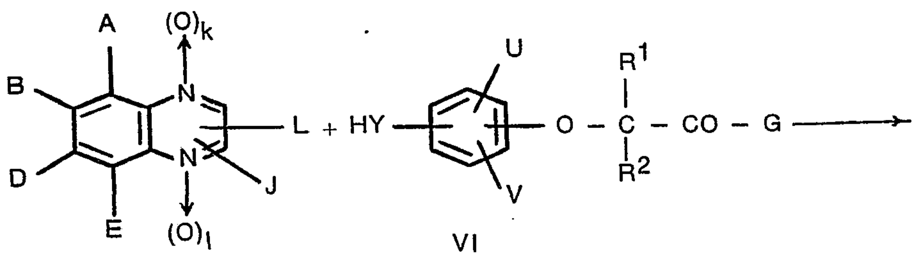

VI

V

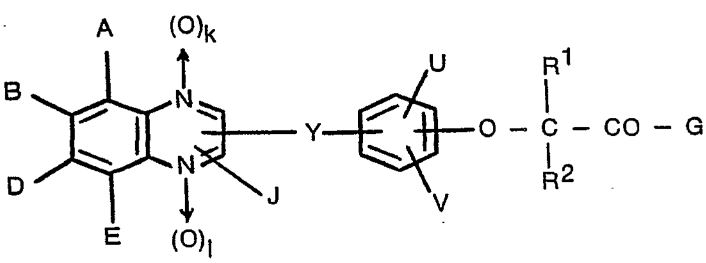

or

I

b) the following steps in sequence:

(i) the condensation of the apppropriate quinoxaline derivative of formula V, wherein L is a leaving group (for example, alkylsulfonyl, chlorine, bromine or iodine) with the appropriate compound of formula VII, wherein Q is hydroxy or $C_1$ to $C_6$ alkoxy to give a compound of formula VIII wherein Q is hydroxy or $C_1$ to $C_6$ alkoxy;

(ii) the dealkylation of the compound of formula VIII prepared in step (i) above wherein Q is $C_1$ to $C_6$ alkoxy to give a compound of formula IX; and

(iii) the condensation of the product of formula IX obtained in step (i) or step (ii) above with a compound of formula X according to the process described for SCHEME B above (Steps (i) and (ii) are shown in SCHEME D); or

SCHEME D

(i)

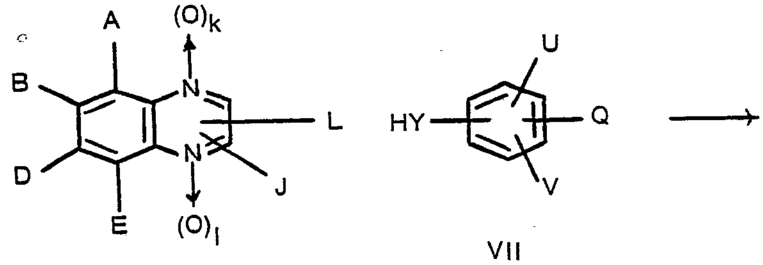

VII

V

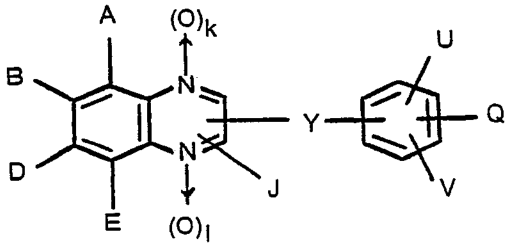

VIII

(ii)

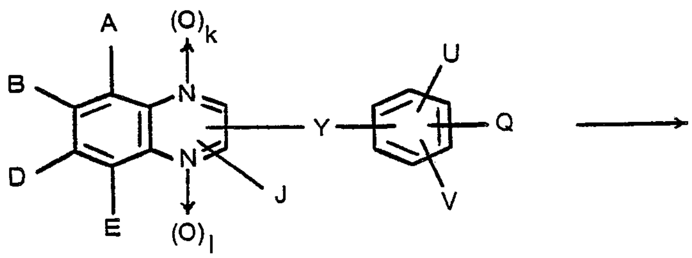

VIII

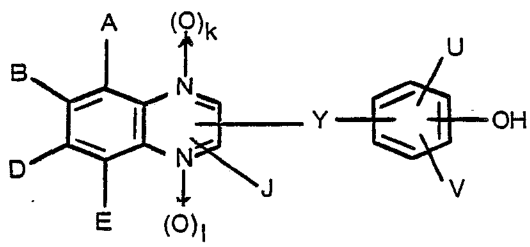

IX

c) the following steps in sequence:

(i) the condensation of the appropriate quinoxaline derivative of formula XI with the appropriate benzene derivative of formula XII wherein L is a leaving group (for example, alkylsulfonyl, chlorine, bromine or iodine) and Q is hydroxy or $C_1$ to $C_6$ alkoxy, to give a compound of formula VIII wherein Q is as hereinbefore defined;

(ii) the dealkylation of the compound of formula VIII prepared in step (i) above wherein Q is $C_1$ to $C_6$ alkoxy to give a compound of formula IX according to the process described for SCHEME D step (ii) above; and

(iii) the condensation of the product of formula IX obtained in step (i) or step (ii) above with a compound of formula X according to the process described for SCHEME B above (step (i) is shown in SCHEME E).

SCHEME E

(i)

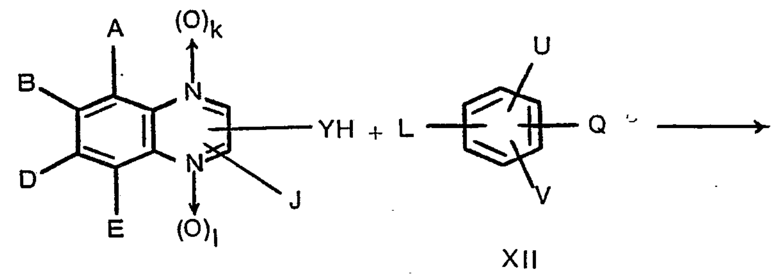

XI

XII

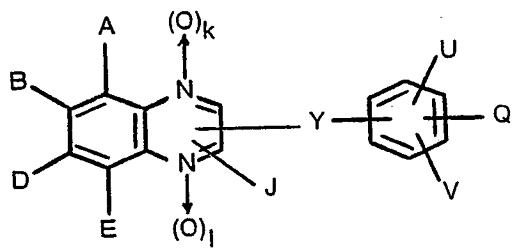

VIII

The condensation reaction illustrated in SCHEMES B to E and outlined above are preferably carried out in the presence of an alkaline material and preferably in the presence of a solvent. Suitable alkaline materials include alkali metal and alkaline earth metal hydroxides and carbonates such as sodium hydroxide; potassium hydroxide, sodium carbonate and potassium carbonate. Suitable solvents include ketones such as, for example, acetone, methyl ethyl ketone and methyl isobutyl ketone, and dipolar aprotic solvents such as, for example, dimethylformamide, dimethylacetamide, dimethylsulfoxide, *N*-methylpyrrolidone, hexamethylphosphoramide and sulfolan.

The reaction conditions required to effect the condensation reactions illustrated in SCHEMES B, C, D, and E and outlined above vary according to the nature of the reactants and the solvent used. In general the reaction is facilitated by the application of heat and usually a reaction temperature in the range of 40°C to 150°C and reaction time of between 0.5 and 20 hours is satisfactory. However, higher or lower reaction temperatures and/or shorter or longer reaction times may be used if desired.

The dealkylation reactions illustrated in SCHEME D and outlined in paragraphs b) (ii) and c) (ii) above may be effected using a variety of reagents known in the art. For example, aryl-alkyl ethers may be cleaved using reagents such as pyridine hydrochloride, hydriodic acid, hydrobromic acid, sodium thioethoxide in dimethylformamide, acetyl-*p*-toluene-sulphonate, sodium or potassium iodide in formic or acetic acid lithium iodide in 2,4,6-collidine and boron tribromide. Reaction times and reaction conditions vary widely depending on the dealkylation agent used and the ether to be cleaved. The reaction conditions generally employed when using the above "ether-cleavage" reagents are known to those skilled in the art and may be adapted without undue experimentation to effect the "ether-cleavage" reactions illustrated in SCHEME D and outlined in paragraph b) (ii) and c) (ii) above. Some of the compounds of formula VIII

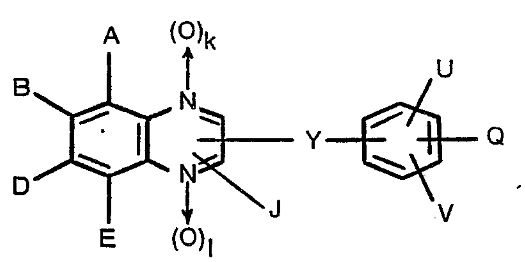

VIII,

which are useful intermediates in the preparation of compounds of formula I, are also believed to be novel compounds. Therefore, in a further embodiment the invention provides compounds of formula VIII wherein A, B, D, E, J, k, l, Y, U, V and Q are as hereinbefore defined subject to the proviso that when the compound has the formula XIII:

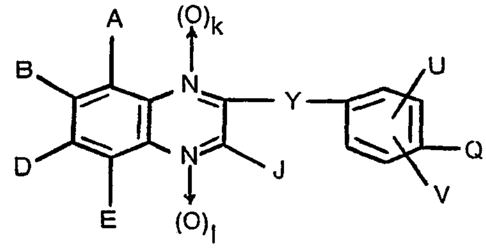

(XIII)

and A, B, D and E are hydrogen or one or two of A, B, D and E are halogen and the others are hydrogen, J, U and V are hydrogen, Y is oxygen and k and l are 0, Q is $C_1$ to $C_6$ alkoxy.

The compounds of formula I are active as herbicides and therefore, in a further aspect the invention provides a process for severely damagaging or killing unwanted plants which process comprises applying to the plants, or to the growth medium of the plants, an effective amount of a compound of formula I as hereinbefore defined.

Generally speaking the compounds of formula I are herbicidally effective against a variety of plants. However, certain of the compounds of the invention are selectively active against monocotyledonous plants, dicotyledonous plants being relatively unaffected by rates of application of the compounds of the invention which are severely damaging or lethal to other plant species.

Moreover, certain of the compounds of formula I are selectively active within the group of monocotyledonous plants and may be used at a rate sufficient to kill or severely damage monocotyledonous weeds in a monocotyledonous cereal crop.

Therefore, in yet a further aspect the invention provides a process for selectively controlling the growth of weeds in crops which process comprises applying to the crop, or to the growth medium of the crop, a compound of formula I, as hereinbefore defined, in an amount sufficient to severely damage or kill the weeds but insufficient to damage the crop substantially.

The compounds of formula I may be applied directly to the plant (post-emergence application) or to the soil before the emergence of the plant (pre-emergence application). However, the compounds are, in general, more effective when applied to the plant post-emergence.

The compounds of formula I may be used on their own to inhibit the growth of, severely damage, or kill plants but are preferably used in the form of a composition comprising a compound of the invention in admixture with a carrier comprising a solid or liquid diluent. Therefore, in yet a further aspect the invention provides plant growth inhibiting, plant damaging, or plant killing compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 0.01% to 90% by weight of the active ingredient. Dilute compositions ready for use preferably contain from 0.01 to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, eg kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as droplets in water.

Surface-active agents may be of the cationic, anionic, or non-ionic type. The cationic agents are, for example, quarternary ammonium compounds (eg cetyltrimethylammonium bromide). Suitable anionic agents are soaps; salts of aliphatic mono esters of sulphuric acid, for example sodium lauryl sulphate; and salts of sulphonated aromatic compounds, for example sodium dodecylbenzenesulphonate, sodium, calcium, and ammonium lignosulphonate, butylnaphthalene sulphonate, and a mixture of the sodium salts of diisopropyl- and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonyl-phenol or octyl-cresol. Other non-ionic agents are the partial esters derived from long chain fatty acids and hexitol anhydrides, for example sorbitan monolaurate; the condensation products of the partial ester with ethylene oxide; and the lecithins.

The aqueous solutions or dispersions may be prepared by dissolving the active ingredient in water or an organic solvent optionally containing wetting or dispersing agent(s) and then, when organic solvents are used, adding the mixture so obtained to water optionally containing wetting or dispersing agent(s). Suitable organic solvents include, for example, ethylene dichloride, isopropyl alcohol, propylene glycol, diacetone alcohol, toluene, kerosene, methylnaphthalene, the xylenes and trichloroethylene.

The compositions for use in the form of aqueous solutions or dispersions are generally supplied in the form of a concentrate containing a high proportion of the active ingredient, and the concentrate is then

diluted with water before use. The concentrates are usually required to withstand storage for prolonged periods and after such storage, to be capable of dilution with water to form aqueous preparations which remain homogeneous for a sufficient time to enable them to be applied by conventional spray equipment. Concentrates conveniently contain 20—90%, preferably 20—70%, by weight of the active ingredient(s). Dilute preparations ready for use may contain varying amounts of the active ingredient(s) depending upon the intended purpose; amounts of 0.01% to 10.0% and preferably 0.1% to 2%, by weight of active ingredient(s) are normally used.

A preferred form of concentrated composition comprising the active ingredient which has been finely divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth. Preferred suspending agents are those which impart thixotropic properties to, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound chosen for use, the identity of the plants whose growth is to be inhibited the formulations selected for use and whether the compound is to be applied for foliage or root uptake. As a general guide, however, an application rate of from 0.005 to 20 kilograms per hectare is suitable while from 0.01 to 5 kilograms per hectare may be preferred.

The compositions of the invention may comprise, in addition to one or more compounds of the invention, one or more compounds not of the invention but which possess biological activity. For example, as hereinbefore indicated the compounds of the invention are in general substantially more effective against monocotyledonous plants or grass species than against dicotyledonous plants or broad-leaved species. As a result, in certain applications the herbicidal use of the compounds of the invention alone may be sufficient to protect a crop. Accordingly in yet a still further embodiment the invention provides a herbicidal composition comprising a mixture of at least one herbicidal compound of formula I as hereinbefore defined with at least one other herbicide.

The other herbicide may be any herbicide not having the formula I. It will generally be a herbicide having a complementary action. For example, one preferred class is of mixtures comprising a herbicide active against broad-leaved weeds. A second preferred class is of mixtures comprising a contact herbicide.

Examples of useful complementary herbicides include:

A. benzo-1,1,3-thiadiazin-4-one-2,2-dioxides such as 3-isopropylbenzo-2,1,3-thiadiazin-4-one-2,2-dioxide (common name bentazon);

B. hormone herbicides and in particular the phenoxy-alkanoic acids such as 4-chloro-2-methylphenoxy acetic acid (common name MCPA), 2-(2,4-dichlorophenoxy)propionic acid (common name dichlorprop), 2,4,5-trichlorophenoxyacetic acid (common name 2,4,5-T), 4-(4-chloro-2-methylphenoxy)butyric acid (common name MCPB), 2,4-dichlorophenoxyacetic acid (comon name 2,4-D), 4-(2,4-dichlorophenoxy)-butyric acid (common name 2,4-DB), 2-(4-chloro-2-methylphenoxy)propionic acid (common name mecoprop), and their derivatives (eg salts, esters, amides and the like);

C. 3-[4-(4-halophenoxy)phenyl]-1,1-dialkylureas such as 3-[4-(4-chlorophenoxy)phenyl]-1,1-dimethylurea (common name chloroxuron);

D. dinitrophenols and their derivatives (eg acetates) such as 2-methyl-4,6-dinitrophenol (common name DNOC), 2-tertiarybutyl-4,6-dinitrophenol (common name dinoterb), 2-secondarybutyl-4,6-dinitrophenol (common name dinoseb) and its ester dinoseb acetate;

E. dinitroaniline herbicides such as N',N'-diethyl-2,6-dinitro-4-trifluoromethyl-*m*-phenylenediamine (common name dinitramine), 2,6-dinitro-N,N-dipropyl-4-trifluoromethylaniline (common name trifluralin) and 4-methylsulfonyl-2,6-dinitro-N,N-dipropylaniline (common name nitralin);

F. phenyl-urea herbicides such as N'-(3,4-dichlorophenyl)-N,N-dimethylurea (common name diuron) and N,N-dimethyl-N'-[3-(trifluoromethyl)phenyl]urea (common name fluometuron);

G. phenylcarbamoyloxyphenylcarbamates such as 3-[(methoxycarbonyl)amino]phenyl (3-methylphenyl)carbamate (common name phenmedipham) and 3-[(ethoxycarbonyl)amino]phenyl phenylcarbamate (common name desmedipham);

H. 2-phenylpyridazin-3-ones such as 5-amino-4-chloro-2-phenylpyridazin-3-one (common name pyrazon);

I. uracil herbicides such as 3-cyclohexyl-5,6-trimethyleneuracil (common name lenacil), 5-bromo-3-*sec*-butyl-6-methyluracil (common name bromacil) and 3-*tert*-butyl-5-chloro-6-methyluracil (common name terbacil);

J. triazine herbicides such as 2-chloro-4-ethylamino-6-(*iso*-propylamino)-1,3,5-triazine (common name atrazine), 2-chloro-4,6-di(ethylamino)-1,3,5-triazine (common name simazine) and 2-azido-4-(isopropylamino)-6-methylthio-1,3,5-triazine (common name aziprotryne);

K. 1-alkoxy-1-alkyl-3-phenylurea herbicides such as 3-(3,4-dichlorophenyl)-1-methoxy-1-methylurea (common name linuron), 3-(4-chlorophenyl)-1-methoxy-1-methylurea (common name monolinuron) and 3-(4-bromo-4-chlorophenyl)-1-methoxy-1-methylurea (common name chlorobromuron);

L. thiolcarbamate herbicides such as S-propyl dipropyl-thiocarbamate (common name verolate);

M. 1,2,4-triazin-5-one herbicides such as 4-amino-4,5-dihydro-3-methyl-6-phenyl-1,2,4-triazine-5-one (common name metamitron) and 4-amino-6-*tert*-butyl-4,5-dihydro-3-methylthio-1,3,4-triazin-5-one (common name metribuzin);

N. benzoic acid herbicides such as 2,3,6-trichlorobenzoic acid (common name 2,3,6-TBA), 3,6-dichloro-2-methoxybenzoic acid (common name dicamba) and 3-amino-2,5-dichlorobenzoic acid (common name chloramben).

O. anilide herbicides such as N-butoxymethyl-α-chloro-2',6'-diethylacetanilide (common name butachlor), the corresponding N-methoxy compound (common name alachlor), the corresponding N-*iso*-propyl compound (common name propachlor) and 3',4'-dichloro-propionanilide (common name propanil);

P. dihalobenzonitrile herbicides such as 2,6-dichlorobenzonitrile (common name dichlobenil), 3,5-dibromo-4-hydroxybenzonitrile (common name bromoxynil) and 3,5-diiodo-4-hydroxybenzonitrile (common name ioxynil).

Q. haloalkanoic herbicides such as 2,2-dichloropropionic acid (common name dalapon), trichloroacetic acid (common name TCA) and salts thereof;

R. diphenylether herbicides such as 4-nitrophenyl 2-nitro-4-trifluoromethylphenyl ether (common name fluorodifen), methyl 5-(2,4-dichlorophenoxy)-2-nitrobenzoate (common name bifenox), 2-nitro-5-(2-chloro-4-trifluoromethylphenoxy)benzoic acid and 2-chloro-4-trifluoromethylphenyl 3-ethoxy-4-nitrophenyl ether and the compounds disclosed in European Patent Application Publication No. 0003416; and

S. miscellaneous herbicides including N,N-dimethyldiphenylacetamide (common name diphenamid), N-(1-naphthyl)phthalamic acid (common name naptalam) and 3-amino-1,2,4-triazole.

Examples of useful contact herbicides include:

T. bipyridylium herbicides such as those in which the active entity is the 1,1'-dimethyl-4,4'-dipyridylium ion (common name paraquat) and those in which the active entity is the 1,1'-ethylene-2,2,'-dipyridylium ion (common name diquat),

U. organoarsenical herbicides such as monosodium methanearsonate (common name MSMA); and

V. amino acid herbicides such as N-(phosphonomethyl)glycine (common name glyphosate) and its salts and esters.

The invention is now illustrated by, but in no way limited to, the following Examples.

Example 1

*Methyl 2-[4-(6-chloro-2-quinoxalinyloxy)phenoxy]propionate* (1)

A mixture of 2,6-dichloroquinoxaline (10 mmole prepared from 4-chloro-1,2-dinitrobenzene an ethyl aminoacetate according to A F Crowther et al J. Chem. Soc., *1949*, 1260), methyl 2-[4-hydroxyphenoxy]-propionate (10 mmole), anhydrous sodium carbonate (11 mmole) and dimethylformamide (40 ml) was heated under reflux for a period of 3 hours. The reaction mixture was cooled and poured into water and the aqueous mixture was extracted with diethyl ether. The etherial extract was dried over anhydrous sodium sulfate and the solvent was removed by distillation under reduced pressure to give a solid residue. The residue was crystallised from methanol to give methyl 2-[4-(6-chloro-2-quinoxalinyloxy)phenoxy]-propionate as white needles (2.5 g, 70%), mp 126°C.

The assigned structure was confirmed by proton magnetic resonance spectroscopy and mass spectrometry.

Example 2

*Methyl 2-[4-(6-chloro-1-oxide-quinoxalin-2-yloxy)phenoxy]propionate* (4)

a) Potassium persulfate (7.42 g) was slowly added to a mixture of 2,6-dichloroquinoline (5.0 g) and concentrated sulfuric acid (25 ml) stirred at a temperature of 10°C. On completion of the addition the mixture was allowed to warm to room temperature and stirring was continued for a further 24 hours. The reaction mixture was poured into ice water (400 ml), neutralized with aqueous sodium bicarbonate and extracted with dichloromethane. The organic extract was washed with brine, dried (anhydrous sodium sulfate) and the solvent was removed by evaporation. The residue was crystallised from ethanol to give 2,6-dichloroquinoxaline-1-oxide as brown needles, mp 185°C.

b) A mixture of 2,6-dichloroquinoxaline-1-oxide (1.0 g; 4.7 mmole), methyl 2-(4-hydroxyphenoxy)-propionate (0.92 g, 4.7 mmole), anhydrous potassium carbonate (0.65 g, 4.7 mmole) and methyl ethyl ketone (70 ml) was heated under reflux for a period of 30 hours. The solvent was removed by distillation under reduced pressure and the residue was partitioned between dichloromethane and water. The organic layer was separated and dried and the solvent was evaporated to yield a dark oil. The residue was chromatographed over silica gel (40 g) with dichloromethane elution to give methyl 2-[4-(6-chloro-1-oxide-quinoxalin-2-loxy)phenoxy]propionate as a pale brown solid (0.75 g; 43%), mp 110°C.

The assigned structure was confirmed by proton magnetic resonance spectroscopy and mass spectrometry.

Example 3

Compounds no 8 to 11, 14 to 21, 31 to 34, 36 to 38, 43 and 44 detailed in Table 1 were prepared from the appropriate quinoxaline and the appropriate alkyl (hydroxyphenoxy)alkanecarboxylate following essentially the same procedure as that described in Example 1 or Example 2. Compound no 42 (see Table 1) was prepared by reacting 2,6-dichloroquinoxaline and diethyl 2-(4-hydroxyphenoxy)-2-methylmalonate following essentially the same procedure as that described in Example 1.

The structures assigned to each of the compounds was confirmed by proton magnetic resonance spectroscopy and mass spectrometry.

Example 4

*2-[4-(6-Chloro-2-quinoxalinyloxy)phenoxy]propionic acid* (25)

A mixture of 2,6-dichloroquinoxaline (10 mmole), 2-(4-hydroxyphenoxy)propionic acid (10 mmole), anhydrous potassium carbonate (20 mmole) and dry dimethylformamide (50 ml) was heated under reflux for a period of 3 hours. The reaction mixture was cooled and poured into water. Acidification of the aqueous mixture with aqueous 15% HCl gave a precipitate which was collected by filtration. The dried product was recrystallised from toluene to give 2-[4-(6-chloro-2-quinoxalinyloxy)phenoxy]propionic acid as white crystals, mp 130°C decomposition.

The assigned structure was confirmed by proton magnetic resonance spectroscopy and mass spectrometry.

Example 5

*Sodium 2-[4-(6-chloro-2-quinoxalinyloxy)phenoxy]propionate* (26) was prepared by the neutralisation of the corresponding acid (Example 4) with aqueous sodium hydroxide and removal of the solvent under reduced pressure.

Example 6

*n-Propyl 2-[4-(6-chloro-2-quinoxalinyloxy)phenoxy]propionate* (12)

a) A mixture of 2-[4-(6-chloro-2-quinoxalinyloxy)phenoxy]propionic acid (2.0 g; Example 4) and thionyl chloride (15 ml) was heated under reflux for a period of 1 hour. The excess thionyl chloride was removed by distillation to give 2-[4-(6-chloro-2-quinoxalinyloxy)phenoxy]propionyl chloride.

b) A mixture of the propionyl chloride prepared as described in part a) above, n-propanol (10 ml) and triethylamine (2 ml) was stirred at room temperature for a period of 30 minutes. The mixture was poured into water (100 ml) and the aqueous mixture was extracted with diethyl ether. The etherial extract was dried over anhydrous sodium sulfate and the solvent was removed by distillation under reduced pressure to give n-propyl 2-[4-(6-chloro-2-quinoxalinyloxy)phenoxy]propionate as white crystals, mp 80°C.

The assigned structure was confirmed by proton magnetic resonance spectroscopy and mass spectrometry.

Example 7

Compounds no 13, 22 to 24, 29 and 41 detailed in Table 1 were prepared from 2-[4-(6-chloro-2-quinoxalinyloxy)phenoxy]propionyl chloride (see Example 6 part a)) and the appropriate alcohol or thiol following essentially the same procedure as that described in Example 6 part b). Compound no 28 (see Table 1) was similarly prepared from 2-[4-(6-chloro-2-quinoxalinyloxy)phenoxy]propionyl chloride and acetone oxime.

The structures assigned to each of the compounds was confirmed by proton magnetic resonance spectroscopy and mass spectrometry and appropriate physical data is recorded in Example 14 Table 2.

Example 8

*2-(N,N,N-triemethylammonio)ethyl 2-[4-(6-chloro-2-quinoxalinyloxy)phenoxy]propionate iodide* (30) was made by reacting 2-(N,N-dimethylamino)ethyl 2-[4-(6-chloro-2-quinoxalinyloxy)phenoxy]propionate 29, see Example 7) with methyl iodide. The salt had a melting point of 68°C.

Example 9

*Ethyl 2-[4-(6-chloro-2-quinoxalinylthio)phenoxy]propionate* (27)

a) A solution of 4-mercaptophenol (10 mmole) in ethanol (10 ml) was added in one portion to a solution of sodium ethoxide (10 mmole) in ethanol (30 ml). After stirring the mixture at room temperature for a period of 15 minutes, 2,6-dichloroquinoxaline (10 mmole) was added and the mixture was stirred for a further period of 30 minutes. The reaction mixture was diluted with water (500 ml) and the precipitate was collected by filtration and dried to give 4-(6-chloro-2-quinoxalinylthio)phenol, mp 204°C.

b) A mixture of the thio ether (1.0 g) prepared as described in part a) above, ethyl 2-bromopropionate (0.6 g), potassium carbonate (0.5 g) and methyl ethyl ketone (30 ml) was heated under reflux for a period of 8 hours. After cooling, the mixture was filtered and the solvent was removed by distillation under reduced pressure to give ethyl 2-[4-(6-chloro-2-quinoxalinylthio)phenoxy]propionate, mp 110—115°C.

The assigned structure was confirmed by proton magnetic resonance spectroscopy and mass spectrometry.

Example 10

*Ethyl 2-[4-(6-chloro-4-oxide-quinoxalin-2-yloxy)phenoxy]propionate* (35)

a) m-Chloroperbenzoic acid (10.32 g of 90% active ingredient) was added portionwise, over a period of 40 minutes, to a mixture of 2,6-dichloroquinoxaline (9.85 g) and dichloromethane (100 ml) stirred at ice bath temperature. On completion of the addition the mixture was allowed to warm to room temperature

and stirring was continued for a further period of 4 days. Dichloromethane was added to the suspension and the mixture was washed with aqueous 5% sodium bicarbonate (3 × 500 ml). The dichloromethane solution was dried (anhydrous sodium sulfate) and the solvent was evaporated. The residue was recrystallised from methanol to give 2,6-dichloroquinoxaline-4-oxide (6.1 g, 57%) as pale orange needles, mp 176°C.

b) 2,6-Dichloroquinoxaline-4-oxide was reacted with ethyl 2-(4-hydroxyphenoxy)propionate, following essentially the same procedure as that described in Example 2 part b), to give ethyl 2-[4-(6-chloro-4-oxide-quinoxalin-2-yloxy)phenoxy]propionate, mp 105°C.

The assigned structure was confirmed by proton magnetic resonance spectroscopy and mass spectrometry.

Example 11

*Ethyl 2-[4-(6-amino-2-quinoxalinyloxy)phenoxy]propionate* (39)

Ethyl 2-[4-(6-nitro-2-quinoxalinyloxy)phenoxy]propionate (20 g, compound no 17 prepared as described in Example 3 was dissolved in ethyl acetate (600 ml) and hydrogenated at room temperature and pressure using a palladium on charcoal catalyst. On completion of hydrogen uptake the solution was filtered through a pad of "Celite"® diatomaceous earth and the solvent was removed from the filtrate by distillation under reduced pressure. The residue was chromatographed over alumina with dichloromethane elution to give, after evaporation of the solvent, ethyl 2-[4-(6-amino-2-quinoxalinyloxy)phenoxy]propionate as yellow crystals, mp 134°C.

The assigned structure was confirmed by proton magnetic resonance spectroscopy and mass spectrometry.

Example 12

*Ethyl 2-{4-[6-(dimethylamino)-2-quinoxalinyloxy]phenoxy}propionate* (40)

A mixture of ethyl 2-[4-(6-amino-2-quinoxalinyloxy)phenoxy]propionate (10 mmole), methyl iodide (25 mmole), acetone (50 ml) and potassium carbonate (25 mmole) was heated under reflux for a period of 24 hours. The reaction mixture was filtered and the solvent was evaporated. The residue was chromatographed over alumina with dichloromethane elution to give ethyl 2-{4-[6-(dimethylamino)-2-quinoxalinyloxy]phenoxy}propionate as a yellow oil.

The assigned structure was confirmed by proton magnetic resonance spectroscopy and mass spectrometry.

Example 13

*Preparation of alkyl 2-[4-6-chloro-2-quinoxalinyloxy)phenoxy]propionates from 4-(6-chloro-2-quinoxalinyloxy)phenol*

a) A mixture of 2,6-dichloroquinoxaline (25 mmole), hydroquinone (25 mmole), calcium hydroxide (25 mmole) and dimethylformamide (50 ml) was heated under reflux for a period of 1 hour. The mixture was cooled and poured into water (500 ml) and the resulting precipitate was collected by filtration and dried. The dried product was chromatographed over silica gel with dichloromethane/acetone (9:1 v/v) elution to give, after evaporation of the solvent, 4-(6-chloro-2-quinoxalinyloxy)phenol, mp 206°C.

b) Compounds no 1, 10, 12, 13 and 22 were prepared by heating under reflux a mixture of 4-(6-chloro-2-quinoxalinyloxy)phenol, methyl ethyl ketone, anhydrous potassium carbonate and, respectively, methyl 2-bromopropionate, ethyl 2-bromopropionate, n-propyl 2-bromopropionate, n-butyl 2-bromopropionate and 2-methylpropyl 2-bromopropionate. The structures assigned to the products were confirmed by proton magnetic resonance spectroscopy and mass spectrometry and their physical properties were identical to the physical properties of the compounds prepared as described in Examples 1, 3, 6, 7 and 7 respectively.

Example 14

Many of the compounds of the invention detailed in Table 1 are solids and can be identified by melting point. For convenience the melting points are tabulated in Table 2a below.

Some of the compounds of the invention detailed in Table 1 are oils and were characterised by, and can be identified by their pmr spectrum. For convenience the pmr spectroscopy data is recorded in Table 2b below.

TABLE 2a

| Compound No. | Melting Point °C | Compound No. | Melting Point °C |
|---|---|---|---|
| 1 | 126 | 24 | 99 |
| 4 | 110 | 25 | 130 dec |
| 8 | 109 | 27 | 110–115 |
| 9 | 108 | 28 | 122 |
| 10 | 92–93 | 30 | 68 dec |
| 11 | 95 | 31 | 121 |
| 12 | 80 | 32 | 93 |
| 13 | 79–80 | 33 | 109 |
| 15 | 140 | 34 | 74 |
| 16 | 70 | 35 | 105 |
| 17 | 105 | | |
| 18 | 89 | 36 | 128 |
| 19 | 74 | 37 | 70 |
| 20 | 96 | 38 | 68 |
| 21 | 78 | 39 | 134 |
| 23 | 82–83 | 44 | 88 |

TABLE 2b

| Compound No. | Proton Chemical Shift δ in ppm ($CDCl_3$) | | | |
|---|---|---|---|---|
| | Aryl Groups | $R^1$ | $R^2$ | G |
| 14 | 8.6,s,1H;8.0,brs,1H 7.6,m,2H;7.1,m,4H | see G | 4.7,m,2H see G | 1.5,m,13H |
| 22 | 8.8,s,1H;8.2,brs,1H; 7.7,brs,2H;7.1,m,4H | 1.7,d,3H | 4.9,q,1H | 4.0,d,2H; 1.8,m,1H; 1.0,d,6H |
| 29 | 8.7,s,1H;8.1,brs,1H; 7.7,brs,2H;7.1,m,4H | 1.7,d,3H | 4.9,q,1H | 4.3,t,2H; 2.7,t,2H; 2.3,s,6H |
| 40 | 8.7,s,1H;6.9–7.7,m, 7H (6–$(CH_3)_2N$ 3.0,s,6H) | 1.9,d,3H | 4.8,q,1H | 4.2,q,2H; 1.2,t,3H |
| 41 | 8.7,s,1H;7.0–8.0,m, 7H | see G | 4.8,q,1H | 2.9,brt, 2H; 1.0–1.8,m,10H |
| 42 | 8.7,s,1H;8.0,brs,1H; 7.6,m,2H;7.2,m,4H | see G | 1.8,s,3H | 4.4,q,4H; 1.3,t,6H |
| 43 | 8.8,s,1H;7.0–8.1, m,7H | 1.9,d,3H | 4.7,q,1H | 4.1,q,2H; 1.1,t,3H |

Example 15

Concentrated formulations of the compound of the invention were prepared by:

a) in the case of oils and waxy solids, dissolving the compound in toluene containing 7% v/v "Teric" N13 ("Teric" is a Trade Mark and "Teric" N13, a product of ethoxylation of nonylphenol, is available from ICI Australia Limited) and 3% v/v "Kemmat" SC15B ("Kemmat" is a Trade Mark and "Kemmat" SC15B is a formulation of calcium dodecylbenzene sulfonate); or

b) in the case of crystalline solids, adding 5 parts by weight of the compound and 1 part by weight of "Dyapol" PT ("Dyapol" is a Trade Mark and "Dyapol" PT is an anionic suspending agent) to 94 parts by weight of an aqueous solution containing 0.25% v/v of "Terric" N8 (a product of ethoxylation of nonylphenol) and ball-milling the mixture to produce a stable suspension. The emulsifiable concentrates and suspensions were then diluted with water to give an aqueous composition of the required concentration suitable for use in the evaluation of the pre-emergence and post-emergence herbicidal activity of the compounds of the invention.

Example 16

The pre-emergent herbicidal activity of the compounds of the invention formulated as described in Example 15 was assessed by the following procedure.

The seeds of the test species were sown in rows 2 cm deep in soil contained in seed boxes. The monocotyledonous plants and the dicotyledonous plants were sown in separate boxes and after sowing the two boxes were sprayed with the required quantity of a composition of the invention. Two duplicate seed boxes were prepared in the same manner but were not sprayed with a composition of the invention and were used for comparison purposes. All the boxes were placed in a glasshouse, lightly watered with an overhead spray to initiate germination and then sub-irrigated as required for optimum plant growth. After three weeks the boxes were removed from the glass house and the effect of the treatment was visually assessed. The results are presented in Table 3 where the damage to plants is rated on a scale of from 0 to 3 where 0 represents from 0 to 25% damage, 3 represents 75 to 99% kill and 3+ represents 100% kill. A dash (—) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 3
PRE-EMERGENCE HERBICIDAL ACTIVITY

| Compound No. | Application Rate kg/ha | Test Plant | | | | | | | |
|---|---|---|---|---|---|---|---|---|---|
| | | Wh | Ot | Rg | Jm | P | Ip | Ms | Sf |
| 1 | 5 | 2 | 2 | 3+ | 3+ | 0 | 0 | 0 | 0 |
| 1 | 1 | 2 | 2 | 3+ | 3+ | 0 | 0 | 0 | 0 |
| 1 | 0.5 | 0 | 2 | 3 | 3+ | 0 | 0 | 0 | 0 |
| 4 | 5 | 2 | 2 | 3+ | 3+ | 0 | 0 | 0 | 0 |
| 4 | 1 | 0 | 0 | 0 | 0 | 0 | 0 | 0 | 0 |
| 8 | 5 | 1 | 1 | 3+ | 3+ | 0 | 0 | 0 | 0 |
| 8 | 1 | 0 | 0 | 2 | 3 | 0 | 0 | 0 | 0 |
| 10 | 0.5 | 0 | 1 | 2 | 3+ | 0 | 0 | 0 | 0 |
| 12 | 0.25 | 0 | 0 | 0 | 1 | 0 | 0 | 0 | 0 |
| 13 | 0.25 | 0 | 0 | 0 | 0 | 0 | 0 | 0 | 0 |
| 14 | 0.5 | 0 | 1 | 0 | 1 | 0 | 0 | 0 | 0 |
| 18 | 5 | 3 | 0 | 3+ | 3+ | 0 | 0 | 0 | 0 |
| 18 | 1 | 0 | 0 | 3 | 2 | 0 | 0 | 0 | 0 |
| 21 | 5 | 3 | 3+ | 3+ | 3+ | 0 | 0 | 0 | 0 |
| 21 | 1 | 2 | 3 | 2 | 3+ | 0 | 0 | 0 | 0 |
| 21 | 0.25 | 0 | 0 | 0 | 3 | 0 | 0 | 0 | 0 |
| 23 | 5 | 3+ | 3+ | 3+ | 3+ | 0 | 0 | 0 | 0 |
| 23 | 1 | 2 | 2 | 3 | 3+ | 0 | 0 | 0 | 0 |
| 27 | 5 | 1 | 2 | 3 | 3 | 0 | 0 | 0 | 0 |
| 27 | 1 | 0 | 0 | 0 | 0 | 0 | 0 | 0 | 0 |
| 28 | 1 | 0 | 0 | 0 | 0 | 0 | 0 | 0 | 0 |
| 29 | 1 | 2 | 1 | 3 | 3+ | 0 | 0 | 0 | 0 |
| 29 | 0.5 | 0 | 0 | 2 | 3 | 0 | 0 | 0 | 0 |
| 30 | 1 | 1 | 0 | 1 | 3 | 0 | 0 | 0 | 0 |
| 31 | 5 | 2 | 0 | 3 | 3+ | 0 | 0 | 0 | 0 |
| 31 | 1 | 0 | 0 | 0 | 3 | 0 | 0 | 0 | 0 |
| 31 | 0.25 | 0 | 0 | 0 | 0 | 0 | 0 | 0 | 0 |

Example 17

The post-emergent heribidical activity of the compounds of the invention formulated as described in Example 15 was assessed by the following procedure.

The seeds of the test species were sown in rows 2 cm deep in soil contained in seed boxes. The monocotyledonous plants and the dicotyledonous plants were sown in separate seed boxes in duplicate. The four seed boxes were placed in a glasshouse, lightly watered with an overhead spray to initiate germination and then subirrigated as required for optimum plant growth. After the plants had grown to a height of about 10 to 12.5 cm one box of each of the monocotyledonous plants and the dicotyledonous plants was removed from the glasshouse and sprayed with the required quantity of a composition of the invention. After spraying the boxes were returned to the glasshouse for a further 3 weeks and the effect of treatment was visually assessed by comparison with the untreated controls. The results are presented in Table 4 where the damage to plants is rated on a scale of from 0 to 3 where 0 represents 0 to 25% damage, 3 represents 75 to 99% kill and 3+ represents 100% kill. A dash (—) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 4
POST-EMERGENCE HERBICIDAL ACTIVITY

| Compound No. | Application Rate kg/ha | Test Plant | | | | | | | |
|---|---|---|---|---|---|---|---|---|---|
| | | Wh | Ot | Rg | Jm | P | Ip | Ms | Sf |
| 1 | 5 | 3+ | 3+ | 3+ | 3+ | 0 | 0 | 0 | 0 |
| 1 | 1 | 3+ | 3+ | 3+ | 3+ | 0 | 0 | 0 | 0 |
| 1 | 0.5 | 3+ | 3+ | 3+ | 3+ | 0 | 0 | 0 | 0 |
| 1 | 0.25 | 3+ | 3+ | 3+ | 3+ | 0 | 0 | 0 | 0 |
| 1 | 0.1 | 3+ | 3+ | 3+ | 3+ | 0 | 0 | 0 | 0 |
| 4 | 5 | 3+ | 3+ | 3+ | 3+ | 0 | 0 | 0 | 0 |
| 4 | 1 | 3+ | 3+ | 3 | 3+ | 0 | 0 | 0 | 0 |
| 8 | 5 | 1 | 2 | 3+ | 3+ | 0 | 0 | 0 | 0 |
| 8 | 1 | 0 | 1 | 2 | 2 | 0 | 0 | 0 | 0 |
| 10 | 0.5 | 3+ | 3+ | 3+ | 3+ | 0 | 0 | 0 | 0 |
| 10 | 0.1 | 3+ | 3+ | 3+ | 3+ | 0 | 0 | 0 | 0 |
| 12 | 0.25 | 3+ | 3+ | 3+ | 3+ | 0 | 0 | 0 | 0 |
| 12 | 0.1 | 3+ | 3+ | 3+ | 3+ | 0 | 0 | 0 | 0 |
| 13 | 0.25 | 3+ | 3+ | 3+ | 3+ | 0 | 0 | 0 | 0 |
| 14 | 0.5 | 3+ | 3+ | 3+ | 3+ | 0 | 0 | 0 | 0 |
| 18 | 5 | 2 | 0 | 3+ | 3+ | 0 | 0 | 0 | 0 |
| 18 | 1 | 2 | 0 | 3 | 3+ | 0 | 0 | 0 | 0 |
| 21 | 5 | 3+ | 3+ | 3+ | 3+ | 0 | 0 | 0 | 0 |
| 21 | 1 | 3+ | 3+ | 3+ | 3+ | 0 | 0 | 0 | 0 |
| 21 | 0.25 | 3+ | 3+ | 3+ | 3+ | 0 | 0 | 0 | 0 |
| 21 | 0.1 | 3+ | 3+ | 3+ | 3+ | 0 | 0 | 0 | 0 |
| 23 | 5 | 3+ | 3+ | 3+ | 3+ | 0 | 0 | 0 | 0 |
| 23 | 1 | 3+ | 3+ | 3+ | 3+ | 0 | 0 | 0 | 0 |
| 27 | 5 | 3 | 3 | 3+ | 3+ | 0 | 0 | 0 | 0 |
| 27 | 1 | 3 | 2 | 3 | 3+ | 0 | 0 | 0 | 0 |
| 28 | 1 | 3 | – | 3 | 3+ | 0 | 0 | 0 | 0 |
| 29 | 1 | 3+ | 3+ | 3+ | 3+ | 0 | 0 | 0 | 0 |
| 29 | 0.5 | 3+ | 3+ | 3+ | 3+ | 0 | 0 | 0 | 0 |

TABLE 4 (Continued)

| Compound No. | Application Rate kg/ha | Test Plant | | | | | | | |
|---|---|---|---|---|---|---|---|---|---|
| | | Wh | Ot | Rg | Jm | P | Ip | Ms | Sf |
| 30 | 1 | 3 | 3 | 3+ | 3+ | 0 | 0 | 0 | 0 |
| 31 | 5 | 3+ | 3+ | 3+ | 3+ | 0 | 0 | 0 | 0 |
| 31 | 1 | 3+ | 3+ | 3+ | 3+ | 0 | 0 | 0 | 0 |
| 31 | 0.25 | 3+ | – | 3+ | 3+ | 0 | 0 | 0 | 0 |

Example 18

The compounds were formulated for test by mixing an appropriate amount with 5 ml of an emulsion prepared by diluting 160 ml of a solution containing 21.8 g per litre of "Span" 80 and 78.2 g per litre of "Tween" 20 in methylcyclohexanone to 500 ml with water. "Span" 80 is a Trade Mark for a surface-active agent comprising sorbitan monlaurate. "Tween" 20 is a Trade Mark for a surface-active agent comprising a condensate of sorbitan monolaurate with 20 molar proportions of ethylene oxide. Each 5 ml emulsion containing a test compound was then diluted to 40 ml with water and sprayed on to young pot plants (post-emergence test) of the species named in Table 5 below. Damage to test plants were assessed after 14 days on a scale of 0 to 5 where 0 to 20% damage and 5 is complete kill. In a test for pre-emergence herbidical activity, seeds of the test plants were sown in a shallow slit formed in the surface of soil in fibre trays. The surface was then levelled and sprayed, and fresh soil then spread thinly over the sprayed surface. Assessment of herbicidal damage was carried out after 21 days using the same scale of 0 to 5 as the post-emergence test. In both cases the degree of herbidical damage was assessed by comparison with untreated control plants. The results are given in Table 5 below. A dash (—) means that no experiment was carried out.

The names of the test plants were as follows:

| | |
|---|---|
| Sb | Sugar beet |
| Rp | Rape |
| Ct | Cotton |
| Sy | Soy bean |
| Mz | Maize |
| Ww | Winter wheat |
| Rc | Rice |
| Sn | *Senecio vulgaris* |
| Ip | *Ipomea purpurea* |
| Am | *Amaranthus retroflexus* |
| Pi | *Polygonum aviculare* |
| Ca | *Chenopodium album* |
| Po | *Portulaca oleracea* |
| Xa | *Xanthium pensylvanicum* |
| Ab | *Abutilon theophrasti* |
| Cv | *Convolvulus arvensis* |

| | |
|---|---|
| Ot | Cultivated oats and wild oats (*Avena fatua*)<br>Wild oats are used in the post-emergence test and cultivated oats in the pre-emergence test |
| Dg | *Digitaria sanguinalis* |
| Pu | *Poa annua* |
| St | *Setaria viridis* |
| Ec | *Echinochloa crus-galli* |
| Sh | *Sorghum halepense* |
| Ag | *Agropyron repens* |
| Cn | *Cyperus rotundus* |

TABLE 5 – PART A

| Compound No. | APPLICATION Method | Rate (kg/ha) | TEST PLANT Sb | Rp | Ct | Sy | Mz | Ww | Rc | Sn | Ip | Am | Pi | Ca |
|---|---|---|---|---|---|---|---|---|---|---|---|---|---|---|
| 1 | PRE | 2.0 | 0 | 0 | 0 | 0 | 4 | 5 | 5 | 2 | 0 | 0 | 0 | – |
| 1 | PRE | 0.5 | 0 | 0 | 0 | 0 | 2 | 4 | 5 | 0 | 0 | 0 | 0 | – |
| 1 | POST | 2.0 | 0 | 0 | 0 | 0 | 5 | 4 | 4 | 0 | 0 | 0 | 0 | 0 |
| 1 | POST | 0.5 | 0 | 1 | 0 | 0 | 5 | 4 | 4 | 0 | 0 | 0 | 0 | 0 |
| 1 | POST | 0.01 | 0 | – | – | – | 4 | 4 | 0 | 0 | 0 | 0 | 0 | 0 |
| 8 | PRE | 2.0 | 0 | 0 | 0 | 0 | 4 | 4 | 4 | 0 | 0 | 0 | 0 | 0 |
| 8 | POST | 0.5 | 0 | 0 | 0 | 0 | 4 | 3 | 2 | 1 | 0 | 2 | 0 | 0 |
| 8 | POST | 0.5 | 0 | 0 | 0 | 0 | 4 | 3 | 2 | 1 | 0 | 2 | 0 | 0 |
| 10 | PRE | 0.2 | 0 | 0 | 0 | 0 | 4 | 5 | 5 | 0 | 0 | 0 | 0 | 0 |
| 10 | PRE | 0.01 | 0 | 0 | 0 | 0 | 1 | 0 | 1 | 0 | 0 | 0 | 0 | 0 |
| 10 | POST | 0.2 | 0 | 0 | 0 | 1 | 5 | 4 | 4 | 2 | 0 | 0 | 0 | 0 |
| 10 | POST | 0.01 | 1 | 0 | 0 | – | 4 | 4 | 1 | 0 | 0 | 1 | 0 | – |
| 12 | PRE | 0.2 | 0 | 0 | 0 | 0 | 4 | 4 | 4 | – | 0 | 0 | 0 | 0 |
| 12 | PRE | 0.01 | 0 | 0 | 0 | 0 | 0 | 0 | 0 | 0 | 0 | 0 | 0 | 0 |
| 12 | POST | 0.2 | 0 | 0 | 0 | 0 | 5 | 4 | 4 | 1 | 0 | 0 | 0 | 0 |
| 12 | POST | 0.01 | 0 | 1 | 0 | – | 4 | 4 | 0 | 0 | 0 | 0 | 0 | 0 |
| 13 | PRE | 0.125 | 0 | 0 | 0 | 0 | 1 | 1 | 0 | 0 | 0 | 0 | 0 | 0 |
| 13 | PRE | 0.025 | 0 | 0 | 0 | 0 | 1 | 1 | 0 | 0 | 0 | 0 | 0 | 0 |
| 13 | POST | 0.125 | 0 | 0 | 0 | 0 | 4 | 4 | 3 | 1 | 0 | 3 | 0 | 0 |
| 13 | POST | 0.025 | 0 | 0 | 0 | 0 | 4 | 4 | 3 | 1 | 0 | 3 | 0 | 0 |
| 14 | PRE | 0.125 | 0 | 0 | 0 | 0 | 1 | 0 | 0 | 3 | 0 | 0 | 0 | 0 |
| 14 | PRE | 0.025 | 0 | 0 | 0 | 0 | 1 | 0 | 0 | 3 | 0 | 0 | 0 | 0 |
| 14 | POST | 0.125 | 0 | 0 | 0 | 0 | 4 | 3 | 0 | 0 | 0 | 0 | 0 | 0 |
| 14 | POST | 0.025 | 0 | 0 | 0 | 0 | 4 | 3 | 0 | 0 | 0 | 0 | 0 | 0 |
| 23 | PRE | 0.5 | – | – | – | – | 3 | 4 | 3 | – | – | – | – | – |
| 23 | PRE | 0.1 | – | – | – | – | 2 | 0 | 0 | – | – | – | – | – |
| 23 | POST | 0.5 | – | – | – | – | 4 | 4 | 3 | – | – | – | – | – |
| 23 | POST | 0.1 | – | – | – | – | 4 | 4 | 0 | – | – | – | – | – |

TABLE 5 – PART B

| Compound No. | APPLICATION Method Rate (kg/ha) | TEST PLANT | | | | | | | | | | | |
|---|---|---|---|---|---|---|---|---|---|---|---|---|---|
| | | Po | Xa | Ab | Cv | Ot | Dg | Pu | St | Eα | Sh | Ag | Cn |
| 1 | PRE 2.0 | 0 | 0 | 0 | – | 4 | 5 | 5 | 5 | 5 | 4 | 3 | 0 |
| 1 | PRE 0.5 | 0 | 1 | 0 | – | 3 | 4 | 3 | 4 | 4 | 2 | 0 | 0 |
| 1 | POST 2.0 | 0 | 0 | 0 | 1 | 5 | 5 | 3 | 5 | 5 | 5 | 4 | 0 |
| 1 | POST 0.5 | 0 | 0 | 0 | 0 | 4 | 4 | 0 | 5 | 5 | 5 | 4 | 0 |
| 1 | POST 0.01 | 0 | 0 | 0 | 0 | 0 | 2 | 0 | 4 | 4 | 4 | 0 | 0 |
| 8 | PRE 2.0 | 0 | 0 | 0 | – | 2 | 0 | 1 | 4 | 3 | 2 | 0 | 0 |
| 8 | POST 2.0 | – | 0 | 0 | 0 | 4 | 5 | 2 | 5 | 5 | 4 | 4 | – |
| 8 | POST 0.5 | – | 0 | 0 | 0 | 4 | 4 | 1 | 4 | 4 | 4 | 4 | – |
| 10 | PRE 0.2 | 0 | 0 | 0 | – | 4 | 5 | 4 | 5 | 5 | 5 | 4 | 0 |
| 10 | PRE 0.01 | 0 | 1 | 1 | – | 1 | 1 | 0 | 0 | 0 | 0 | 0 | 0 |
| 10 | POST 0.2 | 0 | 0 | 1 | 0 | 4 | 4 | 2 | 5 | 5 | 5 | 4 | 0 |
| 10 | POST 0.01 | – | 0 | 0 | 0 | 3 | 4 | 0 | 5 | 5 | 3 | 3 | 0 |
| 12 | PRE 0.2 | 0 | 0 | 0 | – | 3 | 5 | 4 | 5 | 4 | 0 | 2 | 0 |
| 12 | PRE 0.01 | 0 | 1 | 0 | – | 0 | 0 | 0 | 0 | 0 | 0 | 0 | – |
| 12 | POST 0.2 | 0 | 0 | 0 | 0 | 4 | 4 | 4 | 5 | 5 | 5 | 4 | 0 |
| 12 | POST 0.01 | 0 | 0 | 0 | – | 2 | 3 | 0 | 4 | 5 | 2 | 0 | – |
| 13 | PRE 0.125 | 0 | 0 | 0 | – | 1 | 0 | 0 | 1 | 1 | 0 | 0 | 0 |
| 13 | PRE 0.025 | 0 | 0 | 0 | – | 1 | 0 | 0 | 1 | 1 | 0 | 0 | 0 |
| 13 | POST 0.125 | – | 0 | 0 | 0 | 4 | 4 | 0 | 5 | 5 | 4 | 4 | 0 |
| 13 | POST 0.025 | – | 0 | 0 | 0 | 4 | 4 | 0 | 5 | 5 | 4 | 4 | 0 |
| 14 | PRE 0.125 | 0 | 1 | 0 | – | 0 | 0 | 0 | 1 | 0 | – | 0 | 0 |
| 14 | PRE 0.025 | 0 | 1 | 0 | – | 0 | 0 | 0 | 1 | 0 | – | 0 | 0 |
| 14 | POST 0.125 | – | 0 | 0 | 0 | 1 | 3 | 0 | 4 | 4 | 5 | 0 | 0 |
| 14 | POST 0.025 | – | 0 | 0 | 0 | 1 | 3 | 0 | 4 | 4 | 5 | 0 | 0 |
| 23 | PRE 0.5 | – | – | – | – | 2 | 4 | – | 3 | 4 | 3 | – | – |
| 23 | PRE 0.1 | – | – | – | – | 0 | 0 | – | 0 | 0 | 0 | – | – |
| 23 | POST 0.5 | – | – | – | – | 4 | 4 | – | 4 | 5 | 5 | 4 | – |
| 23 | POST 0.1 | – | – | – | – | 0 | 2 | – | 3 | 4 | 3 | 0 | – |

Example 18

This Example illustrates the selective herbicidal activity of compounds of the invention when applied in the field.

The test compound was formulated following essentially the same procedure described in Example 17.

The seeds of the test plant species were sown using a Stanhay Precision Seeder on flat-topped hills spaced 1 metre apart. Two species were sown on each hill. The flat-topped hills were grouped in sub-plots on the basis of the rate of application of the test chemical. The species were sown at different times to that they would all reach approximately the same stage of growth at the same time.

Each flat-topped hill to be sprayed with the formulated test compound was pegged to a 1.25 metre centre and sprayed to a width of 1 metre using an Oxford Precision Sprayer fitted with two No "O" T-jets.

In the pre-emergence test the flat-topped hills were sprayed with the test compound after sowing and the damage to the test plants was visually assessed 14, 21, 35 and 63 days after spraying. The results, expressed as percentage kill, are given in Table 6

Part A.

In the post-emergence test the flat-topped hills were sprayed with the test compound after the test plants had reached the 2—3 leaf stage and the damage was visually assessed 7, 14, 28 and 56 days after spraying. The results, expressed as percentage kill, are given in Table 6 Part B.

The names of the test plants were as follows:

| | |
|---|---|
| Sy | Soya bean (Bethal) |
| Ct | Cotton (Delta Pine 16) |
| Pn | Peanunt (Red Spanish) |
| Mz | Maize (XL 45) |
| Ss | *Setaria anceps* |
| Dg | *Digitaria sanguinalis* |
| Ec | *Echinochloa crus-galli* |
| Sg | Sorghum (Goldrush) |
| Sh | *Sorghum halepense* |

TABLE 6 – PART A

PRE-EMERGENCE FIELD TEST

| Compound No. | Rate (kg/ha) | DAT* | Percentage Kill of the Test Plants | | | | | | | | |
|---|---|---|---|---|---|---|---|---|---|---|---|
| | | | Sy | Ct | Pn | Mz | Ss | Dg | Ec | Sg | Sh |
| 10 | 2.0 | 14 | 0 | 5 | 3 | 100 | – | – | – | 100 | – |
| 10 | 2.0 | 21 | 0 | 5 | 5 | 100 | 100 | 100 | 100 | 100 | 100 |
| 10 | 2.0 | 35 | 0 | 3 | 5 | 100 | 100 | 100 | 100 | 100 | 100 |
| 10 | 2.0 | 63 | 0 | 0 | 0 | 100 | 100 | 100 | 100 | 100 | 100 |
| 10 | 1.0 | 14 | 0 | 3 | 0 | 100 | – | – | – | 100 | – |
| 10 | 1.0 | 21 | 3 | 0 | 0 | 100 | 100 | 100 | 100 | 100 | 100 |
| 10 | 1.0 | 35 | 0 | 3 | 3 | 100 | 100 | 100 | 100 | 100 | 100 |
| 10 | 1.0 | 63 | 0 | 0 | 0 | 100 | 100 | 100 | 100 | 100 | 100 |
| 10 | 0.5 | 14 | 0 | 5 | 0 | 92 | – | – | – | 97 | – |
| 10 | 0.5 | 21 | 0 | 5 | 3 | 95 | 100 | 100 | 100 | 98 | 100 |
| 10 | 0.5 | 35 | 0 | 0 | 0 | 90 | 100 | 100 | 100 | 93 | 100 |
| 10 | 0.5 | 63 | 0 | 0 | 0 | 75 | 100 | 100 | 100 | 85 | 100 |
| UC+ | – | 14 | 3 | 3 | 3 | 0 | – | – | – | 0 | – |
| UC | – | 21 | 0 | 0 | 0 | 0 | 0 | 0 | 0 | 0 | 0 |
| UC | – | 35 | 0 | 0 | 0 | 0 | 0 | 0 | 0 | 0 | 0 |
| UC | – | 63 | 0 | 0 | 0 | 0 | 0 | 0 | 0 | 0 | 0 |

* DAT – Number of Days After Treatment that assessment was made.

+ UC – Untreated Controls.

TABLE 6 – PART B

POST-EMERGENCE FIELD TEST

| Compound No. | Rate (kg/ha) | DAT* | Percentage Kill of the Test Plants | | | | | | | | |
|---|---|---|---|---|---|---|---|---|---|---|---|
| | | | Sy | Ct | Pn | Mz | Ss | Dg | Ec | Sg | Sh |
| 10 | 2.0 | 7 | 28 | 5 | 8 | 93 | 80 | 75 | 55 | 90 | – |
| 10 | 2.0 | 14 | 10 | 3 | 3 | 100 | 98 | 100 | 100 | 100 | – |
| 10 | 2.0 | 28 | 20 | 8 | 0 | 100 | 100 | 100 | 100 | 100 | – |
| 10 | 2.0 | 56 | 0 | 0 | 0 | 100 | 100 | 100 | 100 | 100 | – |
| 10 | 1.0 | 7 | 13 | 5 | 5 | 88 | 65 | 63 | 48 | 85 | 100 |
| 10 | 1.0 | 14 | 5 | 3 | 8 | 100 | 98 | 100 | 100 | 100 | 100 |
| 10 | 1.0 | 28 | 8 | 3 | 3 | 100 | 100 | 100 | 100 | 100 | 100 |
| 10 | 1.0 | 56 | 0 | 0 | 0 | 100 | 100 | 100 | 100 | 100 | 100 |
| 10 | 0.5 | 7 | 5 | 3 | 3 | 65 | 63 | 55 | 55 | 78 | – |
| 10 | 0.5 | 14 | 5 | 3 | 3 | 100 | 95 | 99 | 100 | 100 | 100 |
| 10 | 0.5 | 28 | 5 | 3 | 0 | 100 | 98 | 100 | 100 | 100 | 100 |
| 10 | 0.5 | 56 | 0 | 0 | 0 | 100 | 100 | 100 | 100 | 100 | 100 |
| UC+ | – | 7 | 0 | 0 | 0 | 0 | 0 | 0 | 0 | 0 | 0 |
| UC | – | 14 | 0 | 0 | 0 | 0 | 0 | 0 | 0 | 0 | 0 |
| UC | – | 28 | 0 | 0 | 0 | 0 | 0 | 0 | 0 | 0 | 0 |
| UC | – | 56 | 0 | 0 | 0 | 0 | 0 | 0 | 0 | 0 | 0 |

* DAT – Number of Days After Treatment that assessment was made.

\+ UC – Untreated Controls.

**Claims for the Contracting States: BE CH DE FR GB IT LI NL SE**

1. A compound of formula I

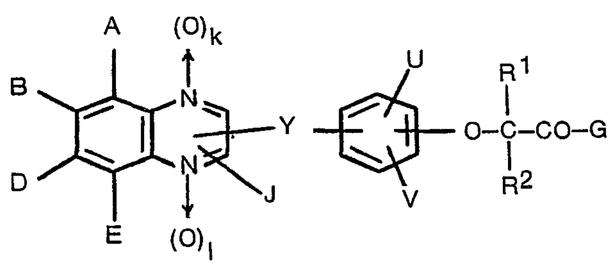

I

or a salt thereof, wherein

A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N—($C_1$ to $C_6$ alkyl)amino, N,N-di($C_1$ to $C_6$ alkyl)amino and N,N,N-tri($C_1$ to $C_6$ alkyl)ammonia, the group —O—N=$R^6$ wherein $R^6$ is a $C_1$ to $C_{10}$ alkylidene, and the group —OM wherein M is an alkali metal ion or an alkaline earth metal ion;

Y is chosen from oxygen and sulfur; and k and l are independently chosen from 0 and 1 and k + l is 0 or 1;

subject to the proviso that when the compound has the formula II

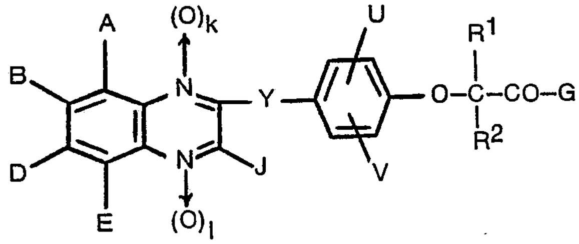

II

and A, B, D and E are hydrogen, or one or two of A, B, D and E are halogen and the others hydrogen, J, U, V and $R^2$ are hydrogen, $R^1$ is hydrogen or $C_1$ to $C_6$ alkyl, Y is oxygen and k and l are 0, G is not hydroxy, $C_1$ to $C_{10}$ alkoxy, or the group OM wherein M is as defined above, but subject to the further proviso that the compound may be the methyl ester of 2-[4-(6-chloro-2-quinoxalinyloxy)phenoxy]propionic acid.

2. A compound of formula I

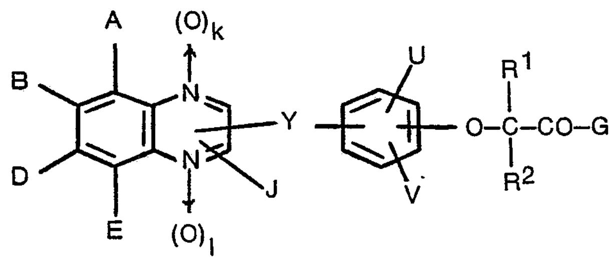

I

or a salt thereof, wherein

A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group —O—N=$R^6$ wherein $R^6$ is a $C_1$ to $C_{10}$ alkylidene, and the group —OM wherein M is an alkali metal ion or an alkaline earth metal ion;

Y is chosen from oxygen and sulfur; and k and l are independently chosen from 0 and 1 and k + l is 0 or 1;

subject to the proviso that when the compound has the formula II

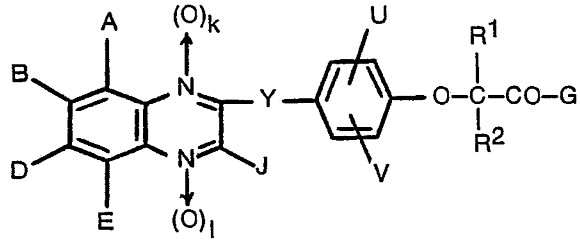

II

and A, B, D and E are hydrogen, or one or two of A, B, D and E are halogen and the others hydrogen, J, U, V and $R^2$ are hydrogen, $R^1$ is hydrogen or $C_1$ to $C_6$ alkyl, Y is oxygen and k and 1 are 0, G is not hydroxy, $C_1$ to $C_{10}$ alkoxy, $C_1$ to $C_{10}$ haloalkoxy, $C_2$ to $C_6$ alkenyloxy, $C_3$ to $C_6$ alkynyloxy, cyclohexyloxy, phenoxy, benzyloxy, the group $C_2$ to $C_{10}$ alkoxy substituted other than at its α-carbon atom with the substituent $C_1$ to $C_6$ alkoxy or the group OM wherein M is as defined above.

3. A compound according to claim 1 or 2 of formula II wherein A, B, D, E, J, U, V, $R^1$, $R^2$, G, Y, k and l are as defined in claim 1.

4. A compound according to claim 1, 2 or 3 wherein

A is chosen from hydrogen and halogen;

B is chosen from hydrogen, halogen, $C_1$ to $C_6$ alkyl and $C_1$ to $C_6$ haloalkyl;

D is chosen from hydrogen, halogen, $C_1$ to $C_6$ alkyl, $C_1$ to $C_6$ haloalkyl, amino, N,N-di($C_1$ to $C_6$ alkyl)amino and nitro;

E and V are hydrogen;

J and U are independently chosen from hydrogen and halogen;

$R^1$ is chosen from $C_1$ to $C_6$ alkyl;

$R^2$ is chosen from hydrogen and $C_1$ to $C_6$ alkyl;

G is chosen from hydroxy, $C_1$ to $C_{10}$ alkoxy, $C_2$ to $C_{10}$ alkenyloxy, $C_3$ to $C_{10}$ alkynyloxy, $C_1$ to $C_{10}$ alkylthio, cyclohexyloxy, the group $C_2$ to $C_{10}$ alkoxy substituted other than at its α-carbon atom with a substituent chosen from amino, N,N-di($C_1$ to $C_6$ alkyl)amino and N,N,N-tri($C_1$ to $C_6$ alkyl)ammonio, the group —O—N=$R^6$ wherein $R^6$ is $C_1$ to $C_{10}$ alkylidene, and the group OM wherein M is an alkali metal ion;

Y is oxygen; and k and l are independently chosen from 0 and 1 and k + l is 0 or 1.

5. A compound according to any one of claims 1 to 4 inclusive wherein:

A, E, J, U, V and $R^2$ are hydrogen;

B and D are independently chosen from hydrogen, halogen and methyl;

$R^1$ is methyl;

G is chosen from hydroxy, $C_1$ to $C_6$ alkoxy, $C_2$ to $C_6$ alkenyloxy, $C_3$ to $C_6$ alkynyloxy, cyclohexyloxy, the group $C_2$ to $C_6$ alkoxy substituted other than at its α-carbon atom with a substituent chosen from N,N-di($C_1$ to $C_6$ alkyl)amino and N,N,N-tri($C_1$ to $C_6$ alkyl)ammonio, and the group OM wherein M is an alkali metal ion;

Y is oxygen;

k is 0 or 1; and l is 0.

6. A compound according to any one of claims 1 to 5 inclusive wherein:

A, B, E, J, U, V and $R^2$ are hydrogen;

D is chosen from bromine and chlorine;

$R^2$ is methyl;

G is chosen from hydroxy, $C_1$ to $C_6$ alkoxy, allyloxy, cyclohexyloxy, 2-[N,N-di($C_1$ to $C_6$ alkyl)amino]-ethoxy, 2-[N,N,N-tri($C_1$ to $C_6$ alkyl)ammonio]ethoxy and the group OM wherein M is sodium or potassium;

Y is oxygen;

k is 0 or 1; and l is 0.

7. A compound which is the methyl or cyclohexyl ester of 2-[4-(6-chloro-2-quinoxalinyloxy)phenoxy]propionic acid or the cyclohexyl ester of 2-[4-(6-bromo-2-quinoxalinyloxy)phenoxy]propionic acid.

8. A compound of formula I

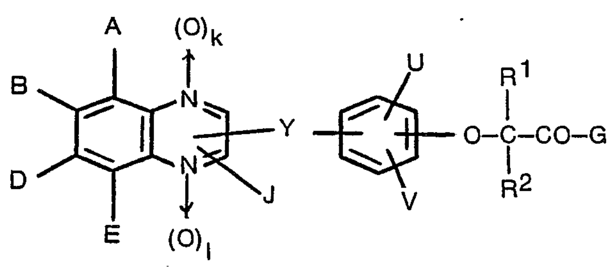

I

or a salt thereof wherein

A, B, D and E are independently chosen from hydrogen, halogen, nitro, $C_1$ to $C_6$ alkyl, $C_1$ to $C_6$ haloalkyl, amino,

$C_1$ to $C_6$ alkylamino and di($C_1$ to $C_6$ alkyl)amino;

J is hydrogen;

U and V are independently chosen from hydrogen and halogen;

$R^1$ is chosen from hydrogen, $C_1$ to $C_6$ alkyl and ($C_1$ to $C_5$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, phenoxy, benzyloxy, the group $C_2$ to $C_{10}$ alkoxy substituted other than at its α-carbon atom with $C_1$ to $C_6$ alkoxy and the group OM wherein M is an alkali metal ion or an alkaline earth metal ion; Y is chosen from oxygen and sulfur; and

k and l are each 0;

subject to the proviso that when the compound has the formula II

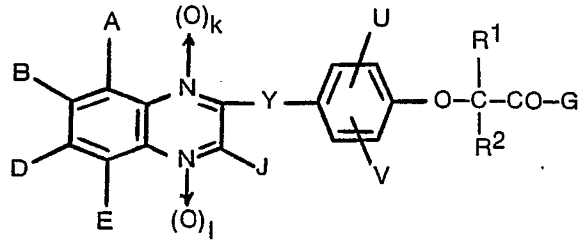

II

and A, B, D and E are hydrogen, or one or two of A, B, D and E are halogen and the others hydrogen, U, V and $R^2$ are hydrogen, $R^1$ is hydrogen or $C_1$ to $C_6$ alkyl and Y is oxygen, G is not hydroxy, $C_1$ to $C_{10}$ alkoxy or the group OM wherein M is as defined above.

9. A composition having herbidical activity against monocotyledonous weeds in dicotyledonous crops characterised by consisting essentially of an enantiomer of formula I

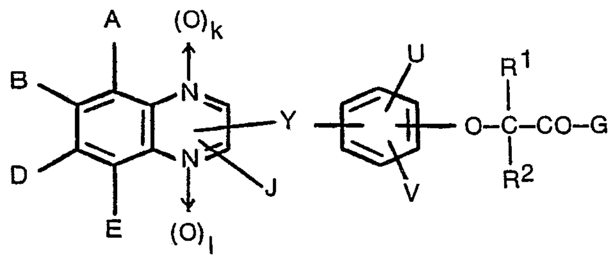

I

or a salt thereof, wherein

A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group —O—N=$R^6$ wherein $R^6$ is a $C_1$ to $C_{10}$ alkylidene, and the group —OM wherein M is an alkali metal ion or an alkaline earth metal ion;

Y is chosen from oxygen and sulfur; and

k and l are independently chosen from 0 and 1 and k + l is 0 or 1;

said composition being substantially free from the other enantiomer.

10. A herbicidal composition comprising, as active ingredient, a compound according to any one of claims 1 to 8 inclusive, and a carrier therefor.

11. A herbicidal composition comprising, as active ingredients, (a) a compound of formula (I)

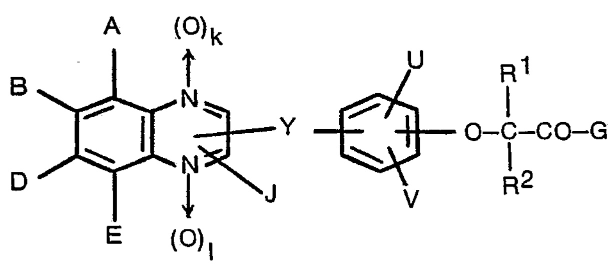

I

or a salt thereof, wherein A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group —O—N=$R^6$

wherein $R^6$ is a $C_1$ to $C_{10}$ alkylidene, and the group —OM wherein M is an alkali metal ion or an alkaline earth metal ion;

Y is chosen from oxygen and sulfur; and

k and l are independently chosen from 0 and 1 and k + l is 0 or 1;

and (b) another herbicide which is a benzo-2,1,3-thiadiazin-4-one-2,2-dioxide; a hormone herbicide; a 3-[4-(4-halophenoxy)phenyl]-1,1-dialkylurea; a dinitrophenol or a derivative thereof; a dinitroaniline herbicide; a phenylurea herbicide; a phenylcarbamoyloxyphenylcarbamate; a 2-phenylpyridazin-3-one; a uracil herbicide; a triazine herbicide; a 1-alkoxy-1-alkyl-3-phenylurea herbicide; a thiolcarbamate herbicide; a 1,2,4-triazin-5-one herbicide; a benzoic acid herbicide; an anilide herbicide; a dihalobenzonitrile herbicide; a haloalkanoic acid herbicide or a salt thereof; a diphenylether herbicide (for example 4-nitrophenyl 2-nitro-4-trifluoromethylphenyl ether, methyl 5-(2,4-dichlorophenoxy)-2-nitrobenzoate, 2-nitro-5-(2-chloro-4-trifluoromethylphenoxy)benzoic acid, 2-chloro-4-trifluoromethylphenyl-3-ethoxy-4-nitrophenyl ether or 2-nitro-5-(2-chloro-4-trifluoromethylphenoxy)-N-methanesulphonylbenzamide); N,N-dimethyldiphenylacetamide; N-(1-naphthyl)-phthalamic acid; 3-amino-1,2,4-triazole; a bipyridylium herbicide; an organoarsenical herbicide; or an aminoacid herbicide or a salt or ester thereof.

12. A process for severely damaging or killing unwanted plants, which process comprises applying to said plants, or to the growth medium of said plants, an effective amount of a compound according to any one of claims 1 to 8 inclusive or an effective amount of a composition as defined according to any one of claims 9 to 11.

13. A process for selectively controlling the growth of monocotyledonous weeds in dicotyledonous crops which process comprises applying to said crop, or to the growth medium of said crop, a compound as defined according to any one of claims 1 to 8 inclusive or a composition as defined according to any one of claims 9 to 11 in an amount sufficient to severely damage or kill the weeds but insufficient to substantially damage the crop.

14. A process according to claim 12 or 13 wherein the compound is applied at a rate in the range from 0.005 to 20 kilograms per hectare.

15. A process for the synthesis of a compound of formula I according to any one of claims 1 to 8 inclusive, which process comprises either the reaction of a quinoxaline derivative of formula IX with a compound of formula X

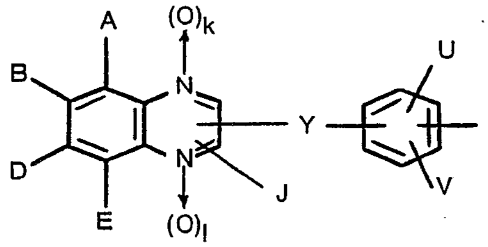

(IX)

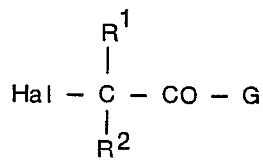

(X)

wherein A, B, D, E, J, U, V, $R^1$, $R^2$, G, Y, k and l are as defined in any one of claims 1 to 6 and 8, and Hal is chlorine, bromine or iodine, or the reaction of a quinoxaline derivative of formula V with a compound of formula VI

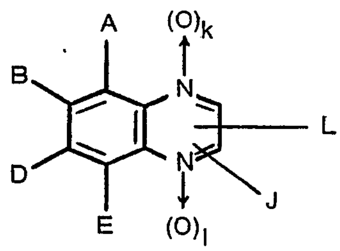

(V)

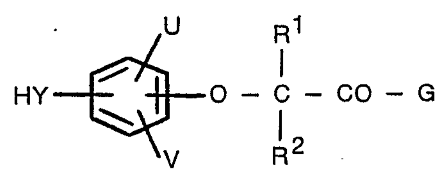

(VI)

wherein A, B, D, E, J, U, V, $R^1$, $R^2$, G, Y, k and l are as defined in any of claims 1 to 6 and 8, and L is a leaving group.

16. A process according to claim 15 wherein the quinoxaline compound of formula IX is prepared by reacting a quinoxaline derivative of formula V wherein A, B, D, E, J, k and l are as defined in any one of claims 1 to 6 and 8, and L is a leaving group, with a phenol or thiophenol of formula VII

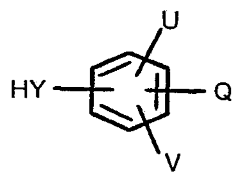

(VII)

wherein U, V and Y are as defined in any one of claims 1 to 6 and 8, and Q is hydroxy or $C_1$ to $C_6$ alkoxy to give a compound of formula VIII

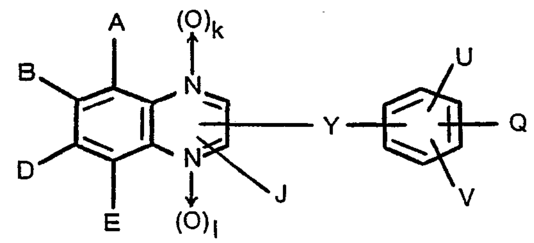

(VIII)

wherein A, B, D, E, J, U, V, Y, k and l are as defined in any one of claims 1 to 6 and 8 and Q is as defined above, and when Q is $C_1$ to $C_6$ alkoxy dealkylating the compound of formula VIII to give a compound of formula IX as defined in claim 15.

17. A process for the synthesis of a compound of formula I

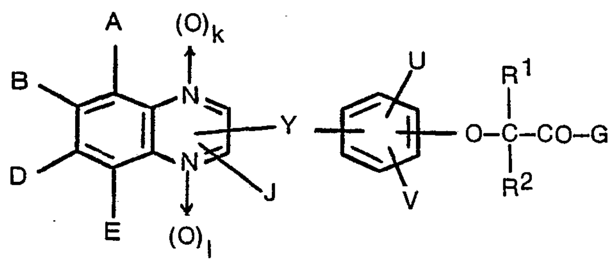

I

or a salt thereof, wherein A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group $—O—N=R^6$ wherein $R^6$ is a $C_1$ to $C_{10}$ alkylidene, and the group —OM wherein M is an alkali metal ion or an alkaline earth metal ion;

Y is chosen from oxygen and sulfur; and

k and l are independently chosen from 0 and 1 and k + l is 0 or 1;

which comprises dealkylating a compound of formula (VIII)

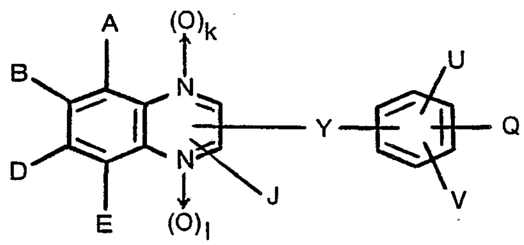

(VIII)

wherein A, B, D, E, J, U, V, Y, k and l are as defined above and Q is $C_1$ to $C_6$ alkoxy to give a compound of formula IX

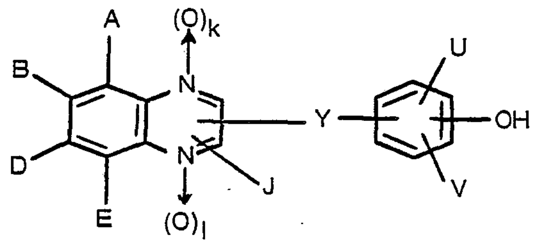

IX

wherein A, B, D, E, J, U, V, Y, k and l are as defined above; and then reacting the compound of formula IX with a compound of formula X

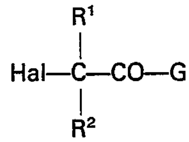

(X)

wherein $R^1$, $R^2$ and G are as defined above and Hal is chlorine, bromine or iodine.

18. A compound of formula VIII

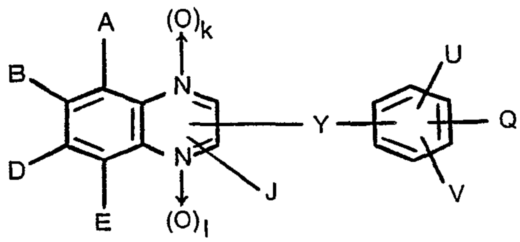

(VIII)

wherein A, B, D, E, J, U, V, Y, k and l are as defined in any one of claims 1 to 6 and 8 inclusive and Q is hydroxy or $C_1$ to $C_6$ alkoxy subject to the proviso that when the compound has the formula XIII:

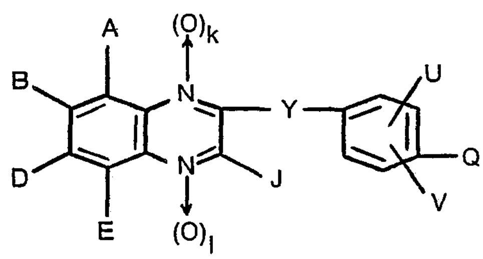

(XIII)

and A, B, D and E are hydrogen or one or two of A, B, D and E are halogen and the others are hydrogen, J, U and V are hydrogen, Y is oxygen and k and l are 0, Q is $C_1$ to $C_6$ alkoxy.

**Claims for the Contracting State: AT**

1. A process for the synthesis of a compound of formula I

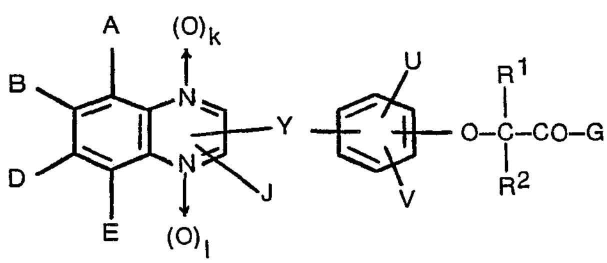

I

or a salt thereof, wherein

A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group —O—N=$R^6$ wherein $R^6$ is a $C_1$ to $C_{10}$ alkylidene, and the group —OM wherein M is an alkali metal ion or an alkaline earth metal ion;

Y is chosen from oxygen and sulfur; and k and l are independently chosen from 0 and 1 and k + l is 0 or 1;

subject to the proviso that when the compound has the formula II

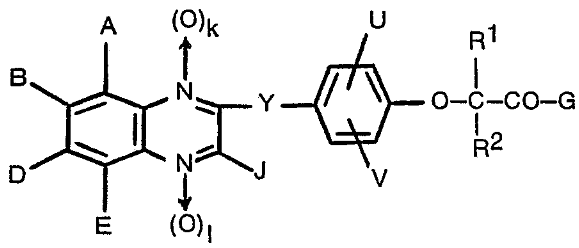

II

and A, B, D and E are hydrogen, or one or two of A, B, D and E are halogen and the others hydrogen, J, U, V and $R^2$ are hydrogen, $R^1$ is hydrogen or $C_1$ to $C_6$ alkyl, Y is oxygen and k and l are 0, G is not hydroxy, $C_1$ to $C_{10}$ alkoxy, or the group OM wherein M is as defined above, but subject to the further proviso that the compound may be the methyl ester of 2-[4-(6-chloro-2-quinoxalinyloxy)phenoxy]propionic acid;

which process comprises either the reaction of a quinoxaline derivative of formula IX with a compound of formula X

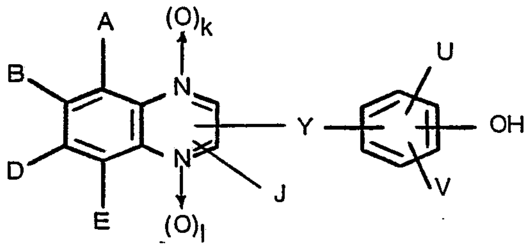

(IX)

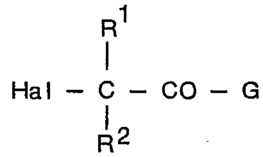

(X)

wherein A, B, D, E, J, U, V, $R^1$, $R^2$, G, Y, k and l are as defined above and Hal is chlorine, bromine or iodine, or the reaction of a quinoxaline derivative of formula V with a compound of formula VI

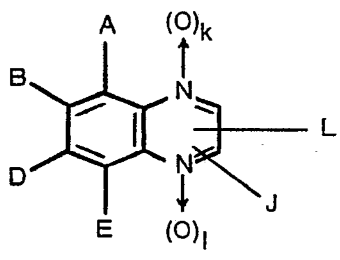

(V)

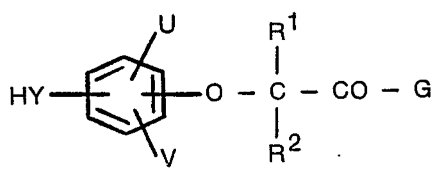

(VI)

wherein A, B, D, E, J, U, V, $R^1$, $R^2$, G, Y, k and l are as defined above, and L is a leaving group.

2. A process according to claim 1 wherein the quinoxaline compound of formula IX is prepared by reacting a quinoxaline derivative of formula V wherein A, B, D, E, J, k and l are as defined in claim 1 and L is a leaving group, with a phenol or thiophenol of formula VII

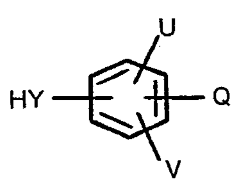

(VII)

wherein U, V and Y are as defined in claim 1 and Q is hydroxy or $C_1$ to $C_6$ alkoxy to give a compound of formula VIII

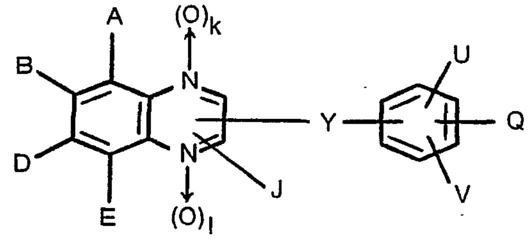 (VIII)

wherein A, B, D, E, J, U, V, Y, k and l are as defined in claim 1 and Q is as defined above, and when Q is $C_1$ to $C_6$ alkoxy dealkylating the compound of formula VIII to give a compound of formula IX as defined in claim 1.

3. A process for the synthesis of a compound of formula I

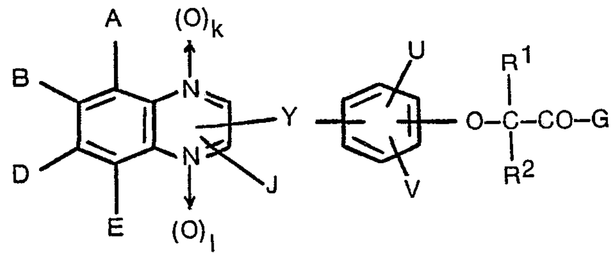 I

or a salt thereof, wherein A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group —O—N=$R^6$ wherein $R^6$ is a $C_1$ to $C_{10}$ alkylidene, and the group —OM wherein M is an alkali metal ion or an alkaline earth metal ion;

Y is chosen from oxygen and sulfur; and

k and l are independently chosen from 0 and 1 and k + l is 0 or 1;

which comprises dealkylating a compound of formula (VIII)

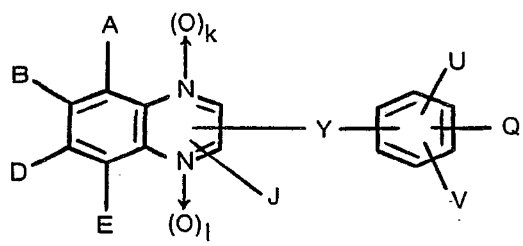 (VIII)

wherein A, B, D, E, J, U, V, Y, k and l are as defined above and Q is $C_1$ to $C_6$ alkoxy to give a compound of formula IX

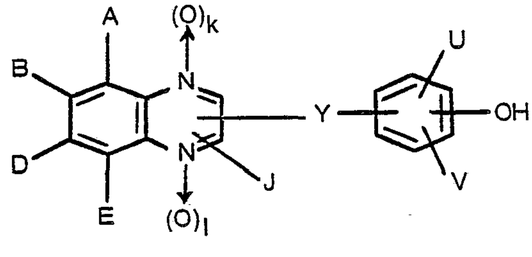 IX

wherein A, B, D, E, J, U, V, Y, k and l are as defined above; and then reacting the compound of formula IX with a compound of formula X

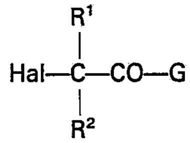

(X)

wherein $R^1$, $R^2$ and G are as defined above and Hal is chlorine, bromine or iodine.

4. A process according to claim 1, 2 or 3 wherein A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group $-O-N=R^6$ wherein $R^6$ is a $C_1$ to $C_{10}$ alkylidene, and the group —OM wherein M is an alkali metal ion or an alkaline earth metal ion;

Y is chosen from oxygen and sulfur; and

k and l are independently chosen from 0 and 1 and k + l is 0 or 1;

subject to the proviso that when the process is for the synthesis of a compound of formula II

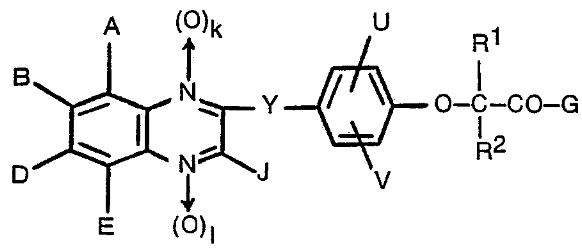

. II

and A, B, D and E are hydrogen, or one or two of A, B, D and E are halogen and the others hydrogen, J, U, V and $R^2$ are hydrogen, $R^1$ is hydrogen or $C_1$ to $C_6$ alkyl, Y is oxygen and k and l are 0, G is not hydroxy, $C_1$ to $C_{10}$ alkoxy, $C_1$ to $C_{10}$ haloalkoxy, $C_2$ to $C_6$ alkenyloxy, $C_3$ to $C_6$ alkynyloxy, cyclohexyloxy, phenoxy, benzyloxy, the group $C_2$ to $C_{10}$ alkoxy substituted other than at its α-carbon atom with the substituent $C_1$ to $C_6$ alkoxy or the group OM wherein M is as defined above.

5. A process according to any one of the preceding claims for the synthesis of a compound of formula II wherein A, B, D, E, J, U, V, $R^1$, $R^2$, G, Y, k and l are as defined in claim 1.

6. A process according to any one of the preceding claims wherein

A is chosen from hydrogen and halogen;

B is chosen from hydrogen, halogen, $C_1$ to $C_6$ alkyl and $C_1$ to $C_6$ haloalkyl;

D is chosen from hydrogen, halogen, $C_1$ to $C_6$ alkyl, $C_1$ to $C_6$ haloalkyl, amino, N,N-di($C_1$ to $C_6$ alkyl)amino and nitro;

E and V are hydrogen;

J and U are independently chosen from hydrogen and halogen;

$R^1$ is chosen from $C_1$ to $C_6$ alkyl;

$R^2$ is chosen from hydrogen and $C_1$ to $C_6$ alkyl;

G is chosen from hydroxy, $C_1$ to $C_{10}$ alkoxy, $C_2$ to $C_{10}$ alkenyloxy, $C_3$ to $C_{10}$ alkynyloxy, $C_1$ to $C_{10}$ alkylthio, cyclohexyloxy, the group $C_2$ to $C_{10}$ alkoxy substituted other than at its α-carbon atom with a substituent chosen from amino, N,N-di($C_1$ to $C_6$ alkyl)amino and N,N,N-tri($C_1$ to $C_6$ alkyl)ammonio, the group $-O-N=R^6$ wherein $R^6$ is $C_1$ to $C_{10}$ alkylidene, and the group OM wherein M is an alkali metal ion;

Y is oxygen; and

k and l are independently chosen from 0 and 1 and k + l is 0 or 1.

7. A process according to any one of the preceding claims wherein:

A, E, J, U, V and $R^2$ are hydrogen;

B and D are independently chosen from hydrogen, halogen and methyl;

$R^1$ is methyl;

G is chosen from hydroxy, $C_1$ to $C_6$ alkoxy, $C_2$ to $C_6$ alkenylxoy, $C_3$ to $C_6$ alkynyloxy, cyclohexyloxy, the group $C_2$ to $C_6$ alkoxy substituted other than at its α-carbon atom with a substituent chosen from N,N-di($C_1$ to $C_6$ alkyl)amino and N,N,N-tri($C_1$ to $C_6$ alkyl)ammonio, and the group OM wherein M is an alkali metal ion;

Y is oxygen;

k is 0 or 1; and l is 0.

8. A process according to any one of the preceding claims wherein:
A, B, E, J, U, V and $R^2$ are hydrogen;
D is chosen from bromine and chlorine;
$R^2$ is methyl;
G is chosen from hydroxy, $C_1$ to $C_6$ alkoxy, allyloxy, cyclohexyloxy, 2-[N,N-di($C_1$ to $C_6$ alkyl)amino]-ethoxy, 2-[N,N,N-tri($C_1$ to $C_6$ alkyl)ammonio]ethoxy and the group OM wherein M is sodium or potassium;
Y is oxygen;
k is 0 or 1; and l is 0.

9. A process according to any one of the preceding claims wherein the reactants are such that a compound which is the methyl or cyclohexyl ester of 2-[4-(6-chloro-2-quinoxalinyloxy)phenoxy]propionic acid or the cyclohexyl ester of 2-[4-(6-bromo-2-quinoxalinyloxy)phenoxy]propionic acid is produced.

10. A process for the synthesis of a compound of formula I

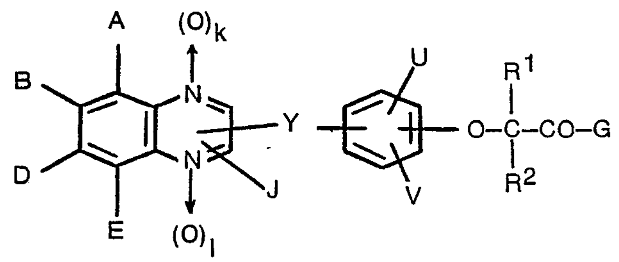

I

or a salt thereof, wherein
A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;
J is hydogen;
U and V are independently chosen from hydrogen and halogen;
$R^1$ is chosen from hydrogen, $C_1$ to $C_6$ alkyl and ($C_1$ to $C_5$ alkoxy)carbonyl;
$R^2$ is chosen from hydrogen and $C_1$ to $C_6$ alkyl;
G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, phenoxy, benzyloxy, the group $C_2$ to $C_{10}$ alkoxy substituted other than at its α-carbon atom with $C_1$ to $C_6$ alkoxy and the group OM wherein M is an alkali metal ion or an alkaline earth metal ion;
Y is chosen from oxygen and sulphur; and
k and l are each 0;
subject to the proviso that when the compound has the formula II

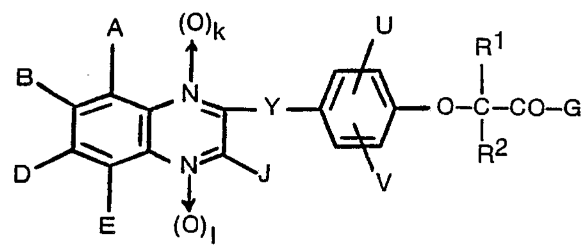

II

and A, B, D and E are hydrogen, or one or two of A, B, D and E are halogen and the others hydrogen, U, V and $R^2$ are hydrogen, $R^1$ is hydrogen or $C_1$ to $C_6$ alkyl and Y is oxygen, G is not hydroxy, $C_1$ to $C_{10}$ alkoxy or the group OM wherein M is as defined above;
which process comprises either the reaction or a quinoxaline derivative of formula IX with a compound of formula X

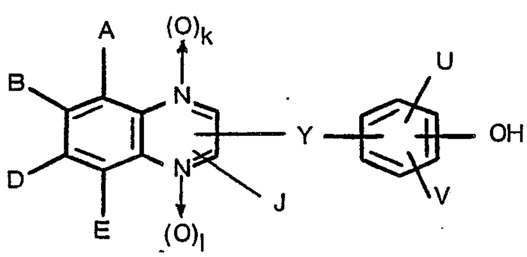

(IX)

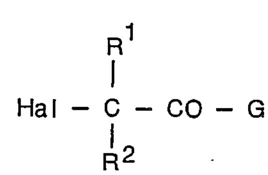

(X)

wherein A, B, D, E, J, U, V, $R^1$, $R^2$, G, Y, k and l are as defined above and Hal is chlorine, bromine or iodine, or the reaction of a quinoxaline derivative of formula V with a compound of formula VI

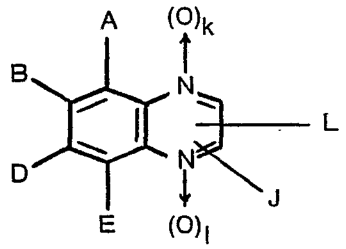

(V)

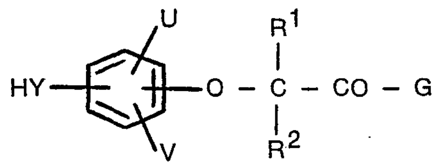

(VI)

wherein A, B, D, E, J, U, V, $R^1$, $R^2$, G, Y, k and l are as defined above, and L is a leaving group.

11. A process for the synthesis of a composition having herbicidal activity against monocotyledonous weeds in dicotyledonous crops characterised by consisting essentially of an enantiomer of formula I

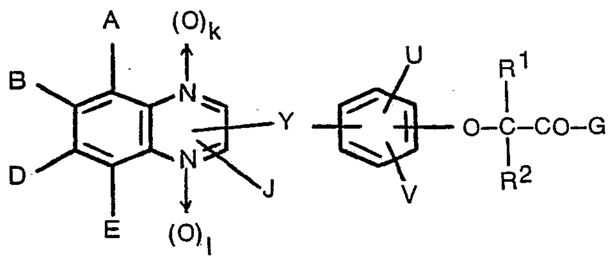

I

or a salt thereof, wherein

A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group —O—N=$R^6$ wherein $R^6$ is a $C_1$ to $C_{10}$ alkylidene, and the group —OM wherein M is an alkali metal ion or an alkaline earth metal ion;

Y is chosen from oxygen and sulfur; and

k and l are independently chosen from 0 and 1 and k + l is 0 or 1;

said composition being substantially free from the other enantiomer;

which process comprises either the reaction of a quinoxaline derivative of formula IX with a compound of formula X

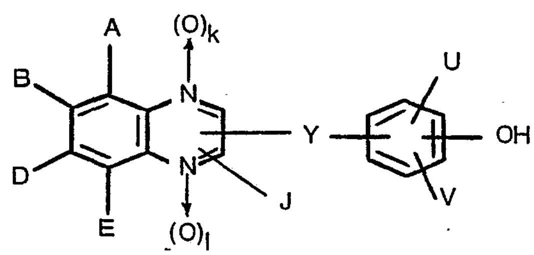

(IX)

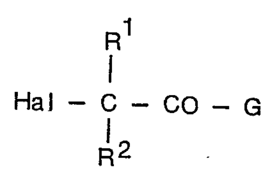

(X)

wherein A, B, D, E, J, U, V, $R^1$, $R^2$, G, Y, k and l are as defined above and Hal is chlorine, bromine or iodine, or the reaction of a quinoxaline derivative of formula V with a compound of formula VI

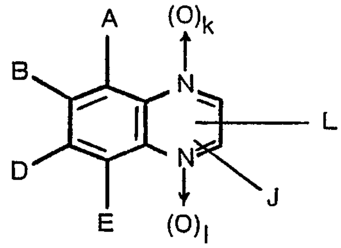

(V)

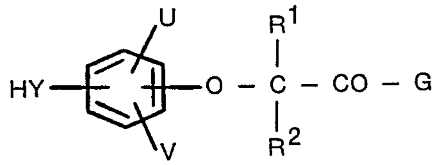

(VI)

wherein A, B, D, E, J, U, V, $R^1$, $R^2$, G, Y, k and l are as defined above and L is a leaving group.

12. A herbicidal composition comprising, as active ingredient, a compound of formula I or II as defined in any one of claims 1 to 10 inclusive, and a carrier therefor.

13. A herbicidal composition comprising, as active ingredients, (a) a compound of formula (I)

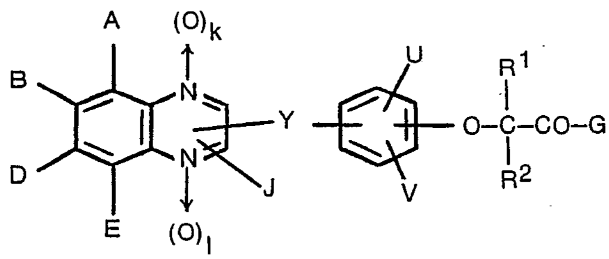

I

or a salt thereof, wherein A, B, D and E are independently chosen from hydrogen, halogen, nitro, $C_1$ to $C_6$ alkyl, $C_1$ to $C_6$ haloalkyl, amino, $C_1$ to $C_6$ alkylamino and di($C_1$ to $C_6$ alkyl)amino;

J, U and V are independently chosen from hydrogen and halogen;

$R^1$ is chosen from hydrogen, $C_1$ to $C_6$ alkyl and ($C_1$ to $C_6$ alkoxy)carbonyl;

$R^2$ is chosen from hydrogen and $C_1$ to $C_6$ alkyl;

G is chosen from hydroxy, $C_1$ to $C_{10}$ alkoxy, $C_1$ to $C_{10}$ haloalkoxy, $C_2$ to $C_{10}$ alkenyloxy, $C_3$ to $C_{10}$ alkynyloxy, $C_1$ to $C_{10}$ alkylthio, cyclohexyloxy, phenoxy, benzyloxy, the group $C_2$ to $C_{10}$ alkoxy substituted other than at its α-carbon atom with a substituent chosen from $C_1$ to $C_6$ alkoxy, amino, N-($C_1$ to $C_6$ alkyl)amino, N,N-di($C_1$ to $C_6$ alkyl)amino and N,N,N-tri($C_1$ to $C_6$ alkyl)ammonio, the group —O—N=$R^6$ wherein $R^6$ is a $C_1$ to $C_{10}$ alkylidene, and the group —OM wherein M is an alkali metal ion or an alkaline earth metal ion;

Y is chosen from oxygen and sulfur; and

k and l are independently chosen from 0 and 1 and k + l is 0 or 1;

and (b) another herbicide which is a benzo-2,1,3-thiadiazin-4-one-2,2-dioxide; a hormone herbicide; a 3-[4-(4-halophenoxy)phenyl]-1,1-dialkylurea; a dinitrophenol or a derivative thereof; a dinitroaniline herbicide; a phenylurea herbicide; a phenylcarbamoyloxyphenylcarbamate; a 2-phenylpyridazin-3-one; a uracil herbicide; a triazine herbicide; a 1-alkoxy-1-alkyl-3-phenylurea herbicide; a thiolcarbamate herbicide; a 1,2,4-triazin-5-one herbicide; a benzoic acid herbicide; an anilide herbicide; a dihalobenzonitrile herbicide a haloalkanoic acid herbicide or a salt thereof; a diphenylether herbicide (for example 4-nitrophenyl 2-nitro-4-trifluoromethylphenyl ether, methyl 5-(2,4-dichlorophenoxy)-2-nitrobenzoate, 2-nitro-5-(2-chloro-4-trifluoromethylphenoxy)benzoic acid, 2-chloro-4-trifluoromethylphenyl-3-ethoxy-4-nitrophenyl ether or 2-nitro-5-(2-chloro-4-trifluoromethylphenoxy)-N-methanesulphonylbenzamide); N,N-dimethyldiphenylacetamide; N-(1-naphthyl)-phthalamic acid; 3-amino-1,2,4-triazole; a bipyridylium herbicide; an organoarsenical herbicide; or an aminoacid herbicide or a salt or ester thereof.

14. A process for severely damaging or killing unwanted plants, which process comprises applying to said plants, or to the growth medium of said plants, an effective amount of a compound as defined in any one of claims 1 to 10 inclusive or an effectve amount of a composition as defined according to any one of claims 11 to 13.

15. A process for selectively controlling the growth of monocotyledonous weeds in dicotyledonous crops which process comprises applying to said crop, or to the growth medium of said crop, a compound as defined in any one of claims 1 to 10 inclusive or a composition as defined in any one of claims 11 to 13 in an amount sufficient to severely damage or kill the weeds but insufficient to substantially damage the crop.

16. A process according to claim 14 or 15 wherein the compound is applied at a rate in the range from 0.005 to 20 kilograms per hectare.

17. A process for the synthesis of a compound of formula VIII

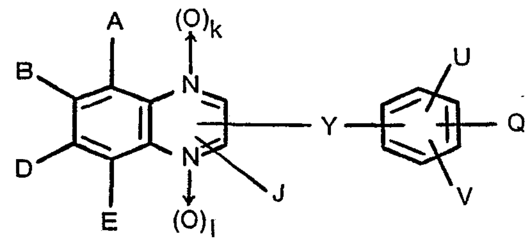

(VIII)

wherein A, B, D, E, J, U, V, Y, k and l are as defined in any one of claims 1 to 8 and 10 inclusive and Q is hydroxy or $C_1$ to $C_6$ alkoxy which process comprises reacting a quinoxaline derivative of formula V

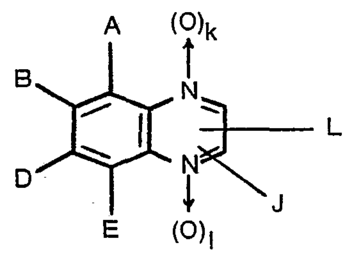

(V)

wherein A, B, D, E, J, k and l are as defined above and L is a leaving group, with a phenol or thiophenol of formula VII

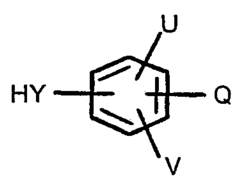

(VII)

wherein U, V, Y and Q are as defined above.

**Patentansprüche für die Vertragsstaaten: BE CH DE FR GB IT LI NL SE**

1. Verbindung der Formel I

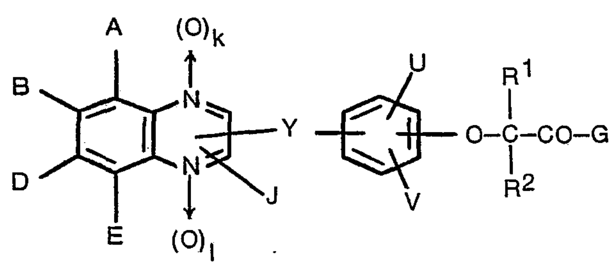

I

oder ein Salz davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist;

mit der Maßgabe, daß, wenn die Verbindung die Formel II

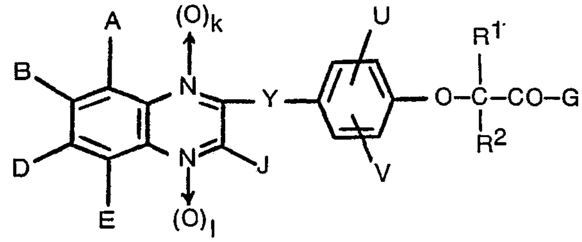

II

hat und A, B, D und E für Wasserstoff oder eines oder Zwei der Symbole A, B, D und E für Halogen und die anderen für Wasserstoff stehen, J, U, V und $R^2$ für Wasserstoff stehen, $R^1$ für Wasserstoff oder $C_1$—$C_6$-Alkyl steht, Y für Sauerstoff steht und k und l 0 sind, G nicht für Hydroxy, $C_1$—$C_{10}$-Alkoxy oder die Gruppe —OM, worin M die oben angegebene Bedeutung besitzt, steht, aber mit der weiteren Maßgabe, daß die Verbindung der Methylester von 2-[4-(6-Chloro-2-chinoxalinyloxy)phenoxy]propionsäure sein kann.

2. Verbindung der Formel I

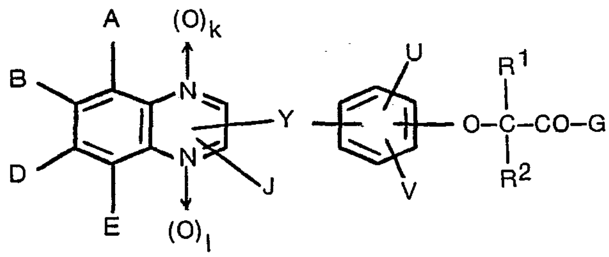

I

oder ein Salz davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k and l unabhängig ausgewählt sind aus 0 und 1 und k + l 0 oder 1 ist;

mit der Maßgabe, daß, wenn die Verbindung die Formel II

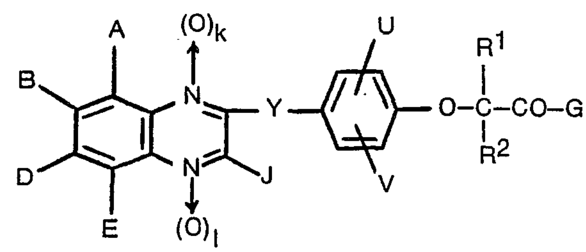

II

hat und A, B, D und E für Wasserstoff oder eines oder zwei der Symbole A, B, D und E für Halogen und die anderen für Wasserstoff stehen, J, U, V und $R^2$ für Wasserstoff stehen, $R^1$ für Wasserstoff oder $C_1$—$C_6$-Alkyl steht, Y für Sauerstoff steht und k und l 0 sind, G nicht für Hydroxy, $C_1$—$C_{10}$-Alkoxy, $C_1$—$C_{10}$-Halogenoalkoxy, $C_2$—$C_6$-Alkenyloxy, $C_3$—$C_6$-Alkinyloxy, Cyclohexyloxy, Phenoxy, Benzyloxy, die Gruppe $C_2$—$C_{10}$-Alkoxy, die mit Ausnahme ihres α-Kohlenstoffatoms mit $C_1$—$C_6$-Alkoxy substituiert ist, oder die Gruppe —OM, worin M die oben angegebene Bedeutung besitzt, steht.

3. Verbindung nach Anspruch 1 oder 2 der Formel II, worin A, B, D, E, J, U, V, $R^1$, $R^2$, G, Y, k und l die in Anspruch 1 angegebenen Bedeutungen besitzen.

4. Verbindung nach Anspruch 1, 2 oder 3, worin

A ausgewählt ist aus Wasserstoff und Halogen;

B ausgewählt ist aus Wasserstoff, Halogen, $C_1$—$C_6$-Alkyl und $C_1$—$C_6$-Halogenoalkyl;

D ausgewählt ist aus Wasserstoff, Halogen, $C_1$—$C_6$-Alkyl, $C_1$—$C_6$-Halogenoalkyl, Amino, N,N-Di($C_1$—$C_6$-alkyl)amino und Nitro;

E und V für Wasserstoff stehen;

J und U unabhängig ausgewählt sind aus Wasserstoff und Halogen;

$R^1$ ausgewählt ist aus $C_1$—$C_6$-Alkyl;

$R^2$ ausgewählt ist aus Wasserstoff und $C_1$—$C_6$-Alkyl;

G ausgewählt ist aus Hydroxy, $C_1$—$C_{10}$-Alkoxy, $C_2$—$C_{10}$-Alkenyloxy, $C_3$—$C_{10}$-Alkinyloxy, $C_1$—$C_{10}$-Alkylthio, Cyclohexyloxy, der Gruppe $C_2$—$C_{10}$-Alkoxy, die mit Ausnahme ihres α-Kohlenstoffatoms mit Amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion steht;

Y für Sauerstoff steht; und

k und l unabhängig ausgewählt sind aus 0 und 1 und k + 0 oder 1 ist;

5. Verbindung nach einem der Ansprüche 1 bis 4, worin

A, E, J, U, V und $R^2$ für Wasserstoff stehen;

B und D unabhängig ausgewählt sind aus Wasserstoff, Halogen und Methyl;

$R^1$ für Methyl steht;

G ausgewählt ist aus Hydroxy, $C_1$—$C_6$-Alkoxy, $C_2$—$C_6$-Alkenyloxy, $C_3$—$C_6$-Alkinyloxy, Cyclohexyloxy, der Gruppe $C_2$—$C_6$-Alkoxy, die mit Ausnahme ihres α-Kohlenstoffatoms mit N,N-Di($C_1$—$C_6$-alkyl)amino oder N,N,N-Tri($C_1$—$C_6$-alkyl)ammonio substituiert ist, und der Gruppe —OM, worin M für ein Alkalimetallion steht;

Y für Sauerstoff steht;

K 0 oder 1 ist, und

l 0 ist.

6. Verbindung nach einem der Ansprüche 1 bis 5, worin

A, B, E, J, U, V und $R^2$ für Wasserstoff stehen;

D ausgewählt ist aus Brom und Chlor;

$R^2$ für Methyl steht;

G ausgewählt ist aus Hydroxy, $C_1$—$C_6$-Alkoxy, Allyloxy, Cyclohexyloxy, 2-[N,N-Di($C_1$—$C_6$-alkyl)-amino]äthoxy, 2-[N,N,N-Tri($C_1$—$C_6$-alkyl)ammonio]äthoxy und der Gruppe —OM, worin M für Natrium oder Kalium steht;

Y für Sauerstoff steht;

k 0 oder 1 ist; und

l 0 ist.

7. Verbindung, welche der Methyl- oder Cyclohexylester von 2-[4-(6-Chloro-2-chinoxalinyloxy)-phenoxy]propionsäure oder der Cyclohexylester von 2-[4-(6-Bromo-2-chinoxalinyloxy)phenoxy]propionsäure ist.

8. Verbindung der Formel I

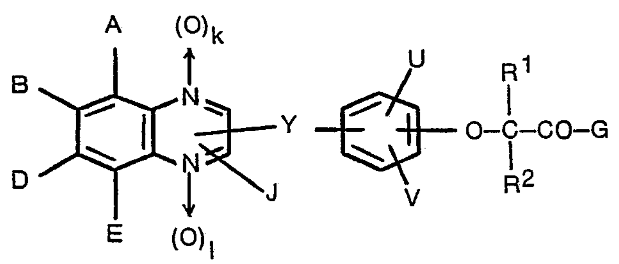

I

oder ein Salz davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, für Wasserstoff steht;

U und V unabhängig ausgewählt sind aus Wasserstoff und Halogen;

$R^1$ ausgewählt ist aus Wasserstoff, $C_1$—$C_6$-Alkyl und ($C_1$—$C_5$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy substituiert ist, und der Gruppe —OM, worin M für ein Alkalimetallion oder ein Erdalkalimetallion steht;

Y ausgewählt ist aus Sauerstoff und Schwefel; und

k und l jeweils 0 sind;

Mit der Maßgabe, daß, wenn die Verbindung die Formel II

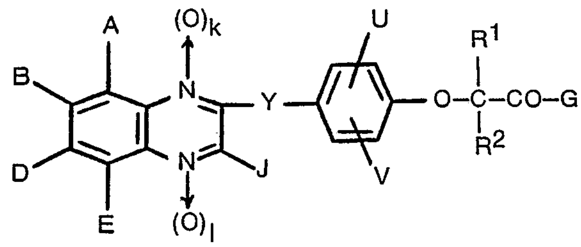

II

hat und A, B, D und E für Wasserstoff oder eines oder zwei der Symbole A, B, D und E für Halogen und die anderen für Wasserstoff stehen, U, V und $R^2$ für Wasserstoff stehen, $R^1$ für Wasserstoff oder $C_1$—$C_6$-Alkyl steht und Y für Sauerstoff steht, G nicht für Hydroxy, $C_1$—$C_{10}$-Alkoxy oder die Gruppe —OM, worin M die oben angegebene Bedeutung besitzt, steht.

9. Zusammensetzung mit herbicider Aktivität gegen einkeimblättrige Gräser in zweikeimblättrigen Anpflanzungen, dadurch gekennzeichnet, daß sie im wesentlichen aus einem Enantiomer der Formel I

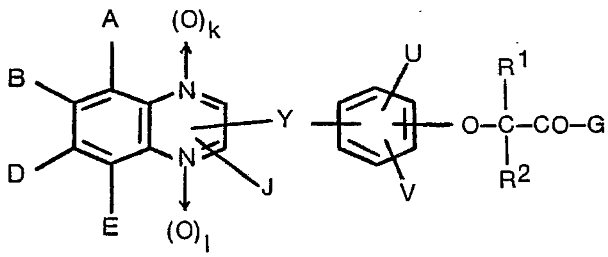

I

oder einem Salz davon besteht, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist;

wobei die Zusammensetzung im wesentlichen frei vom anderen Enantiomer ist.

10. Herbicide Zusammensetzung, welche als aktiven Bestandteil eine Verbindung nach einem der Ansprüche 1 bis 8 und einen Träger hierfür enthält.

11. Herbicide Zusammensetzung, welche als aktive Bestandteile folgendes enthält:

(a) eine Verbindung der Formel I

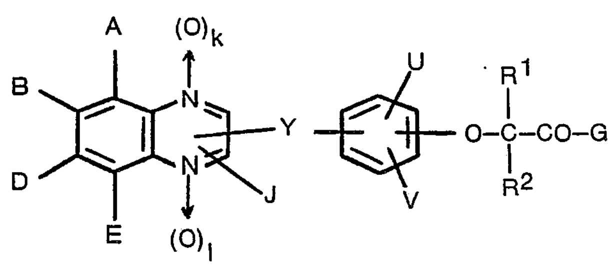

I

oder ein Salz davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die

mit Ausnahme ihres α-Kohlenstoffatoms mit $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist; und

(b) ein weiteres Herbicid, bei welchem es sich um eines der folgenden handelt: ein Benzo - 2,1,3 - thiadiazin - 4 - on - 2,2 - dioxid; ein Hormon-Herbicid; einen 3 - [4 - (4 - Halogenophenoxy)phenyl] - 1,1 - dialkylharnstoff; ein Dinitrophenol oder ein Derivat davon; ein Dinitroanilin-Herbicid; ein Phenylharnstoff-Herbicid; ein Phenylcarbamoyloxyphenylcarbamat; ein 2 - Phenylpyridazin - 3 - on; ein Uracil-Herbicid; ein Triazin-Herbicid; ein 1 - Alkoxy - 1 - alkyl - 3 - phenylharnstoff - Herbicid; ein Thiolcarbamat-Herbicid; ein 1,2,4 - Triazin - 5 - on - Herbicid; ein Benzoesäure-Herbicid; ein Anilid-Herbicid; ein Dihalogenobenzonitril-Herbicid; ein Halogenoalkansäure-Herbicid oder ein Salz davon; ein Diphenyläther-Herbicid (wie z. B. 4 - Nitrophenyl - 2 - nitro - 4 - trifluoromethylphenyl - äther, 5 - (2,4 - Dichlorophenoxy)-2-nitrobenzoesäure - methyl - ester, 2 - Nitro - 5 - (2 - chloro - 4 - trifluoromethylphenoxy)-benzoesäure, 2 - Chloro - 4 - trifluoromethylphenyl - 3 - äthoxy - 4 - nitrophenyl - äther oder 2 - Nitro - 5 - (2 - chloro - 4 - trifluoromethylphenoxy) - N - methansulfonylbenzamid); N,N-Dimethyldiphenylacetamid; N-(1-Naphthyl)phthalamidsäure; 3-Amino-1,2,4-triazol; ein Bipyridylium-Herbicid; ein Organoarsen-Herbicid; oder ein Aminosäure-Herbicid oder ein Salz oder einen Ester davon.

12. Verfahren zum starken Schädigen oder Abtöten von unerwünschten Pflanzen, bei welchem auf die Pflanzen oder auf das Wachstumsmedium der Pflanzen eine wirksame Menge einer Verbindung nach einem der Ansprüche 1 bis 8 oder eine wirksame Menge einer Zusammensetzung nach einem der Ansprüche 9 bis 11 aufgebracht wird.

13. Verfahren zur selektiven Bekämpfung des Wachstums von einkeimblättrigen Gräsern in zweikeimblättrigen Anpflanzungen, bei welchem auf die Anpflanzung oder auf das Wachstumsmedium der Anpflanzung eine Verbindung nach einem der Ansprüche 1 bis 8 oder eine Zusammensetzung nach einem der Ansprüche 9 bis 11 in einer Menge aufgebracht wird, die ausreicht, die Gräser stark zu schädigen oder abzutöten, die aber nicht ausreicht, die Anpflanzung wesentlich zu schädigen.

14. Verfahren nach Anspruch 12 oder 13, bei welchem die Verbindung in einer Rate im Bereich von 0,005 bis 20 kg/ha aufgebracht wird.

15. Verfahren zur Synthese einer Verbindung der Formel I von einem der Ansprüche 1 bis 8, bei welchem entweder ein Chinoxalinderivat der Formel IX mit einer Verbindung der Formel X

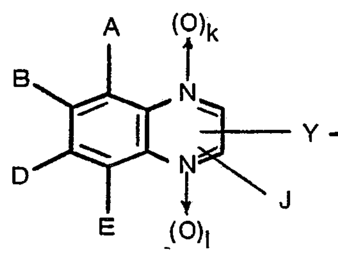

(IX)

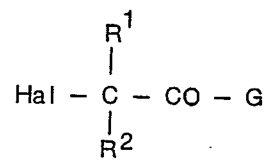

(X)

worin A, B, D, E, J, U, V, $R^1$, $R^2$, G, Y, k und l die in einem der Ansprüche 1 bis 6 und 8 angegebenen Bedeutungen besitzen und Hal für Chlor, Brom oder Jod steht, oder ein Chinoxalinderivat der Formel V mit einer Verbindung der Formel VI

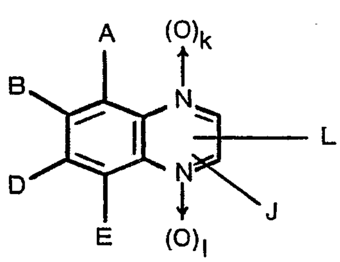

(V)

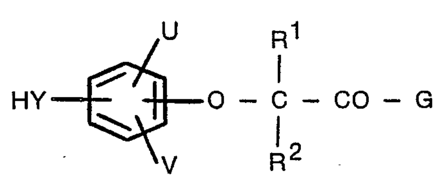

(VI)

worin A, B, D, E, J, U, V, $R^1$, $R^2$, G, Y, k und l die in einem der Ansprüche 1 bis 6 und 8 angegebenen Bedeutungen besitzen und L für eine abspaltbare Gruppe steht, umgesetzt wird.

16. Verfahren nach Anspruch 15, bei welchem die Chinoxalinverbindung der Formel IX dadurch hergestellt wird, daß ein Chinoxalinderivat der Formel V, worin A, B, D, E, J, k und l die in einem der Ansprüche 1

bis 6 und 8 angegebenen Bedeutungen besitzen und L für eine abspaltbare Gruppe steht, mit einem Phenol oder Thiophenol der Formel VII

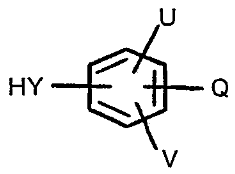

(VII)

worin U, V and Y die in einem der Ansprüche 1 bis 6 und 8 angegebenen Bedeutungen besitzen und Q für Hydroxy oder $C_1$—$C_6$-Alkoxy steht, umgesetzt wird, wobei eine Verbindung der Formel VIII

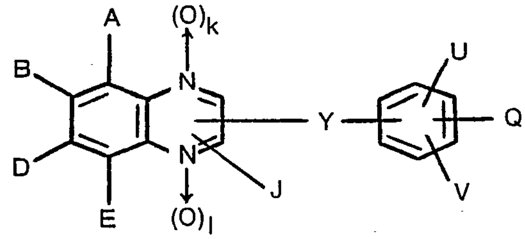

(VIII)

worin A, B, D, E, J, U, V, Y, k und l die in einem der Ansprüche 1 bis 6 und 8 angegebenen Bedeutungen besitzen und Q die oben angegebene Bedeutung besitzt, erhalten wird, und, wenn Q für $C_1$—$C_6$-Alkoxy steht, die Verbindung der Formel VIII dealkyliert wird, wobei eine Verbindung der Formel IX von Anspruch 15 erhalten wird.

17. Verfahren zur Synthese einer Verbindung der Formel I

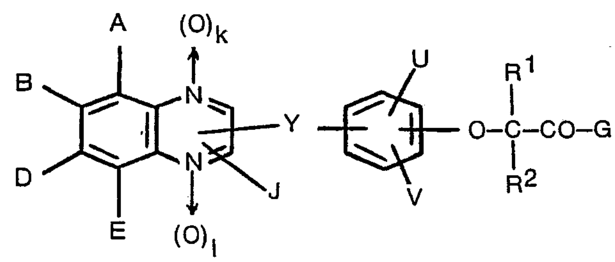

I

oder eines Salzes davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U and V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms durch $C_1$—$C_6$-Alkoxy, Amino, N-($C_1$—$C_6$-Alkyl)amino, N,N-Di($C_1$—$C_6$-alkyl)amino und N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist;

bei welchem eine Verbindung der Formel VIII

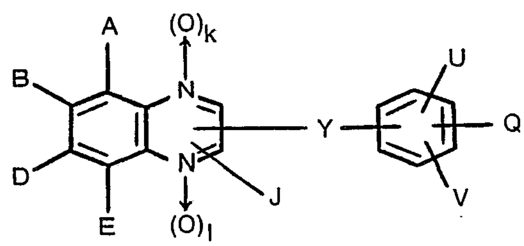

(VIII)

worin A, B, D, E, J, U, V, Y, k und l die oben angegebenen Bedeutungen besitzen und Q für $C_1$—$C_6$-Alkoxy steht, dealkyliert wird, wobei eine Verbindung der Formel IX

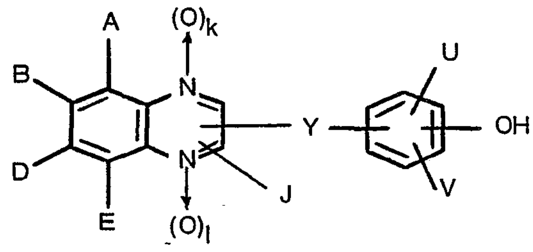

(IX)

worin A, B, D, E, J, U, V, Y, k und l die oben angegebenen Bedeutungen besitzen, erhalten wird, und hierauf die Verbindung der Formel IX mit einer Verbindung der Formel X

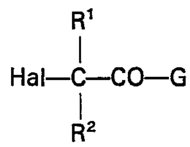

(X)

worin $R^1$, $R^2$ und G die oben angegebenen Bedeutungen besitzen und Hal für Chlor, Brom oder Jod steht, umgesetzt wird.

18. Verbindung der Formel VIII

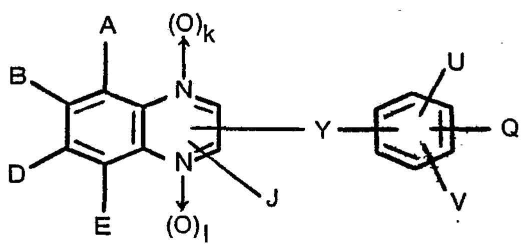

(VIII)

worin A, B, D, E, J, U, V, Y, k und l die in einem der Ansprüche 1 bis 6 und 8 angegebenen Bedeutungen besitzen und Q für Hydroxy oder $C_1$—$C_6$-Alkoxy steht, mit der Maßgabe, daß, wenn die Verbindung die Formel XIII.

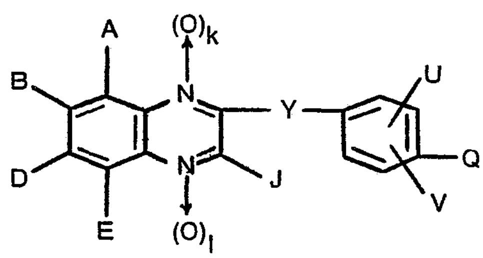

(XIII)

hat und A, B, D und E für Wasserstoff oder eines oder zwei der Symbole A, B, D und E für Halogen und die anderen für Wasserstoff stehen, J, U und V für Wasserstoff stehen, Y für Sauerstoff steht und k und l 0 sind, Q für $C_1$—$C_6$-Alkoxy steht.

**Patentansprüche für den Vertragsstaat: AT**

1. Verfahren zur Synthese einer Verbindung der Formel I

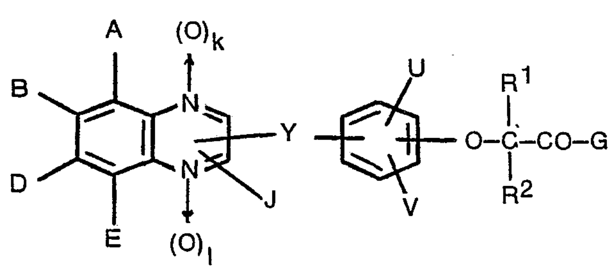

I

oder eines Salzes davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist;

mit der Maßgabe, daß, wenn die Verbindung die Formel II

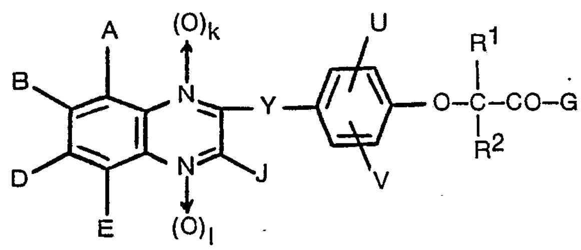

II

hat und A, B, D und E für Wasserstoff oder eines oder zwei der Symbole A, B, D und E für Halogen und die anderen für Wasserstoff stehen, J, U, V und $R^2$ für Wasserstoff stehen, $R^1$ für Wasserstoff oder $C_1$—$C_6$-Alkyl steht, Y für Sauerstoff steht und k und l 0 sind, G nicht für Hydroxy, $C_1$—$C_{10}$-Alkoxy oder die Gruppe—OM, worin M die oben angegebene Bedeutung besitzt, steht, aber mit der weiteren Maßgabe, daß die Verbindung der Methylester von 2-[4-(6-Chloro-2-chinoxalinyloxy)phenoxy]propionsäure sein kann, bei welchem Verfahren entweder ein Chinoxalinderivat der Formel IX mit einer Verbindung der Formel X

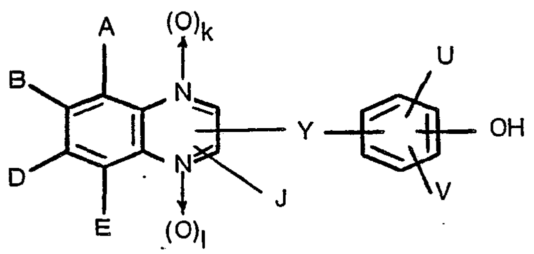

(IX)

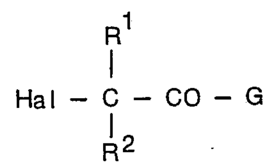

(X)

worin A, B, D, E, J, U, V, $R^1$, $R^2$, G, Y, k und l die oben angegebenen Bedeutungen besitzen und Hal für Chlor, Brom oder Jod steht, oder ein Chinoxalinderivat der Formel V mit einer Verbindung der Formel VI

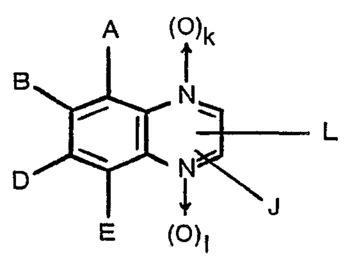

(V)

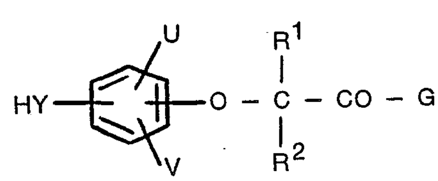

(VI)

worin A, B, D, E, J, U, V, $R^1$, $R^2$, G, Y, k und l die oben angegebenen Bedeutungen besitzen und L für eine abspaltbare Gruppe steht, umgesetzt wird.

2. Verfahren nach Anspruch 1, bei welchem die Chinoxalinverbindung der Formel IX dadurch hergestellt wird, daß ein Chinoxalinderivat der Formel V, worin A, B, D, E, J, k und l die in Anspruch 1 angegebenen Bedeutungen besitzen und L für eine abspaltbare Gruppe steht, mit einem Phenol oder Thiophenol der Formel VII

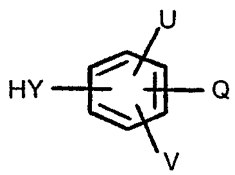

(VII)

worin U, V und Y die in Anspruch 1 angegebenen Bedeutungen besitzen und Q für Hydroxy oder $C_1$—$C_6$-Alkoxy steht, umgesetzt wird, wobei eine Verbindung der Formel VIII

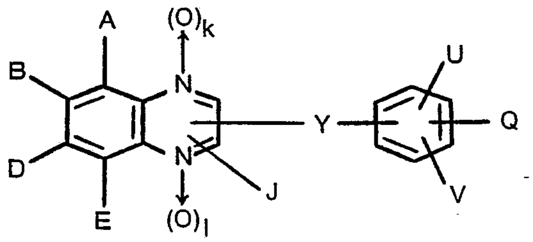

(VIII)

worin A, B, D, E, J, U, V, Y, k und l die in Anspruch 1 angegebenen Bedeutungen besitzen und Q die oben angegebene Bedeutung besitzt, erhalten wird, und, wenn Q für $C_1$—$C_6$-Alkoxy steht, die Verbindung der Formel VIII deaklyliert wird, wobei eine Verbindung der Formel IX von Anspruch 1 erhalten wird.

3. Verfahren zur Synthese einer Verbindung der Formel I

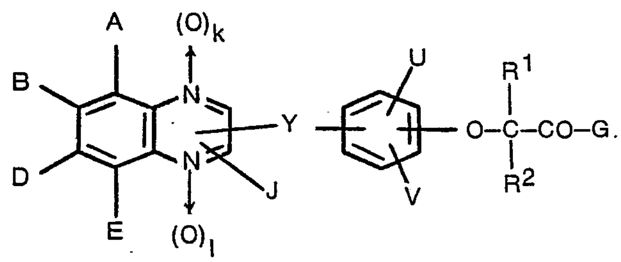

I

oder eines Salzes davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms durch $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist;

bei welchem eine Verbindung der Formel VIII

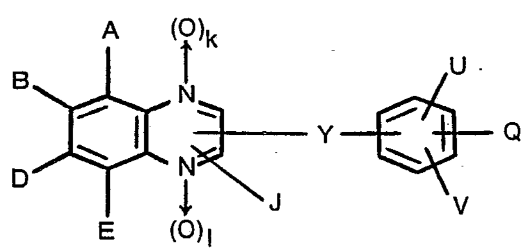

(VIII)

worin A, B, D, E, J, U, V, Y, k und l die oben angegebenen Bedeutungen besitzen und Q für $C_1$—$C_6$-Alkoxy steht, dealkyliert wird, wobei eine Verbindung der Formel IX

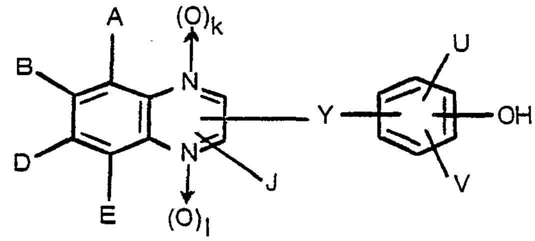

IX

worin A, B, D, E, J, U, V, Y, k und l die oben angegebenen Bedeutungen besitzen, erhalten wird, und hierauf die Verbindung der Formel IX mit einer Verbindung der Formel X

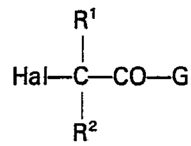

(X)

worin $R^1$, $R^2$ und G die oben angegebenen Bedeutungen besitzen und Hal für Chlor, Brom oder Jod steht, umgesetzt wird.

4. Verfahren nach einem der Ansprüche 1, 2 oder 3, bei welchem

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist;

mit der Maßgabe, daß, wenn das Verfahren für die Synthese einer Verbindung der Formel II

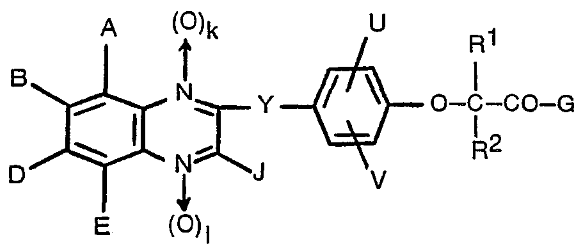

II

angewendet wird und A, B, D und E für Wasserstoff oder eines oder zwei der Symbole A, B, D und E für Halogen und die anderen für Wasserstoff stehen, J, U, V und $R^2$ für Wasserstoff stehen, $R^1$ für Wasserstoff oder $C_1$—$C_6$-Alkyl steht, Y für Sauerstoff steht und k und l 0 sind, G nicht für Hydroxy $C_1$—$C_{10}$-Alkoxy, $C_1$—$C_{10}$-Halogenoalkoxy, $C_2$—$C_6$-Alkenyloxy, $C_3$—$C_6$-Alkinyloxy, Cyclohexyloxy, Phenoxy, Benzyloxy, die Gruppe $C_2$—$C_{10}$-Alkoxy, die mit Ausnahme ihres α-Kohlenstoffatoms mit $C_1$—$C_6$-Alkoxy substituiert ist, oder die Gruppe —OM, worin M die oben angegebene Bedeutung besitzt, steht.

5. Verfahren nach einem der vorhergehenden Ansprüche zur Synthese einer Verbindung der Formel II, worin A, B, D, E, J, U, V, $R^1$, $R^2$, G, Y, k und l die in Anspruch 1 angegebenen Bedeutungen besitzen.

6. Verfahren nach einem der vorhergehenden Ansprüche, bei welchem

A ausgewählt ist aus Wasserstoff und Halogen;

B ausgewählt ist aus Wasserstoff, Halogen, $C_1$—$C_6$-Alkyl und $C_1$—$C_6$-Halogenoalkyl;

D ausgewählt ist aus Wasserstoff, Halogen, $C_1$—$C_6$-Alkyl, $C_1$—$C_6$-Halogenoalkyl, Amino, N,N-Di($C_1$—$C_6$-alkyl)amino und Nitro;

E und V für Wasserstoff stehen;

J und U unabhängig ausgewählt sind aus Wasserstoff und Halogen;

$R^1$ ausgewählt ist aus $C_1$—$C_6$-Alkyl;

$R^2$ ausgewählt ist aus Wasserstoff und $C_1$—$C_6$-Alkyl;

G ausgewählt ist aus Hydroxy, $C_1$—$C_{10}$-Alkoxy, $C_2$—$C_{10}$-Alkenyloxy, $C_3$—$C_{10}$-Alkinyloxy, $C_1$—$C_{10}$-Alkylthio, Cyclohexyloxy, der Gruppe $C_2$—$C_{10}$-Alkoxy, die mit Ausnahme ihres α-Kohlenstoffatoms mit Amino, N,N-Di($C_1$—$C_6$-alkyl)amino oder N,N,N-Tri($C_1$—$C_6$-alkyl)ammonio substituiert

ist, der Gruppe —O—N=$R^6$, worin $R^6$ für $C_1$—$C_{10}$-Alkyliden steht, und der Gruppe —OM, worin M für ein Alkalimetallion steht;

Y für Sauerstoff steht; und

k und l unabhängig ausgewählt sind aus 0 und 1 und k + l 0 oder 1 ist.

7. Verfahren nach einem der vorhergehenden Ansprüche, bei welchem A, E, J, U, V und $R^2$ für Wasserstoff stehen;

B und D unabhängig ausgewählt sind aus Wasserstoff, Halogen und Methyl;

$R^1$ für Methyl steht;

G ausgewählt ist aus Hydroxy, $C_1$—$C_6$-Alkoxy, $C_2$—$C_6$-Alkenyloxy, $C_3$—$C_6$-Alkinyloxy, Cyclohexyloxy, der Gruppe $C_2$—$C_6$-Alkoxy, die mit Ausnahme ihres α-Kohlenstoffatoms mit N,N-Di($C_1$—$C_6$-alkyl)amino oder N,N,N-Tri($C_1$—$C_6$-alkyl)ammonio substituiert ist, und der Gruppe —OM, worin M für ein Alkalimetallion steht;

Y für Sauerstoff steht;

k 0 oder 1 ist; und

l 0 ist.

8. Verfahren nach einem der vorhergehenden Ansprüche, bei welchem

A, B, E, J, U, V und $R^2$ für Wasserstoff stehen;

D ausgewählt ist aus Brom und Chlor;

$R^2$ für Methyl steht;

G ausgewählt ist aus Hydroxy, $C_1$—$C_6$-Alkoxy, Allyloxy, Cyclohexyloxy, 2-[N,N-Di($C_1$—$C_6$-alkyl)-amino]äthoxy, 2-[N,N,N-Tri($C_1$—$C_6$-alkyl)ammonio]äthoxy und der Gruppe —OM, worin M für Natrium oder Kalium steht;

Y für Sauerstoff steht;

k 0 oder 1 ist; und

l 0 ist.

9. Verfahren nach einem der vorhergehenden Ansprüche, bei welchem die Reaktionsteilnehmer so gewählt werden, daß eine Verbindung erhalten wird, die der Methyl- oder Cylcohexylester von 2-[4-(6-Chloro-2-chinoxalinyloxy)phenoxy]propionsäure oder der Cyclohexylester von 2-[4-(6-Bromo-2-chinoxalinyloxy)-phenoxy]propionsäure ist.

10. Verfahren zur Synthese einer Verbindung der Formel I

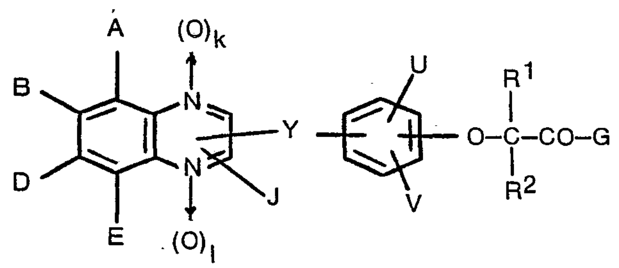

I

oder eines Salzes davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J für Wasserstoff steht;

U und V unabhängig ausgewählt sind aus Wasserstoff und Halogen;

$R^1$ ausgewählt ist aus Wasserstoff, $C_1$—$C_6$-Alkyl und ($C_1$—$C_5$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy substituiert ist, und der Gruppe —OM worin M für ein Alkalimetallion oder ein Erdalkalimetallion steht;

Y ausgewählt ist aus Sauerstoff und Schwefel; und

k und l jeweils 0 sind;

mit der Maßgabe, daß, wenn die Verbindung die Formel II

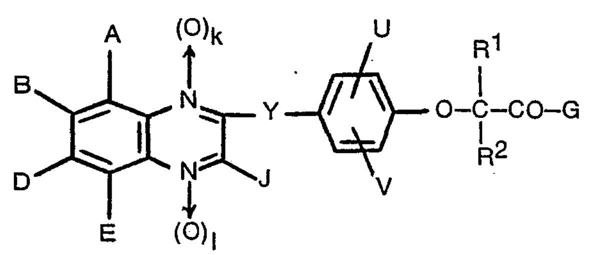

II

hat und A, B, D und E für Wasserstoff oder eines oder zwei der Symbole A, B, D und E für Halogen und die

anderen für Wasserstoff stehen, U, V und $R^2$ für Wasserstoff stehen, $R^1$ für Wasserstoff oder $C_1$—$C_6$-Alkyl steht und Y für Sauerstoff steht, G nicht für Hydroxy, $C_1$—$C_{10}$-Alkoxy oder die Gruppe —OM, worin M die oben angegebene Bedeutung besitzt, steht;
bei welchem Verfahren entweder ein Chinoxalinderivat der Formel IX mit einer Verbindung der Formel X

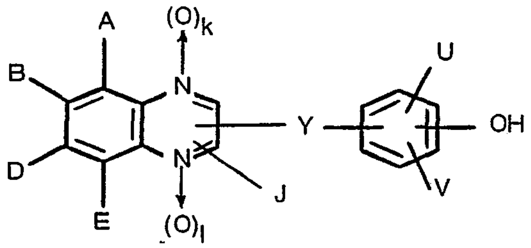

(IX)

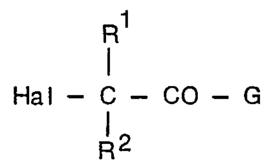

(X)

worin A, B, D, E, J, U, V, $R^1$, $R^2$, G, Y, k und l die oben angegebenen Bedeutungen besitzen und Hal für Chlor, Brom oder Jod steht, umgesetzt wird oder ein Chinoxalinderivat der Formel V mit einer Verbindung der Formel VI

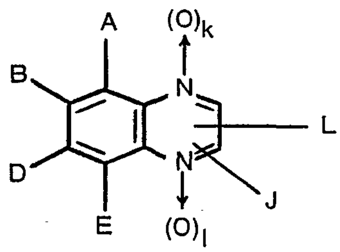

(V)

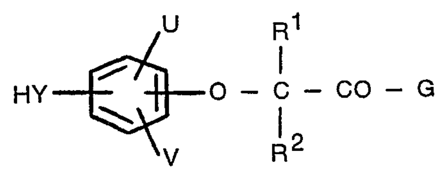

(VI)

worin A, B, D, E, J, U, V, $R^1$, $R^2$, G, Y, k und l die oben angegebenen Bedeutungen besitzen und L für eine abspaltbare Gruppe steht, umgesetzt wird.

11. Verfahren zur Synthese einer Zusammensetzung mit einer herbiciden Aktivität gegen einkeimblättrige Gräser in zweikeimblättrigen Anpflanzungen, dadurch gekennzeichnet, daß sie im wesentlichen aus einem Enantiomer der Formel I

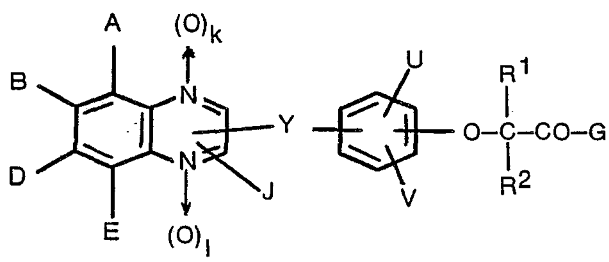

I

oder einem Salz davon besteht, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist;

wobei die Zusammensetzung im wesentlichen frei vom anderen Enantiomer ist;
bei welchem entweder ein Chinoxalinderivat der Formel IX mit einer Verbindung der Formel X

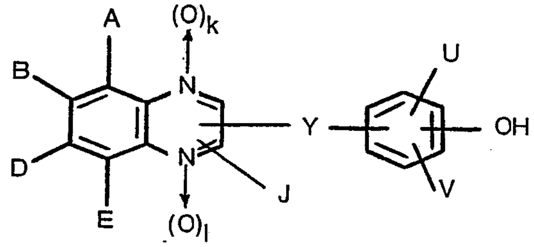

(IX)

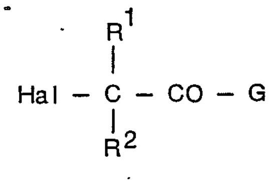

(X)

worin A, B, D, E, J, U, V, $R^1$, $R^2$, G, Y, k und l die oben angegebenen Bedeutungen besitzen und Hal für Chlor, Brom oder Jod steht, umgesetzt wird oder ein Chinoxalinderivat der Formel V mit einer Verbindung der Formel VI

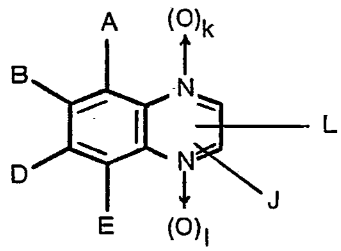

(V)

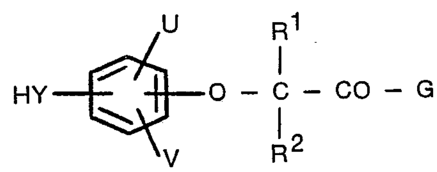

(VI)

worin A, B, D, E, J, U, V, $R^1$, $R^2$, G, Y, k und l die oben angegebenen Bedeutungen besitzen und L für eine abspaltbare Gruppe steht, umgesetzt wird.

12. Herbicide Zusammensetzung, welche als aktiven Bestandteil eine Verbindung der Formel I oder II, wie sie in einem der Ansprüche 1 bis 10 definiert sind, und einen Träger hierfür enthält.

13. Herbicide Zusammensetzung, welche als aktive Bestandteile folgendes enthält:

(a) eine Verbindung der Formel I

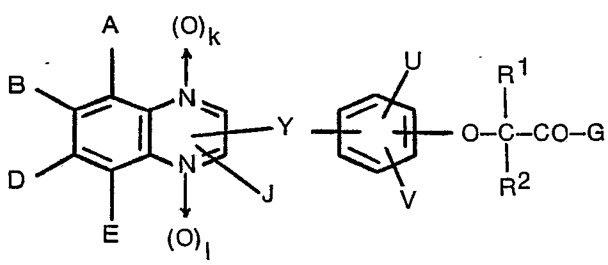

I

oder ein salz davon, worin

A, B, D und E unabhängig ausgewählt sind aus Wasserstoff, Halogen, Nitro, $C_1$—$C_6$-Alkyl, $C_1$—$C_6$-Halogenoalkyl, Amino, $C_1$—$C_6$-Alkylamino und Di($C_1$—$C_6$-alkyl)amino;

J, U und V unabhängig ausgewählt sind aus Wasserstoff und Halogen;

$R^1$ ausgewählt ist aus Wasserstoff, $C_1$—$C_6$-Alkyl und ($C_1$—$C_6$-Alkoxy)carbonyl;

$R^2$ ausgewählt ist aus Wasserstoff und $C_1$—$C_6$-Alkyl;

G ausgewählt ist aus Hydroxy, $C_1$—$C_{10}$-Alkoxy, $C_1$—$C_{10}$-Halogenoalkoxy, $C_2$—$C_{10}$-Alkenyloxy, $C_3$—$C_{10}$-Alkinyloxy, $C_1$—$C_{10}$-Alkylthio, Cyclohexyloxy, Phenoxy, Benzyloxy, der Gruppe $C_2$—$C_{10}$-Alkoxy, die mit Ausnahme ihres α-Kohlenstoffatoms mit $C_1$—$C_6$-Alkoxy, Amino, N-($C_1$—$C_6$-Alkyl)amino, N,N-Di($C_1$—$C_6$-alkyl)amino oder N,N,N-Tri($C_1$—$C_6$-alkyl)ammonio substituiert ist, der Gruppe —O—N=$R^6$, worin $R^6$ für $C_1$—$C_{10}$-Alkyliden steht, und der Gruppe —OM worin M für ein Alkalimetallion oder ein Erdalkalimetallion steht;

Y ausgewählt ist aus Sauerstoff und Schwefel; und

k und l unabhängig ausgewählt sind aus 0 und 1 und k + l 0 oder 1 ist; und

(b) ein weiteres Herbicid, bei welchem es sich um eines der folgenden handelt: ein Benzo - 2,1,3 - thiadiazin - 4 - on - 2,2 - dioxid; ein Hormon-Herbicid; einen 3 - [4 - (4 - Halogenophenoxy)phenyl] - 1,1 - dialkylharnstoff; ein Dinitrophenol oder ein Derivat davon; ein Dinitroanilin-Herbicid; ein Phenylharnstoff-Herbicid; ein Phenylcarbamoyloxyphenylcarbamat; ein 2 - Phenylpyridazin - 3 - on; ein Uracil-Herbicid; ein Triazin-Herbicid; ein 1 - Alkoxy - 1 - alkyl - 3 - phenylharnstoff - Herbicid; ein Thiolcarbamat-Herbicid; ein 1,2,4 - -Triazin - 5 - on - Herbicid; ein Benzoesäure-Herbicid; ein Anilid-Herbicid;

ein Dihalogenobenzonitril-Herbicid; ein Halogenoalkansäure-Herbicid oder ein Salz davon; ein Diphenyläther-Herbicid (wie z. B. 4 - Nitrophenyl - 2 - nitro - 4 - trifluoromethylphenyl - äther, 5 - (2,4 - Dichlorophenoxy)-2-nitrobenzoesäure - methyl - ester, 2 - Nitro - 5 - (2 - chloro - 4 - trifluoromethylphenoxy)-benzoesäure, 2 - Chloro - 4 - trifluoromethylphenyl - 3 - äthoxy - 4 - nitrophenyl - äther oder 2 - Nitro - 5 - (2 - chloro - 4 - trifluoromethylphenoxy) - N - methansulfonylbenzamid); N,N-Dimethyldiphenylacetamid; N-(1-Naphthyl)phthalamidsäure; 3-Amino-1,2,4-triazol; ein Bipyridylium-Herbicid; ein Organoarsen-Herbicid; oder ein Aminosäure-Herbicid oder ein Salz oder einen Ester davon.

14. Verfahren zum starken Schädigen oder Abtöten von unerwünschten Pflanzen, bei welchem auf die Pflanzen oder auf das Wachstumsmedium der Pflanzen eine wirksame Menge einer Verbindung nach einem der Ansprüche 1 bis 10 oder eine wirksame Menge einer Zusammensetzung nach einem der Ansprüche 11 bis 13 aufgebracht wird.

15. Verfahren zur selektiven Bekämpfung des Wachstums von einkeimblättrigen Gräsern in zweikeimblättrigen Anpflanzungen, bei welchem Verfahren auf die Anpflanzung oder auf das Wachstumsmedium der Anpflanzung eine Verbindung nach einem der Ansprüche 1 bis 10 oder eine Zusammensetzung nach einem der Ansprüche 11 bis 13 in einer Menge aufgebracht wird, die ausreicht, die Gräser stark zu schädigen oder abzutöten, die aber nicht ausreicht, die Anpflanzung wesentlich zu schädigen.

16. Verfahren nach Anspruch 14 oder 15, bei welchem die Verbindung in einer Rate im Bereich von 0,005 bis 20 kg/ha aufgebracht wird.

17. Verfahren zur Synthese einer Verbindung der Formel VIII

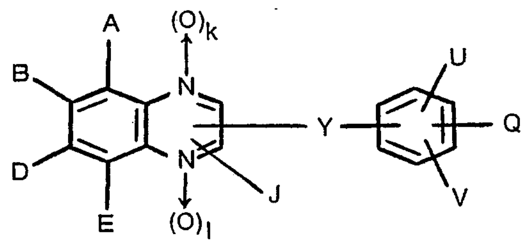

(VIII)

worin A, B, D, E, J, U, V, Y, k und l die in einem der Ansprüche 1 bis 8 und 10 angegebenen Bedeutungen besitzen und Q für Hydroxy oder $C_1$—$C_6$-Alkoxy steht;
bei welchem Verfahren ein Chinoxalinderivat der Formel V

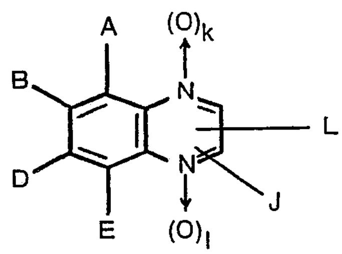

(V)

worin A, B, D, E, J, k und l die oben angegebenen Bedeutungen besitzen und L für eine abspaltbare Gruppe steht, mit einem Phenol oder Thiophenol der Formel VII

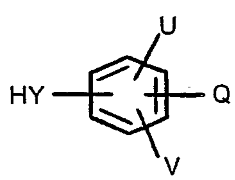

(VII)

worin U, V, Y und Q die oben angegebenen Bedeutungen besitzen, umgesetzt wird.

**Revendications pour les Etats contractants: BE CH DE FR GB IT LI NL SE**

1. Composé de formule I

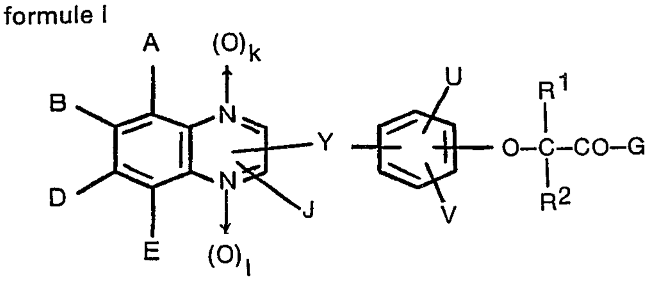

I

ou un sel de ce composé, formule dans laquelle

A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atomes α de carbone avec un substituant choisi entre des substituants alkoxy en $C_1$ à $C_6$, amino, N-(alkyle en $C_1$ à $C_6$)amino, N,N-di-(alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe —OM dans lequel M est un ion de métal alcalin ou un ion de métal alcalinoterreux;

Y est choisi entre l'oxygène et le soufre; et

k et l sont choisis indépendamment entre 0 et 1 et la somme k+l est choisie entre 0 et 1;

sous réserve que lorsque le composé répond à la formule II:

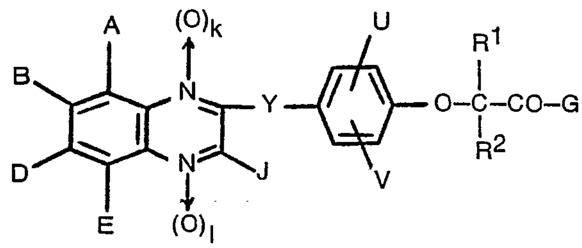

II

et que A, B, D et E représentent l'hydrogène, ou qu'un ou deux de A, B, D et E représentent un halogène et les autres représentent l'hydrogène, J, U, V et $R^2$ représentent de l'hydrogène, $R^1$ représente l'hydrogène ou un groupe alkyle en $C_1$ à $C_6$, Y représente l'oxygène et k et l ont la valeur 0, G ne soit pas un groupe hydroxy, alkoxy en $C_1$ à $C_{10}$ ou le groupe OM dans lequel M a la définition donnée ci-dessus, mais sous réserve en outre que le composé puisse être l'ester méthylique de l'acide 2-[4-(6-chloro-2-quinoxalinyloxy)-phénoxy]propionique.

2. Composé de formule I

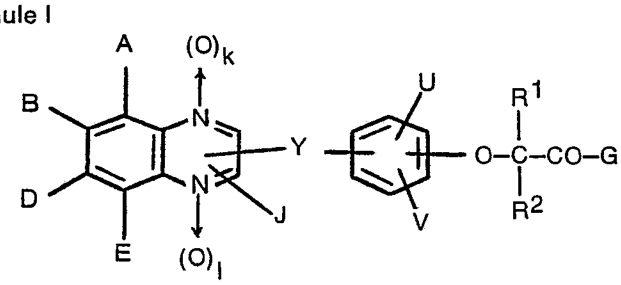

I

ou un sel de ce composé, formule dans laquelle A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α de carbone avec un substituant choisi entre des substituants alkoxy en $C_1$ à $C_6$, amino, N-(alkyle en $C_1$ à $C_6$)amino, N,N-di-(alkyle en $C_1$ à $C_6$)amino et N,N,N-tri(alkyle en $C_1$ à $C_6$)ammonio, le groupe —O—N=$R^6$ dans lequel $R^6$1est un radical alkylidène en $C_1$ à $C_{10}$, et le groupe —OM dans lequel M est un ion de métal alcalin ou un ion métal alcalino-terreux;

Y est choisi entre l'oxygène et le soufre; et

k et l sont choisis indépendamment entre 0 et 1 et la somme k+l est égale à 0 ou à 1;

sous réserve que lorsque le composé répond à la formule II:

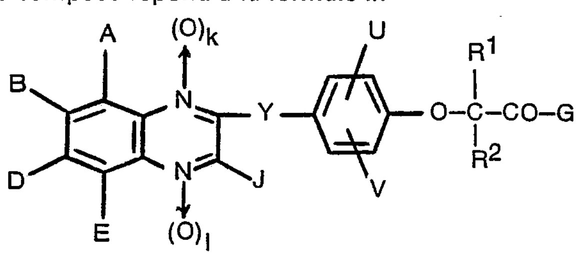

II

et que A, B, D et E représentent l'hydrogène, ou bien un ou deux de A, B, D et E représentent un halogène et

les autres représentent l'hydrogène, J, U, V et $R^2$ représentent l'hydrogène, $R^1$ représente l'hydrogène ou un groupe alkyle en $C_1$ à $C_6$, Y représente l'oxygène et k et l sont égaux à 0, G ne représente pas un groupe hydroxy, alkoxy en $C_1$ à $C_{10}$, halogénalkoxy en $C_1$ à $C_{10}$, alcényloxy en $C_2$ à $C_6$, alcynyloxy en $C_3$ à $C_6$, cyclohexyloxy, phénoxy, benzyloxy, le groupe alkoxy en $C_2$ à $C_{10}$ substitué ailleurs que sur son atome α de carbone avec le substituant alkoxy en $C_1$ à $C_6$, ou le groupe OM dans lequel M a la définition donnée ci-dessus.

3. Composé suivant la revendication 1 ou 2, de formule II dans laquelle A, B, D, E, J, U, V, $R^1$, $R^2$, G, Y, k et l ont les définitions données dans la revendication 1.

4. Composé suivant la revendication 1, 2 ou 3, dans lequel

A est choisi entre l'hydrogène et un halogène;

B est choisi entre l'hydrogène, un halogène, un groupe alkyle en $C_1$ à $C_6$ et un groupe halogénalkyle en $C_1$ à $C_6$;

D est choisi entre l'hydrogène, un halogène, un groupe alkyle en $C_1$ à $C_6$, halogénalkyle en $C_1$ à $C_6$, amino, N,N-di(alkyle en $C_1$ à $C_6$)amino et nitro;

E et V sont l'hydrogène;

J et U sont choisis indépendamment entre l'hydrogène et un halogène;

$R^1$ est un groupe alkyle en $C_1$ à $C_6$.

$R^2$ est choisi entre l'hydrogène et un groupe alkyle en $C_1$ à $C_6$;

G est choisi entre un groupe hydroxy, alkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, le groupe alkoxy en $C_2$ à $C_{10}$ substitué ailleurs que sur son atome α de carbone avec un substituant choisi entre les substituants amino, N,N-di(alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe OM dans lequel M est un ion de métal alcalin;

Y est l'oxygène; et

k et l sont choisis indépendamment entre 0 et 1 et la somme k+l a la valeur 0 ou 1.

5. Composé suivant l'une quelconque des revendications 1 à 4 inclus, dans lequel:

A, E, J, U, V et $R^2$ sont l'hydrogène;

B et D sont choisis indépendamment entre l'hydrogène, un halogène et le groupe méthyle.

$R^1$ est le groupe méthyle;

G est choisi entre un groupe hydroxy, alkoxy en $C_1$ à $C_6$, alcényloxy en $C_2$ à $C_6$, alcynyloxy en $C_3$ à $C_6$, cyclohexyloxy, le groupe alkoxy en $C_2$ à $C_6$ substitué ailleurs que sur son atome α de carbone avec un substituant choisi entre des substituants N,N-di(alkyle en $C_1$ à $C_6$)amino et N,N,N-tri(alkyle en $C_1$ à $C_6$)ammonio et le groupe OM dans lequel M est un ion de métal alcalin;

Y est l'oxygène;

k a la valeur 0 ou 1; et

l a la valeur 0.

6. Composé suivant l'une quelconque des revendications 1 à 5 inclus, dans lequel:

A, B, E, J, U, V et $R^2$ sont l'hydrogène;

D est choisi entre le brome et le chlore;

$R^2$ est le groupe méthyle;

G est choisi entre un groupe hydroxy, alkoxy en $C_1$ à $C_6$, allyloxy, cyclohexyloxy, 2-[N,N-di(alkyle en $C_1$ à $C_6$)amino]-éthoxy, 2-[N,N,N-tri(alkyle en $C_1$ à $C_6$)ammonio]-éthoxy et le groupe OM dans lequel M est le sodium ou le potassium;

Y est l'oxygène;

k a la valeur 0 ou 1; et

l a la valeur 0.

7. Composé qui est l'ester de méthyle ou de cyclohexyle de l'acide 2-[4-(6-chloro-2-quinoxalinyloxy)phénoxy]propionique ou l'ester de cyclohexyle de l'acide 2-[4-(6-bromo-2-quinoxalinyloxy)phénoxy]-propionique.

8. Composé de formule I

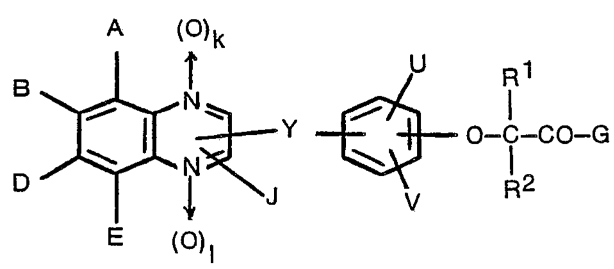

I

ou un sel de ce composé, formule dans laquelle

A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J est l'hydrogène;

U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_5$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, phénoxy, benzyloxy, le groupe alkoxy en $C_2$ à $C_{10}$ substitué ailleurs que sur son atome α de carbone avec un radical alkoxy en $C_1$ à $C_6$, et le groupe OM dans lequel M est un ion de métal alcalin ou un ion de métal alcalino-terreux;

Y est choisi entre l'oxygène et le soufre; et

k et l ont chacun la valeur 0:

sous réserve que lorsque le composé répond à la formule II

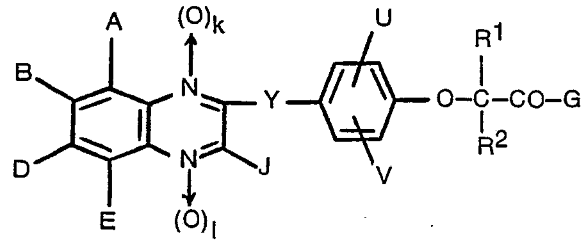

II

et que A, B, D et E sont l'hydrogène, ou bien un ou deux de A, B, D, et E représentent un halogène et les autres représentent l'hydrogène, U, V et $R^2$ sont l'hydrogène, $R^1$ est l'hydrogène ou un groupe alkyle en $C_1$ à $C_6$ et Y est l'oxygène, G ne représente pas un groupe hydroxy, alkoxy en $C_1$ à $C_{10}$ ou le groupe OM dans lequel M a la définition donnée ci-dessus;

9. Composition douée d'activité herbicide contre des mauvaises herbes monocotylédones dans des cultures dicotylédones, caractérisée en ce qu'elle est essentiellement formée d'un énantiomère de formule I

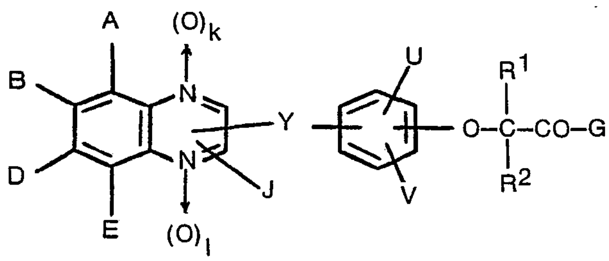

I

ou d'un sel de cet énantiomère,
formule dans laquelle

A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α carbone avec un substituant choisi entre un groupe alkoxy en $C_1$ à $C_6$, amino, N-(alkyle en $C_1$ à $C_6$)amino, N,N-di-(alkyle en $C_1$ à $C_6$)amino et N,N-tri(alkyle en $C_1$ à $C_6$)ammonio, le groupe $—O—N=R^6$ dans lequel $R^6$ est un radical alkylidène en $C_1$ à $C_{10}$, et le groupe —OM dans lequel M est un ion de métal alcalin ou un ion de métal alcalino-terreux;

Y est choisi entre l'oxygène et le soufre; et

k et l sont choisis indépendamment entre 0 et 1 et la somme k+l a la valeur 0 ou 1.

ladite composition étant pratiquement dépourvue de l'autre énantiomère.

10. Composition herbicide, comprenant comme ingrédient actif un composé suivant l'une quelconque des revendications 1 à 8 inclus et un support approprié.

11. Composition herbicide, comprenant comme ingrédients actifs (a) un composé de formule (I)

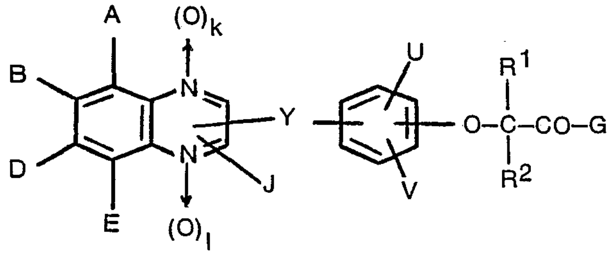

I

ou un sel de ce composé, formule dans laquelle A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α de carbone avec un substituant choisi entre des substituants alkoxy en $C_1$ à $C_6$, amino, N-(alkyle en $C_1$ à $C_6$)amino, N,N-di-(alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe —OM dans lequel M est un ion de métal alcalin ou un ion métal alcalino-terreux;

Y est choisi entre l'oxygène et le soufre; et

k et l sont choisis indépendamment entre 0 et 1 et la somme k+l a la valeur 0 ou 1;

et (b) un autre herbicide qui est un 2,2-dioxyde de benzo-2,1,3-thiadiazine-4-one; un herbicide hormonal; une 3-[4-(4-halogénophénoxy)phényl]-1,1-dialkylurée; un dinitrophénol ou l'un de ses dérivés; un herbicide du type dinitroaniline; un herbicide du type phénylurée; un phénylcarbamoyloxyphénylcarbamate; une 2-phénylpyridazine-3-one; un herbicide du type uracile; un herbicide triazinique; un herbicide du type 1-alkoxy-1-alkyl-3-phénylurée; un herbicide du type thiolcarbamate; un herbicide du type 1,2,4-triazine-5-one; un herbicide du type de l'acide benzoïque; un herbicide du type anilide; un herbicide dihalogénobenzonitrilique; un herbicide du type acide halogénalcanoïque ou d'un sel de cet acide; un herbicide du type éther de diphényle (par exemple l'éther de 4-nitrophényle et de 2-nitro-4-trifluorométhylphényle, le 5-(2,4-dichlorophénoxy)-2-nitrobenzoate de méthyle, l'acide 2-nitro-5-(2-chloro-4-trifluorométhylphénoxy)-benzoïque, l'éther de 2-chloro-4-trifluorométhylphényle et de 3-éthoxy-4-nitrophényle ou le 2-nitro-5-(2-chloro-4-trifluorométhylphénoxy)-N-méthanesulfonylbenzamide); le N,N-diméthyldiphénylacétamide; l'acide N-(1-naphtyl)-phtalamique; le 3-amino-1,2,4-triazole; un herbicide du type bipyridylium; un herbicide organoarsénical ou un herbicide du type d'un aminoacide ou d'un sel ou ester de cet acide.

12. Procédé pour endommager gravement ou détruire des plantes indésirables, procédé qui consiste à appliquer auxdites plantes ou aux milieux où elles se développent, une quantité efficace d'un composé suivant l'une quelconque des revendications 1 à 8 inclus ou une quantité efficace d'un composition telle que définie conformément à l'une quelconque des revendications 9 à 11.

13. Procédé pour combattre sélectivement la croissance de mauvaises herbes monocotylédones dans des cultures dicotylédones, procédé qui consiste à appliquer auxdites cultures ou aux milieux où elles se développent, un composé tel que défini conformément à l'une quelconque des revendications 1 à 8 inclus ou une composition telle que définie conformément à l'une quelconque des revendications 9 à 11 en une quantité suffisante pour endommager gravement ou détruire les mauvaises herbes mais insuffisante pour endommager sensiblement la plante cultivée.

14. Procédé suivant la revendication 12 ou 13, dans laquelle le composé est appliqué à un taux compris dans l'intervalle de 0,005 à 20 kg par hectare.

15. Procédé de synthèse d'un composé de formule I suivant l'une quelconque des revendications 1 à 8 inclus, procédé qui consiste à faire réagir un dérivé de quinoxaline de formule IX avec un composé de formule X

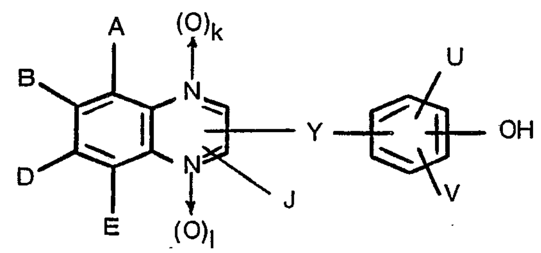

(IX)

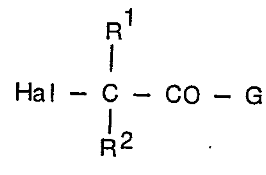

(X)

ou A, B, D, E, J, U, V, $R^1$, $R^2$, G, Y, k et l ont les définitions données dans l'une quelconque des revendications 1 à 6 et 8 et Hal désigne le chlore, le brome ou l'iode, ou à faire réagir un dérivé de quinoxaline de formule V avec un composé de formule VI

(V)

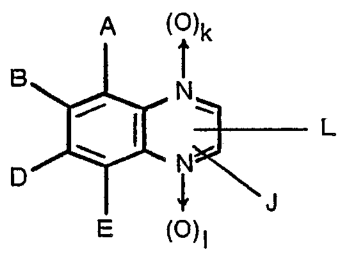

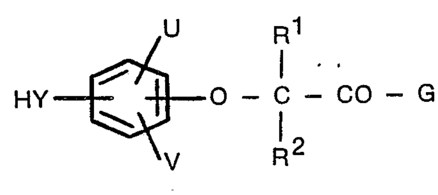

(VI)

où A, B, D, E, J, U, V, $R^1$, $R^2$, G, Y, k et l ont les définitions données dans l'une quelconque des revendications 1 à 6 et 8 et L est un groupe partant.

16. Procédé suivant la revendication 15, dans lequel le composé de quinoxaline de formule IX est préparé par reaction d'un dérivé de quinoxaline de formule V dans laquelle A, B, D, E, J, k et l ont les définitions données dans l'une quelconque des revendications 1 à 6 et 8 et L est un groupe partant, avec un phénol ou un thiophénol de formule VII

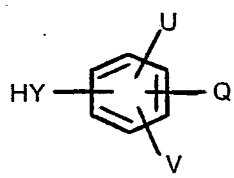

(VII)

où U, V et Y ont les définitions données dans l'une quelconque des revendications 1 à 6 et 8 et Q est un groupe hydroxy ou alkoxy en $C_1$ à $C_6$ pour former un composé de formule VIII

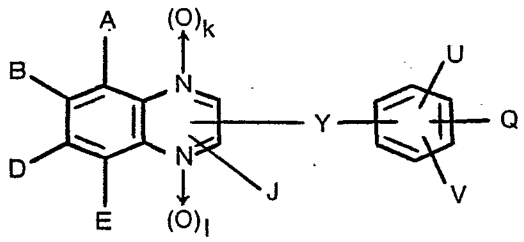

(VIII)

dans laquelle A, B, D, E, J, U, V, Y, k et l ont les définitions données dans l'une quelconque des revendications 1 à 6 et 8 et Q a la définition donnée ci-dessus, et lorsque Q est un groupe alkoxy en $C_1$ à $C_6$, désalkylation du composé de formule VIII pour former un composé de formule IX telle que définie dans la revendication 15.

17. Procédé de synthèse d'un composé de formule I

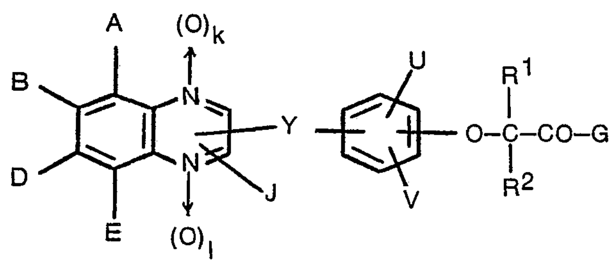

I

ou d'un sel de ce composé, formule dans laquelle A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α de carbone avec un substituant choisi entre des substituants alkoxy en $C_1$ à $C_6$, amino, N-(alkyle en $C_1$ à $C_6$)amino, N,N-di-(alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe —OM dans lequel M est un ion de métal alcalin ou un ion métal alcalino-terreux;

Y est choisi entre l'oxygène et le soufre; et

k et l sont choisis indépendamment entre 0 et 1 et la somme k+l a la valeur 0 et 1;

qui consiste à désalkyler un composé de formule VIII

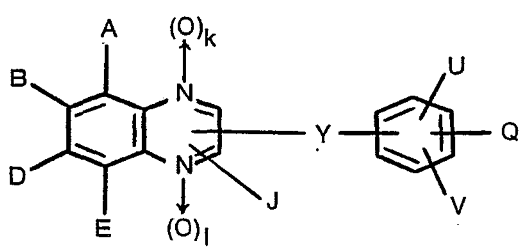

(VIII)

dans laquelle A, B, D, E, J, U, V, Y, k et l ont les définitions données ci-dessus et Q est un groupe alkoxy en $C_1$ à $C_6$, pour former un composé de formule IX

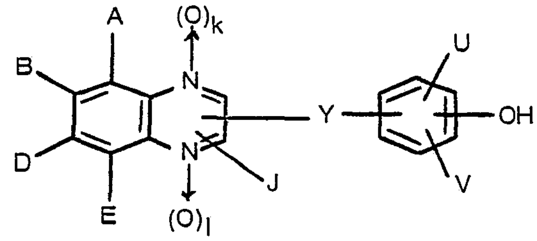

IX

dans laquelle A, B, D, E, J, U, V, Y, k et l ont les définitions données ci-dessus; puis à faire réagir le composé de formule IX avec un composé de formule X

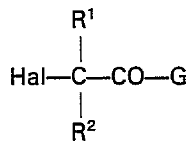

(X)

dans laquelle $R^1$, $R^2$ et G ont les définitions données ci-dessus et Hal est le chlore, le brome ou l'iode.

18. Composé de formula VIII

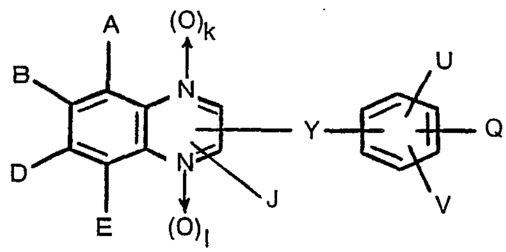

VIII,

dans laquelle A, B, D, E, J, U, V, Y, k et l ont les définitions données dans l'une quelconque des revendications 1 à 6 et 8 inclus et Q est un groupe hydroxy ou alkoxy en $C_1$ à $C_6$, sous réserve que lorsque le composé répond à la formule XIII:

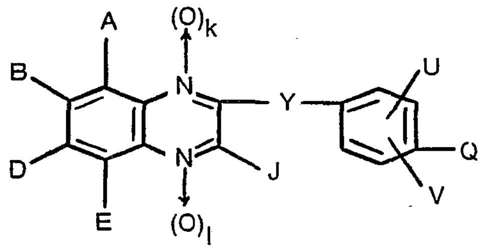

(XIII)

et que A, B, D et E représentent l'hydrogène ou qu'un ou deux de A, B, D et E représentent un halogène et que les autres représentent l'hydrogène, J, U et V représentent l'hydrogène, Y est l'oxygène et k et l sont égaux à 0, Q soit un groupe alkoxy en $C_1$ à $C_6$.

**Revendications pour l'Etat contractant: AT**

1. Procédé de synthèse d'un composé de formule I

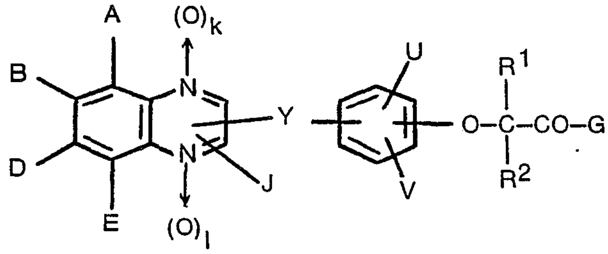

I

ou d'un sel de ce composé, formule dans laquelle

A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α de carbone avec un substituant choisi entre des substituants alkoxy en $C_1$ à $C_6$, amino, N-(alkyle en $C_1$ à $C_6$)amino, N,N-di-(alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe —OM dans lequel M est un ion de métal alcalin ou un ion métal alcalino-terreux;

Y est choisi entre l'oxygène et le soufre; et

k et l sont choisis indépendamment entre 0 et 1 et la somme k+l est choisie entre 0 et 1;

sous réserve que lorsque le composé répond à la formule II:

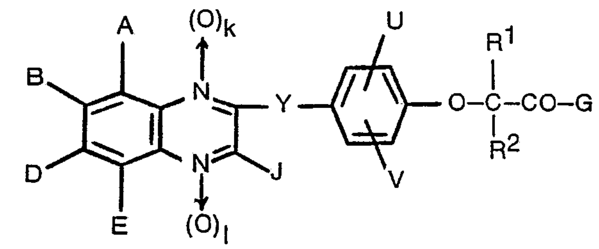

II

et que A, B, D et E représentent l'hydrogène, ou qu'un ou deux de A, B, D et E représentent un halogène et les autres représentent l'hydrogène, J, U, V et $R^2$ représentent de l'hydrogène, $R^1$ représente l'hydrogène ou un groupe alkyle en $C_1$ à $C_6$, Y représente l'oxygène et k et l ont la valeur 0, G ne soit pas un groupe hydroxy, alkoxy en $C_1$ à $C_{10}$ ou le groupe OM dans lequel M a la définition donnée ci-dessus, mais sous réserve en outre que le composé puisse être l'ester méthylique de l'acide 2-[4-(6-chloro-2-quinoxalinyloxy)-phénoxy]propionique.

procédé qui consiste à faire réagir un dérivé de quinoxaline de formule IX avec un composé de formule X

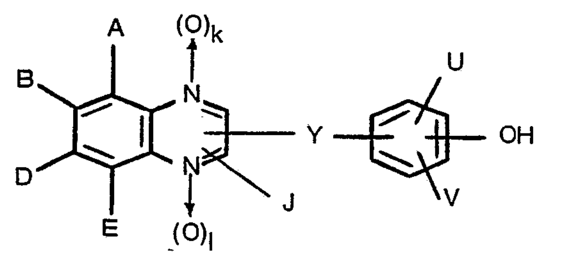

(IX)

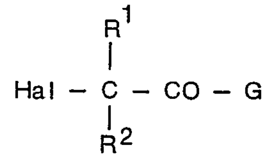

(X)

où A, B, D, E, J, U, V, $R^1$, $R^2$, G, Y, k et l ont les définitions données ci-dessus et Hal représente le chlore, le brome ou l'iode, ou à faire réagir un dérivé de quinoxaline de formule V avec un composé de formule VI

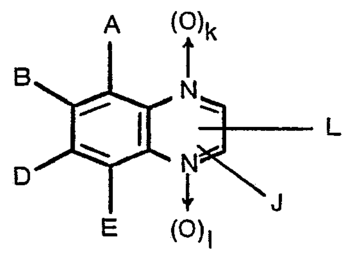

(V)

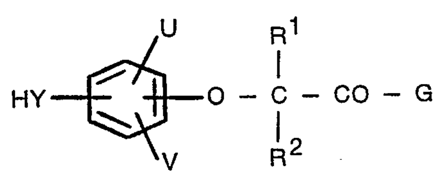

(VI)

où A, B, D, E, J, U, V, $R^1$, $R^2$, G, Y, k et l ont les définitions données ci-dessus et L est un groupe partant.

2. Procédé suivant la revendication 1, dans lequel le composé de quinoxaline de formule IX est préparé par réaction d'un dérivé de quinoxaline de formule V dans laquelle A, B, D, E, J, k et l ont les définitions données dans la revendication 1 et L est un groupe partant, avec un phénol ou un thiophénol de formule VII

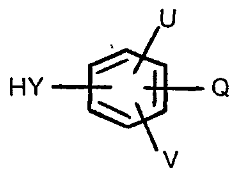

(VII)

dans laquelle U, V et Y ont les définitions données dans la revendication 1 et Q est un groupe hydroxy ou un groupe alkoxy en $C_1$ à $C_6$, pour former un composé de formule VIII

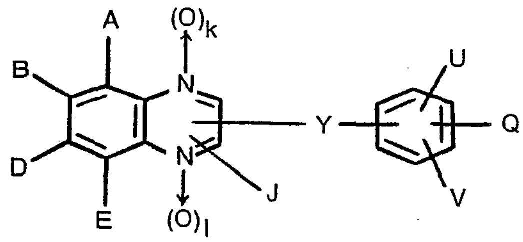

(VIII)

dans laquelle A, B, D, E, J, U, V, Y, k et l ont les définitions données dans la revendication 1 et Q a la définition donnée ci-dessus et, lorsque Q est un groupe alkoxy en $C_1$ à $C_6$, désalkylation du composé de formule VIII pour former un composé de formule IX tel que défini dans la revendication 1.

3. Procédé de synthèse d'un composé de formule I

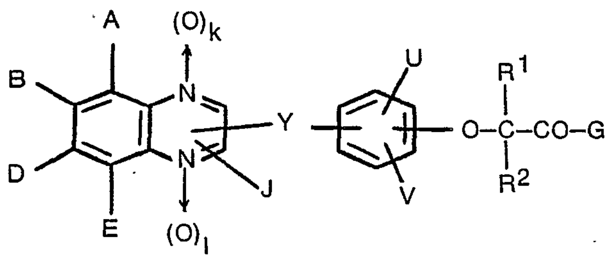

I

ou d'un sel de ce composé, formule dans laquelle

A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α de carbone avec un substituant choisi entre des substituants alkoxy en $C_1$ à $C_6$, amino, N-(alkyle en $C_1$ à $C_6$)amino, N,N-di-(alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe —OM dans lequel M est un ion de métal alcalin ou un ion métal alcalino-terreux;

Y est choisi entre l'oxygène et le soufre; et

k et l sont choisis indépendamment entre 0 et 1 et la somme k+l est égale à 0 ou à 1;

qui consiste à désalkyler un composé de formule (VIII)

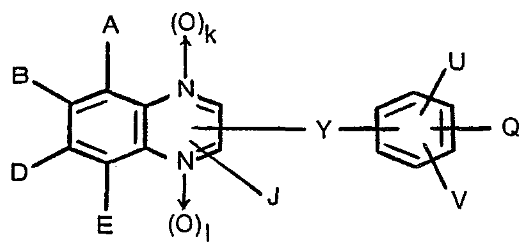

(VIII)

dans laquelle A, B, D, E, J, U, V, Y, k et l ont les définitions données ci-dessus et Q est un groupe alkoxy en $C_1$ à $C_6$ pour former un composé de formule IX

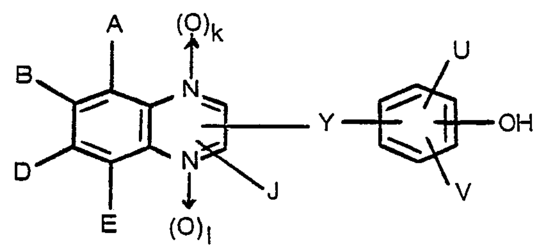

IX

dans laquelle A, B, D, E, J, U, V, Y, k et l ont les définitions données ci-dessus; puis à faire réagir le composé de formule IX avec un composé de formule X

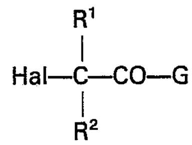

(X)

dans laquelle $R^1$, $R^2$ et G ont les définitions données ci-dessus et Hal est le chlore, le brome ou l'iode.

4. Procédé suivant la revendication 1, 2 ou 3, dans lequel A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogene;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α de carbone avec un substituant choisi entre des substituants alkoxy en $C_1$ à $C_6$, amino, N-(alkyle en $C_1$ à $C_6$)amino, N,N-di-(alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe —OM dans lequel M est un ion de métal alcalin ou un ion métal alcalino-terreux;

Y est choisi entre l'oxygène et le soufre; et

k et l sont choisis indépendamment entre 0 et 1 et la somme k+l est égale à 0 ou à 1;

sous réserve que lorsque le procédé est appliqué à la synthèse d'un composé de formule II

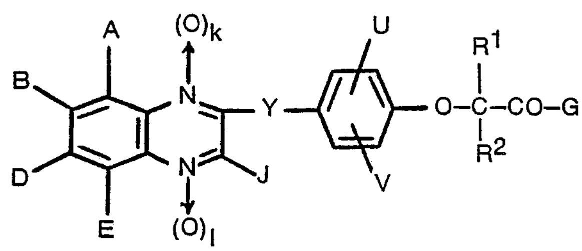

II

et que A, B, D et E représentent l'hydrogène ou bien un ou deux de A, B, D et E représentent un halogène et les autres représentent l'hydrogène, J, U, V et $R^2$ représentent l'hydrogène, $R^1$ est l'hydrogène ou un groupe alkyle en $C_1$ à $C_6$, Y représente l'oxygène et k et l sont égaux à 0, G ne représente pas un groupe hydroxy, alkoxy en $C_1$ à $C_{10}$, halogénalkoxy en $C_1$ à $C_{10}$, alcényloxy en $C_2$ à $C_6$, alcynyloxy en $C_3$ à $C_6$, cyclohexyloxy, phénoxy, benzyloxy, le groupe alkoxy en $C_2$ à $C_{10}$ substitué ailleurs que sur son atome α de carbone avec le substituant alkoxy en $C_1$ à $C_6$, ou le groupe OM dans lequel M a la définition donnée ci-dessus.

5. Procédé suivant l'une quelconque des revendications précédentes, pour la synthèse d'un composé de formule II dans laquelle A, B, D, E, J, U, V, $R^1$, $R^2$, G, Y, k et l ont les définitions données dans la revendication 1.

6. Procédé suivant l'une quelconque des revendications précédentes, dans lequel

A est choisi entre l'hydrogène et un halogène;

B est choisi entre l'hydrogène, un halogène, un groupe alkyle en $C_1$ à $C_6$, et un groupe halogénalkyle en $C_1$ à $C_6$;

D est choisi entre l'hydrogène, un halogène, un groupe alkyle en $C_1$ à $C_6$, halogénalkyle en $C_1$ à $C_6$, amino, N,N-di(alkyle en $C_1$ à $C_6$)amino et nitro;

E et V sont l'hydrogène;

J, et U sont choisis indépendamment entre l'hydrogène et un halogène;

$R^1$ est un groupe alkyle en $C_1$ à $C_6$;

$R^2$ est choisi entre l'hydrogène et un groupe alkyle en $C_1$ à $C_6$;

G est choisi entre un groupe hydroxy, alkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, le groupe alkoxy en $C_2$ à $C_{10}$ substitué ailleurs que sur son atome α de carbone avec un substituant choisi entre des substituants amino, N,N-di-alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe OM dans lequel M est un ion de métal alcalin;

Y est l'oxygène; et

k et l sont choisis indépendamment entre 0 et 1 et la somme k+l a la valeur 0 ou 1.

7. Procédé suivant l'une quelconque des revendications précédentes, dans lequel:

A, E, J, U, V et $R^2$ sont l'hydrogène;

B et D sont choisis indépendamment entre l'hydrogène, un halogène et le groupe méthyle,

$R^1$ est le groupe méthyle;

G est choisi entre un groupe hydroxy, alkoxy en $C_1$ à $C_6$, alcényloxy en $C_2$ à $C_6$, alcynyloxy en $C_3$ à $C_6$, cyclohexyloxy, le groupe alkoxy en $C_2$ à $C_6$ substitué ailleurs que sur son atome α de carbone avec un substituant choisi entre des substituants N,N-di(alkyle en $C_1$ à $C_6$)amino et N,N,N-tri(alkyle en $C_1$ à $C_6$)ammonio, et le groupe OM dans lequel M est un ion de métal alcalin;

Y est l'oxygène;

k a la valeur 0 ou 1; et

l a la valeur 0.

8. Procédé suivant l'une quelconque des revendications précédentes, dans lequel:

A, B, E, J, U, V et $R^2$ sont l'hydrogène;

D est choisi entre le brome et le chlore;

$R^2$ est le groupe méthyle;

G est choisi entre un groupe hydroxy, alkoxy en $C_1$ à $C_6$, allyloxy, cyclohexyloxy, 2-[N,N-di(alkyle en $C_1$ à $C_6$)amino]-éthoxy, 2-[N,N,N-tri(alkyle en $C_1$ à $C_6$)ammonio]éthoxy et le groupe OM dans lequel M est le sodium ou le potassium;

Y est l'oxygène;

k a la valeur 0 ou 1; et

l a la valeur 0.

9. Procédé suivant l'une quelconque des revendications précédentes, dans lequel les corps réactionnels sont choisis de manière à produire un composé qui est l'ester de méthyle ou de cyclohexyle de l'acide 2-[4-(6-chloro-2-quinoxalinyloxy)phénoxy]propionique ou l'ester de cyclohexyle de l'acide 2-[4-(6-bromo-2-quinoxalinyloxy)-phénoxy]propionique.

10. Procédé de synthèse d'un composé de formule I

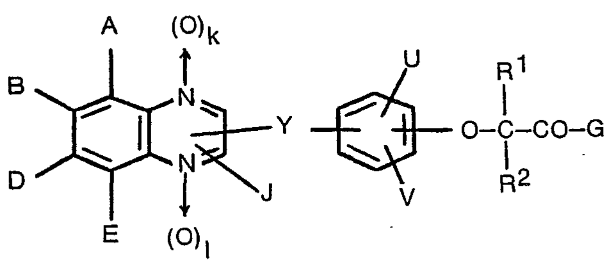

I

ou d'un sel de ce composé, formule dans laquelle A, B, D, et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J est l'hydrogène;

U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_5$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, phenoxy, benzyloxy, le groupe alkoxy en $C_2$ à $C_{10}$ substitué ailleurs que sur son atome α de carbone avec un radical alkoxy en $C_1$ à $C_6$, et le groupe OM dans lequel M est un ion de métal alcalin ou un ion de métal alcalino-terreux;

Y est choisi entre l'oxygène et le soufre; et

k et l ont chacun la valeur 0;

sous réserve que lorsque le composé répond à la formule II

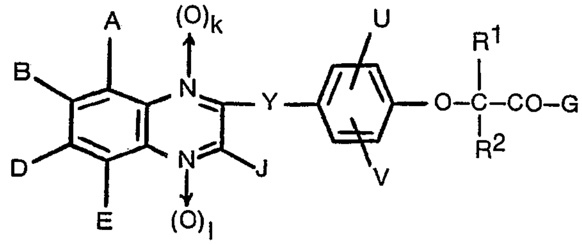

II

et que A, B, D et E sont l'hydrogène, ou bien un ou deux de A, B, D et E représentent un halogène et les autres représentent l'hydrogène, U, V et $R^2$ sont l'hydrogène,

$R^1$ est l'hydrogène ou un groupe alkyle en $C_1$ à $C_6$ et

Y est l'oxygène, G ne représente pas un groupe hydroxy, alkoxy en $C_1$ à $C_{10}$ ou le groupe OM dans lequel M a la définition donnée ci-dessus;

procédé qui consiste à faire réagir un dérivé de quinoxaline de formule IX avec un composé de formule X

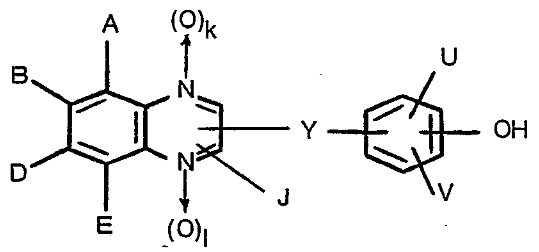

(IX)

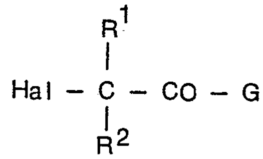

(X)

où A, B, D, E, J, U, V, $R^1$, $R^2$, G, Y, k et l ont les définitions données ci-dessus et Hal représente le chlore, le brome ou l'iode, ou à faire réagir un dérivé de quinoxaline de formule V avec un composé de formule VI

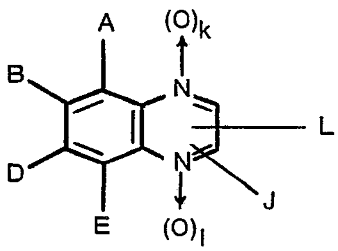

(V)

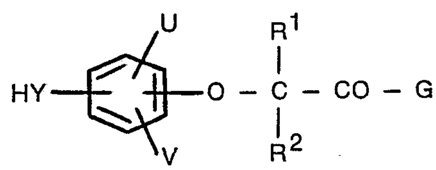

(VI)

où A, B, D, E, J, U, V, $R^1$, $R^2$, G, Y, k et l ont les définitions données ci-dessus et L est un groupe partant.

11. Procédé de synthès d'une composition douée d'activité herbicide contre les mauvaises herbes monocotylédones dans des cultures dicotylédones, caractérisé en ce qu'elle est essentiellement formée d'un énantiomère de formule I

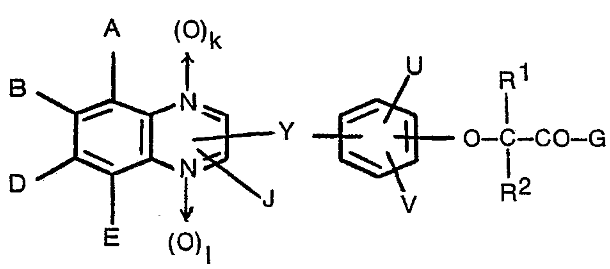

I

ou d'un sel de cet énantiomère,

formule dans laquelle

A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α de carbone avec un substituant choisi entre un groupe alkoxy en $C_1$ à $C_6$, amino, N-(alkyle en $C_1$ à $C_6$)amino, N,N-di(alkyle en $C_1$ à $C_6$)amino et N,N,N-tri(alkyle en $C_1$ à $C_6$)ammonio, le groupe —O—N=$R^6$ dans lequel $R^6$ est un radical alkylidène en $C_1$ à $C_{10}$, et le groupe —OM dans lequel M est un ion de métal alcalin ou un ion métal alcalino-terreux;

Y est choisi entre l'oxygène et le soufre; et

k et l sont choisis indépendamment entre 0 et 1 et la somme k+l a la valeur 0 ou 1;

ladite composition étant prtatiquement dépourvue de l'autre énantiomère;

procédé qui consiste à faire réagir un dérivé de quinoxaline de formule IX avec un composé de formule X

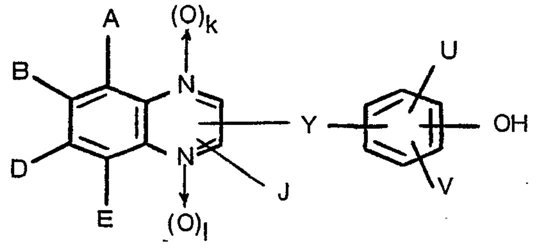

(IX)

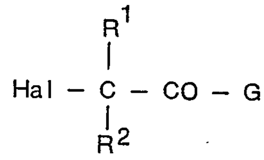

(X)

où A, B, D, E, J, U, V, $R^1$, $R^2$, G, Y k et l ont les définitions données ci-dessus et Hal désigne le chlore, le brome ou l'iode, ou à faire réagir un dérivé de quinoxaline de formule V avec un composé de formule VI

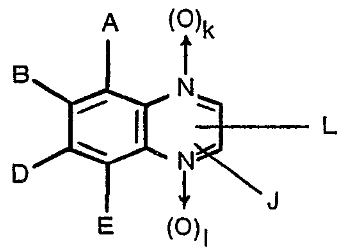

(V)

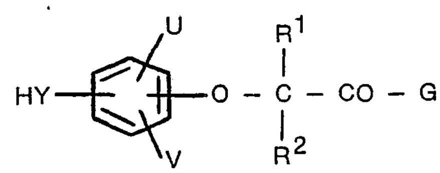

(VI)

où A, B, D, E, J, U, V, $R^1$, $R^2$, G, Y, k et l ont les définitions données ci-dessus et L est un groupe partant.

12. Composition herbicide, comprenant comme ingrédient actif un composé de formule I ou II tel que défini dans l'une quelconque des revendications 1 à 10 inclus, et un support approprié.

13. Composition herbicide, comprenant comme ingrédients actifs (a) un composé de formule (I)

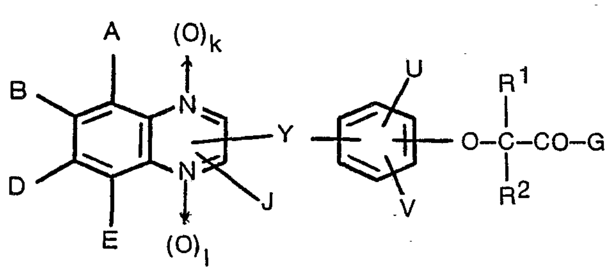

I

ou un sel de ce composé, formule dans laquelle

A, B, D et E sont choisis indépendamment entre l'hydrogène, un halogène, un groupe nitro, alkyle en $C_1$ à $C_6$, halogénalkyle en $C_1$ à $C_6$, amino, alkylamino en $C_1$ à $C_6$ et di(alkyle en $C_1$ à $C_6$)amino;

J, U et V sont choisis indépendamment entre l'hydrogène et un halogène;

$R^1$ est choisi entre l'hydrogène, un groupe alkyle en $C_1$ à $C_6$ et un groupe (alkoxy en $C_1$ à $C_6$)carbonyle;

$R^2$ est choisi entre l'hydrogène et un groupe alkyle en $C_1$ à $C_6$;

G est choisi entre un groupe hydroxy, alkoxy en $C_1$ à $C_{10}$, halogénalkoxy en $C_1$ à $C_{10}$, alcényloxy en $C_2$ à $C_{10}$, alcynyloxy en $C_3$ à $C_{10}$, alkylthio en $C_1$ à $C_{10}$, cyclohexyloxy, phénoxy, benzyloxy, le groupe alkoxy en $C_2$ à $C_{10}$ substitué ailleurs que sur son atome α de carbone avec un substituant choisi entre des substituants alkoxy en $C_1$ à $C_6$, amino, N-(alkyle en $C_1$ à $C_6$)amino, N,N-di-(alkyle en $C_1$ à $C_6$)amino et N,N,N-tri(alkyle en

$C_1$ à $C_6$)ammonio, le groupe —O—N=$R^6$ dans lequel $R^6$ est un radical alkylidène en $C_1$ à $C_{10}$, et le groupe —OM dans lequel M est un ion de métal alcalin ou un ion métal alcalino-terreux;

Y est choisi entre l'oxygène et le soufre; et

k et l sont choisis indépendamment entre 0 et 1 et la somme k+l a la valeur 0 et 1;

et (b) un autre herbicide qui est un 2,2-dioxyde de benzo-2,1,3-thiadiazine-4-one; un herbicide hormonal; une 3-[4-(4-halogénophénoxy)phényl]-1,1-dialkylurée; un dinitrophénol ou l'un de ses dérivés; un herbicide du type dinitroaniline; un herbicide du type phénylurée; un phénylcarbamoylphénylcarbamate; une 2-phénylpyridazine-3-one; un herbicide du type uracile; un herbicide triazinique; un herbicide du type 1-alkoxy-1-alkyl-3-phénylurée; un herbicide du type thiolcarbamate; un herbicide du type 1,2,4-triazine-5-one; un herbicide du type de l'acide benzoïque; un herbicide du type anilide; un herbicide dihalogénobenzonitrilique; un herbicide du type acide halogénalcylique ou d'un sel de cet acide; un herbicide du type éther de diphényle (par exemple l'éther de 4-nitrophényle et de 2-nitro-4-trifluorométhylphényle, le 5-(2,4-dichlorophénoxy)-2-nitrobenzoate de méthyle, l'acide 2-nitro-5-(2-chloro-4-trifluorométhylphénoxy)-benzoïque, l'éther de 2-chloro-4-trifluorométhylphényle et de 3-éthoxy-4-nitrophényle ou le 2-nitro-5-(2-chloro-4-trifluorométhylphénoxy)-N-méthanesulfonylbenzamide); le N,N-diméthyldiphénylacétamide; l'acide N-(1-naphtyl)-phtalamique; le 3-amino-1,2,4-triazole; un herbicide du type bipyridylium; un herbicide organoarsénical ou un herbicide du type d'un aminoacide ou un sel ou ester de cet acide.

14. Procédé pour endommager gravement ou détruire des plantes indésirables, qui consiste à appliquer auxdites plantes ou au milieu de croissance desdites plantes une quantité efficace d'un composé tel que défini dans l'une quelconque des revendications 1 à 10 inclus ou une quantité efficace d'une composition telle que définie conformément à l'une quelconque des revendications 11 à 13.

15. Procédé pour combattre sélectivement la croissance des mauvaises herbes monocotylédones dans des cultures dicotylédones, qui consiste à appliquer auxdites cultures ou aux milieux où elles se développent un composé tel que défini dans l'une quelconque des revendications 1 à 10 inclus ou une composition telle que définie dans l'une quelconque des revendications 11 à 13 en une quantité suffisante pouvant endommager gravement ou détruire les mauvaises herbes mais insuffisante pour endommager sensiblement la plante cultivée.

16. Procédé suivant la revendication 14 ou 15, dans lequel le composé est appliqué à un taux compris dans l'intervalle de 0,005 à 20 kg par hectare.

17. Procédé pour la synthèse d'un composé de formule VIII

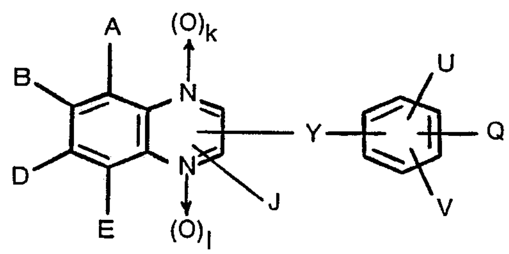

VIII,

dans laquelle A, B, D, E, J, U, V, Y, k et l ont les définitions données dans l'une quelconque des revendications 1 à 8 et 10 inclus et Q est un groupe hydroxy ou alkoxy en $C_1$ à $C_6$, procédé qui consiste à faire réagir un dérivé de quinoxaline de formule V

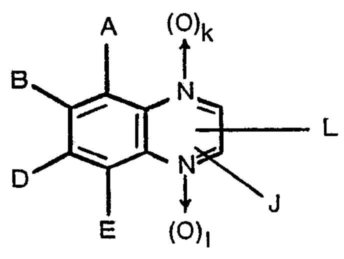

(V)

dans laquelle A, B, D, E, J, k et l ont les définitions données ci-dessus et L est un groupe partant, avec un phénol ou un thiophénol de formule VII

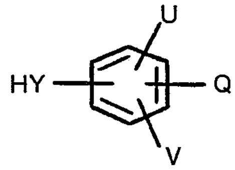

(VII)

dans laquelle U, V, Y et Q ont les définitions données ci-dessus.